# EUROPEAN PATENT SPECIFICATION

(11) **EP 3 129 044 B1**
(45) Date of publication and mention of the grant of the patent: **07.09.2022**
(21) Application number: 15715243.0
(22) Date of filing: 08.04.2015
(51) Int. Cl.: A61K 38/50, A61K 38/48, A61K 38/47, A61P 31/04

(54) **PEPTIDOGLYCAN HYDROLASE FOR USE IN THE TREATMENT OF CHRONIC BACTERIAL INFECTIONS**
PEPTIDOGLYCANHYDROLASE ZUR BEHANDLUNG VON CHRONISCHEN BAKTERIELLEN INFEKTIONEN
HYDROLASE DE PEPTIDOGLYCANE POUR LET TRAITEMENT DES INFECTIONS BACTÉRIENNES CHRONIQUES

(30) Priority: 08.04.2014 EP 14163927
(43) Date of publication of application: 15.02.2017
(73) Proprietor: Lysando AG, 9497 Triesenberg (LI)
(72) Inventor: MILLER, Stefan, 93055 Regensburg (DE); BRIERS, Yves, B-1910 Nederokkerzeel (BE); LAVIGNE, Rob, B-2180 Ekeren (BE)
(74) Representative: Rückerl, Florian
(86) International application number: PCT/EP2015/057625
(87) International publication number: WO 2015/155244

(56) References cited:
- EP-A1- 2 468 856
- WO-A1-2011/023702
- US-A1- 2011 243 915
- US-A1- 2012 189 606
- US-A1- 2013 052 182
- C. C. TENEBACK ET AL: "Bioengineered Lysozyme Reduces Bacterial Burden and Inflammation in a Murine Model of Mucoid Pseudomonas aeruginosa Lung Infection", ANTIMICROBIAL AGENTS AND CHEMOTHERAPY, vol. 57, no. 11, 26 August 2013 (2013-08-26), pages 5559-5564, XP055123713, ISSN: 0066-4804, DOI: 10.1128/AAC.00500-13
- Daniel C. Nelson ET AL: "Endolysins as Antimicrobials" In: "Bacteriophages, Part B", 2012, Elsevier, XP055123698, ISSN: 0065-3527 ISBN: 978-0-12-394438-2 vol. 83, pages 299-365, DOI: 10.1016/B978-0-12-394438-2.00007-4, abstract
- Y. BRIERS ET AL: "Art-175 Is a Highly Efficient Antibacterial against Multidrug-Resistant Strains and Persisters of Pseudomonas aeruginosa", ANTIMICROBIAL AGENTS AND CHEMOTHERAPY, vol. 58, no. 7, 1 July 2014 (2014-07-01), pages 3774-3784, XP55194736, ISSN: 0066-4804, DOI: 10.1128/AAC.02668-14
- FISCHETTI ET AL: "Bacteriophage lysins as effective antibacterials", CURRENT OPINION IN MICROBIOLOGY, CURRENT BIOLOGY LTD, GB, vol. 11, no. 5, 1 October 2008 (2008-10-01), pages 393-400, XP025744750, ISSN: 1369-5274, DOI: 10.1016/J.MIB.2008.09.012 [retrieved on 2008-10-14]

## Description

The present invention relates to a polypeptide comprising a peptidoglycan hydrolase for use as a medicament for the treatment of chronic bacterial infections caused by persister bacteria, wherein the peptidoglycan hydrolase is an endolysin and wherein the chronic bacterial infection is a chronic bacterial infection with *Pseudomonas, Escherichia, Staphylococcus aureus, Acinetobacter, Mycobacterium avium, Mycobacterium tuberculosis, Listeria monocytogenes* and/or *Salmonella* bacteria, and wherein the endolysin is capable of degrading the bacterial peptidoglycan of respective *Pseudomonas, Escherichia, Staphylococcus aureus, Acinetobacter, Mycobacterium avium, Mycobacterium tuberculosis, Listeria monocytogenes* or *Salmonella* bacteria.

One of the challenges in bacterial infectious diseases is the problem of chronic infections, which require long durations of antibiotic treatment and often reoccur. Chronic bacterial infections are typically caused by bacteria "persisters", i.e. a small subpopulation of bacteria that survive an antibiotic treatment by essentially shutting down metabolism, even as their counterparts, who are in a normal metabolic state, are killed off. As a result, the patient initially appears to be fully recovered, but over the course of weeks or months, the persisters start returning to life, often stronger and more aggressive than ever before, and the illness is back. Said bacterial persistence is a major obstacle to the successful treatment of infectious bacterial diseases. It can stretch illnesses out over months, cause bacterial infections to spread to kidneys and other organs, and raise treatment costs extremely. Unlike antibiotic resistant bacteria, whose ability to withstand drug treatments is based on genetic differences, persisters are genetically identical to the other members of their bacterial community. Persisters are often called antibiotics tolerant bacterial strains. Given its significant negative impact, bacterial persistence has become a growing threat. Moreover, up to date, no treatment that directly targets persisters is known. Bacterial persistence has been verified in a number of species, such as *Escherichia coli, Pseudomonas aeruginosa, Staphylococcus spp., Acinetobacter baumannii, Mycobacterium spp., Listeria monocytogenes, Salmonella.* It is assumed that all bacterial pathogens can establish life-long chronic infections.

US 2012/189606 discloses a peptidoglycan hydrolase comprising an endolysin, autolysin or bacteriocin for use in the treatment of bacterial infections including multiresistant bacteria and chronic pneumonia.

Teneback et al. (Antimicrob Agents Chemother. 2013 Nov;57(11):5559-5564) disclose recombinant lysozyme for use in the treatment of *P. aeruginosa* lung infections in a mouse model of chronic lung infection.

US 2013/052182 discloses an endolysin for use in eliminating bacterial biofilms and bacterial infections including chronic pneumonia, wherein the endolysin is PhiKZgp144.

EP 2 468 856 discloses fusion peptides comprising an antimicrobial peptide such as sushi peptide or defensin linked to an enzyme such as endolysin, autolysin or bacteriocin for use in the treatment of bacterial infections including chronic pneumonia.

US 2011/243915 discloses endolysins fused to membrane or lipopolysaccharide disrupting peptides for use in the treatment of bacterial infections including chronic pneumonia or multiresistant bacterial strains.

WO 2011/23702 discloses a modified OBPgpLYS Pseudomonas putida endolysin N-terminally fused to various peptides and its effect on Acinetobacter baumannii, E. coli and Salmonella typhimurium.

Nonetheless, there is still a need for antibacterial agents that treat chronic bacterial infections caused by persister bacteria.

This object is solved by the subject matter defined in the claims.

The following figure serves to illustrate the invention.

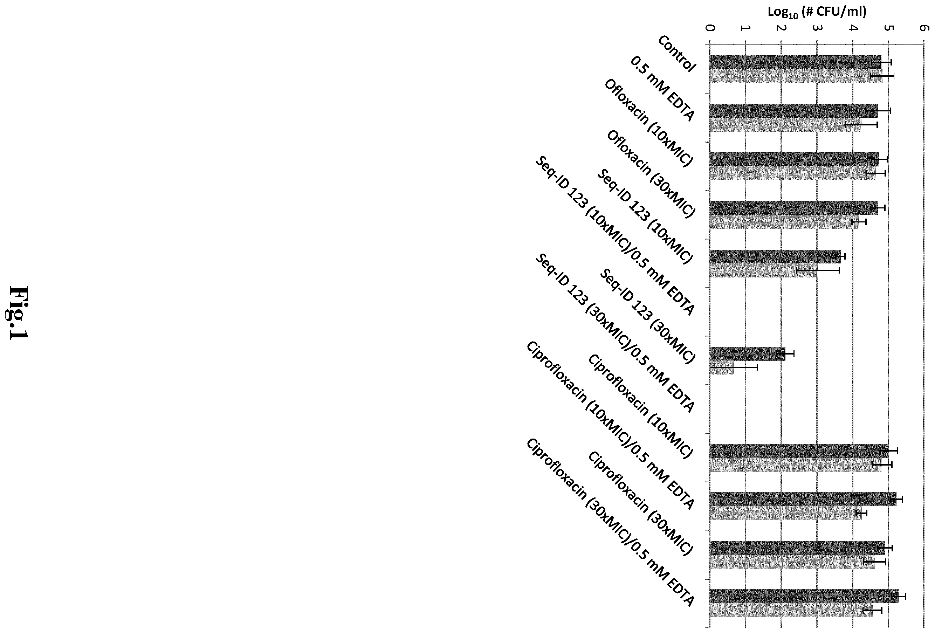

Figure 1 shows in a schematic diagram the bactericidal effect of a polypeptide according to SEQ ID NO: 123 (10x and 30x MIC) on isolated persister fractions of *P. aeruginosa* PA14 (light grey) and PA1255 (dark grey) is compared to ciprofloxacin (10xMIC and 30xMIC), both in the absence and presence of 0.5 mM EDTA. Controls include 0.5 mM EDTA and ofloxacin (10xMIC and 30xMIC). Mean values (± SEMs) are shown for at least three independent replicates.

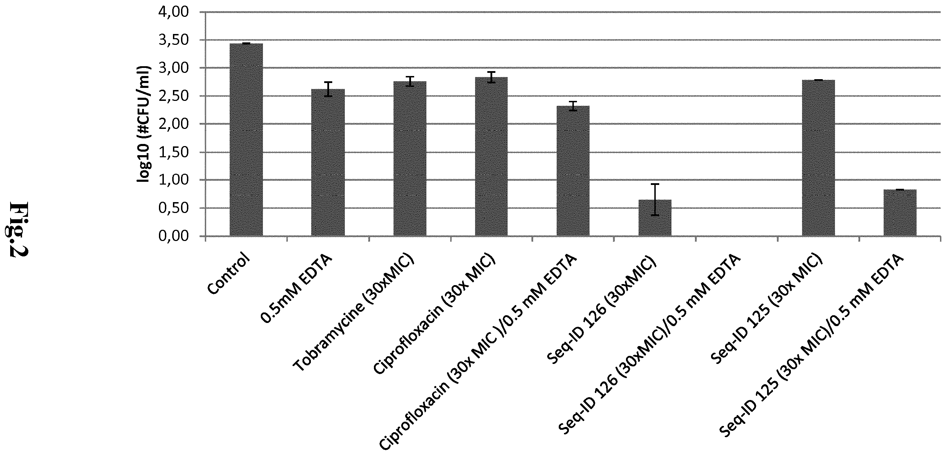

Figure 2 shows A. *baumannii* RUH134 persister killing by the polypeptide according to SEQ ID NO: 126 and the polypeptide according to SEQ ID NO: 125. The bactericidal effects of the polypeptide according to SEQ ID NO: 126 (30xMIC) and the polypeptide according to SEQ ID NO: 125 (30xMIC) on isolated persister fractions are compared to those of ciprofloxacin (30xMIC), in both the absence and the presence of 0.5 mM EDTA. Additional controls include 0.5 mM EDTA and tobramycin (30xMIC). Mean values (± standard error of the mean [SEM]) are shown for at least three independent replicates (except for the polypeptide according to SEQ ID NO: 125). The control shows the number of persisters before treatment.

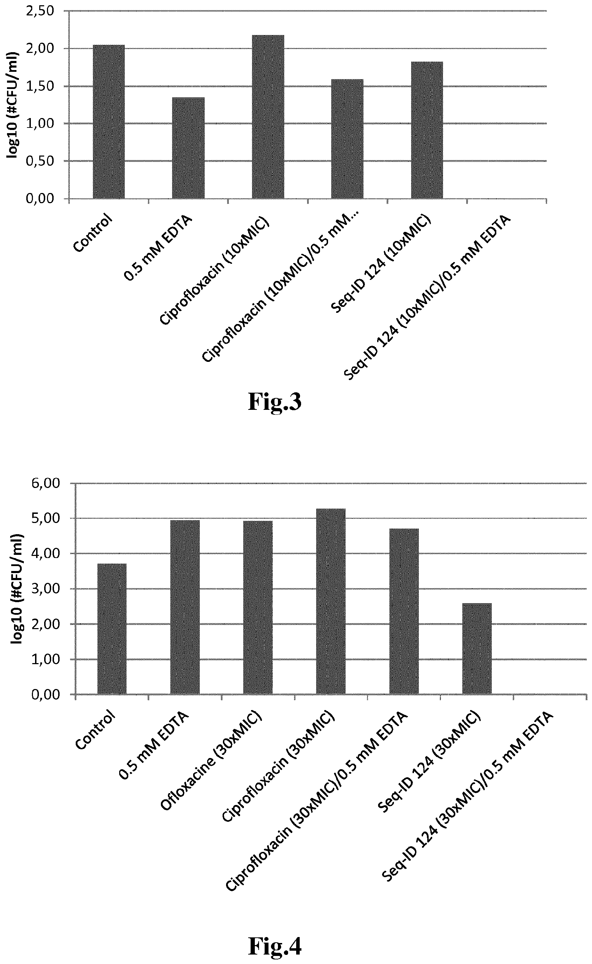

Figure 3 shows *E. coli* UTI-89 persister killing by the polypeptide according to SEQ ID NO: 124. The bactericidal effects of the polypeptide according to SEQ ID NO: 124 (10× MIC) on isolated persister fractions are compared to those of ciprofloxacin (10xMIC), in both the absence and the presence of 0.5 mM EDTA. Additional control is 0.5 mM EDTA.
Figure 4 shows *P. aeruginosa* PA14 persister killing by the polypeptide according to SEQ ID NO: 124. The bactericidal effects of the polypeptide according to SEQ ID NO: 124 (30× MIC) on isolated persister fractions are compared to those of ciprofloxacin (30xMIC), in both the absence and the presence of 0.5 mM EDTA. Additional controls include 0.5 mM EDTA and ofloxacin

The term "polypeptide" as used herein refers in particular to a polymer of amino acid residues linked by peptide bonds in a specific sequence. The amino acid residues of a polypeptide may be modified by e.g. covalent attachments of various groups such as carbohydrates and phosphate. Other substances may be more loosely associated with the polypeptide, such as heme or lipid, giving rise to conjugated polypeptides which are also comprised by the term "polypeptide" as used herein. The term as used herein is intended to encompass also proteins. Thus, the term "polypeptide" also encompasses for example complexes of two or more amino acid polymer chains. The term "polypeptide" does encompass embodiments of polypeptides which exhibit optionally modifications typically used in the art, e.g. biotinylation, acetylation, pegylation, chemical changes of the amino-, SH- or carboxyl-groups (e.g. protecting groups) etc.. As will become apparent from the description below, the polypeptide of to the present invention may also be a fusion protein, i.e. linkage of at least two amino acid sequences which do not occur in this combination in nature. The term "polypeptide" as used herein is not limited to a specific length of the amino acid polymer chain, but typically the polypeptide will exhibit a length of more than about 50 amino acids, more than about 100 amino acids or even more than about 150 amino acids. Usually, but not necessarily, a typical polypeptide of the present invention will not exceed about 750 amino acids in length.

The term "cell wall " as used herein refers to all components that form the outer cell enclosure of Gram-negative and Gram-positive bacteria and thus guarantee their integrity. In particular, the term "cell wall" as used herein refers to peptidoglycan, the outer membrane of the Gram-negative and peptidoglycan of Gram-positive bacteria with the lipopolysaccharide, the bacterial cell membrane, but also to additional layers deposited on the peptidoglycan as e.g. capsules, outer protein layers or slimes.

The term "amino acid sequence stretch" as used herein refers to a particular stretch of amino acid sequence in the amino acid sequence of the polypeptide of the invention. Said sequence refers to a sequence of a cationic peptide, a polycationic peptide, an amphiphatic peptide, a hydrophobic peptide, a sushi peptide and/or an antimicrobial peptide. The term does not refer to conventional tags like His-tags, such as Hiss-tags, His₆-tags, His₇-tags, His₈-tags, His₉-tags, His₁₀-tags, His₁₁-tags, His₁₂-tags, His₁₆-tags and His₂₀-tags, Strep-tags, Avi-tags, Myc-tags, Gst-tags, JS-tags, cystein-tags, FLAG-tags or other tags known in the art, thioredoxin or maltose binding proteins (MBP). The term "tag" in contrast to the term "amino acid sequence stretch" as used herein refers to a peptide which can be useful to facilitate expression and/or affinity purification of a polypeptide, to immobilize a polypeptide to a surface or to serve as a marker or a label moiety for detection of a polypeptide e.g. by antibody binding in different ELISA assay formats as long as the function making the tag useful for one of the above listed facilitation is not caused by the positively charge of said peptide. However, the His₆-tag may, depending on the respective pH, also be positively charged, but is used as affinity purification tool as it binds to immobilized divalent cations and is not understood to be a "amino acid sequence stretch" as used herein. Preferably an amino acid sequence stretch as used herein as a length of about 6 to about 39 amino acid residues.

As used herein, the term "cationic peptide" refers preferably to a peptide having positively charged amino acid residues. Preferably a cationic peptide has a pKa-value of 9.0 or greater. Typically, at least four of the amino acid residues of the cationic peptide can be positively charged, for example, lysine or arginine. "Positively charged" refers to the side chains of the amino acid residues which have a net positive charge at about physiological conditions. The term "cationic peptide" as used herein refers also to polycationic peptides, but also includes cationic peptides which comprise for example less than 20%, preferably less than 10% positively charged amino acid residues.

The term "polycationic peptide" as used herein refers preferably to a peptide composed of mostly positively charged amino acid residues, in particular lysine and/or arginine residues. A peptide is composed of mostly positively charged amino acid residues if at least about 20, 30, 40, 50, 60, 70, 75, 80, 85, 90, 95 or about 100 % of the amino acid residues are positively charged amino acid residues, in particular lysine and/or arginine residues. The amino acid residues being not positively charged amino acid residues can be neutrally charged amino acid residues and/or negatively charged amino acid residues and/or hydrophobic amino acid residues. Preferably the amino acid residues being not positively charged amino acid residues are neutrally charged amino acid residues, in particular serine and/or glycine.

The term, "antimicrobial peptide" (AMP) as used herein refers preferably to any naturally occurring peptide that has microbicidal and/or microbistatic activity on for example bacteria, viruses, fungi, yeasts, mycoplasma and protozoa. Thus, the term "antimicrobial peptide" as used herein refers in particular to any peptide having anti-bacterial, anti-fungal, anti-mycotic, anti-parasitic, anti-protozoal, anti-viral, anti-infectious, anti-infective and/or germicidal, algicidal, amoebicidal, microbicidal, bactericidal, fungicidal, parasiticidal, protozoacidal, protozoicidal properties. Preferred are anti-bacterial peptides. The antimicrobial peptide may be a member of the RNase A super family, a defensin, cathelicidin, granulysin, histatin, psoriasin, dermicidine or hepcidin. The antimicrobial peptide may be naturally occurring in insects, fish, plants, arachnids, vertebrates or mammals. Preferably the antimicrobial peptide may be naturally occurring in insects, fish, plants, arachnids, vertebrates or mammals. Preferably the antimicrobial peptide may be naturally occurring in radish, silk moth, wolf spider, frog, preferably in *Xenopus laevis, Rana* frogs, more preferably in *Rana catesbeiana,* toad, preferably Asian toad *Bufo bufo gargarizans,* fly, preferably in *Drosophila,* more preferably in *Drosophila melanogaster,* in *Aedes aegypti,* in honey bee, bumblebee, preferably in *Bombus pascuorum,* flesh fly, preferably in *Sarcophaga peregrine,* scorpion, horseshoe crab, catfish, preferably in *Parasilurus asotus,* cow, pig, sheep, porcine, bovine, monkey and human. As used herein, an "antimicrobial peptide" (AMP) may in particular be a peptide which is not a cationic peptide, polycationic peptide, amphiphatic peptide, sushi peptide, defensins, and hydrophobic peptide, but nevertheless exhibits antimicrobial activity.

The term "sushi peptide" as used herein refers to complement control proteins (CCP) having short consensus repeats. The sushi module of sushi peptides functions as a protein-protein interaction domain in many different proteins. Peptides containing a Sushi domain have been shown to have antimicrobial activities. Preferably, sushi peptides are naturally occurring peptides.

The term "amphiphatic peptide" as used herein refers to synthetic peptides having both hydrophilic and hydrophobic functional groups. Preferably, the term "amphiphatic peptide" as used herein refers to a peptide having a defined arrangement of hydrophilic and hydrophobic groups e.g. amphiphatic peptides may be e.g. alpha helical, having predominantly non polar side chains along one side of the helix and polar residues along the rest of its surface.

The term "hydrophobic group" as used herein refers preferably to chemical groups such as amino acid side chains which are substantially water insoluble, but soluble in an oil phase, with the solubility in the oil phase being higher than that in water or in an aqueous phase. In water, amino acid residues having a hydrophobic side chain interact with one another to generate a non-aqueous environment. Examples of amino acid residues with hydrophobic side chains are valine, isoleucine, leucine, methionine, phenylalanine, tryptophan, cysteine, alanine, tyrosine, and proline residues.

The term "hydrophobic peptide" as used herein refers to a hydrophobic peptide, which is preferably composed of mostly amino acid residues with hydrophobic groups. Such peptide is preferably composed of mostly hydrophobic amino acid residues, i.e. at least about 20, 30, 40, 50, 60, 70, 75, 80, 85, 90, 95 or at least about 100 % of the amino acid residues are hydrophobic amino acid residues. The amino acid residues being not hydrophobic are preferably neutral and preferably not hydrophilic.

The term "comprising" as used herein shall not be construed as being limited to the meaning "consisting of" (i.e. excluding the presence of additional other matter). Rather, "comprising" implies that optionally additional matter may be present. The term "comprising" encompasses as particularly envisioned embodiments falling within its scope "consisting of" (i.e. excluding the presence of additional other matter) and "comprising but not consisting of" (i.e. requiring the presence of additional other matter), with the former being more preferred.

The present invention relates to a polypeptide comprising a peptidoglycan hydrolase for use as a medicament for the treatment of chronic bacterial infections caused by persister bacteria, wherein the peptidoglycan hydrolase is an endolysin and wherein the chronic bacterial infection is a chronic bacterial infection with *Pseudomonas* (e.g. *Pseudomonas aeruginosa), Escherichia* (e.g. *E. coli), Staphylococcus* spp. (e.g. *Staphylococcus aureus), Acinetobacter* (e.g. *Acinetobacter baumanii), Mycobacterium* spp. (e.g. *Mycobacterium avium, Mycobacterium tuberculosis), Listeria monocytogenes* and/or *Salmonella* bacteria, and wherein the endolysin is capable of degrading the bacterial peptidoglycan of respective *Pseudomonas, Escherichia, Staphylococcus aureus, Acinetobacter, Mycobacterium avium, Mycobacterium tuberculosis, Listeria monocytogenes* or *Salmonella* bacteria.

The present invention further relates to a pharmaceutical composition comprising a polypeptide comprising a peptidoglycan hydrolase for use as a medicament for the treatment of chronic bacterial infections caused by persister bacteria, wherein the peptidoglycan hydrolase is an endolysin and wherein the chronic bacterial infection is a chronic bacterial infection with *Pseudomonas* (e.g. *Pseudomonas aeruginosa), Escherichia* (e.g. *E. coli), Staphylococcus* spp. (e.g. *Staphylococcus aureus), Acinetobacter* (e.g. *Acinetobacter baumanii), Mycobacterium* spp. (e.g. *Mycobacterium avium, Mycobacterium tuberculosis), Listeria monocytogenes* and/or *Salmonella* bacteria, and wherein the endolysin is capable of degrading the bacterial peptidoglycan of respective *Pseudomonas, Escherichia, Staphylococcus aureus, Acinetobacter, Mycobacterium avium, Mycobacterium tuberculosis, Listeria monocytogenes* or *Salmonella* bacteria.

The polypeptide for use of the present invention comprising the peptidoglycan hydrolase is composed of an amino acid molecule having the amino acid sequence of an endopeptidase, N-acetyl-muramoyl-L-alanine-amidase (amidase), N-acetyl-muramidase, N-acetylglucosaminidase or lytic transglycosylase and thus is suitable for degrading the peptidoglycan of bacterial cell walls. The polypeptide of the present invention thus exhibits the activity of a peptidoglycan hydrolase, i.e. is capable of degrading bacterial peptidoglycan. The peptidoglycan hydrolase may be composed of an amino acid molecule having at least the amino acid sequence selected from the group of an endopeptidase, N-acetyl-muramoyl-L-alanine-amidase (amidase), N-acetyl-muramidase, N-acetyl-glucosaminidase and lytic transglycosylase, i.e. the peptidoglycan hydrolase may have more than one peptidoglycan hydrolyzing activities.

The peptidoglycan structure of a bacterial cell wall is overall largely conserved with minor modifications (Schleifer & Kandler 1972). Bacterial species have interpeptide bridges composed of different amino acids or may even lack an interpeptide bridge. In peptidoglycan structures lacking an interpeptide bridge a Diaminopimelic acid (DAP ) or meso-Diaminopimelic acid (mDAP; an amino acid, representing an epsilon-carboxy derivative of lysine being a typical component of peptidoglycan) (Diaminopimelic acid is residue replaces the amino acid L-Lys and directly cross-links to the terminal amino acid D-Ala of the opposite peptide chain. Thus, there are limited types of chemical bonds available that can be hydrolyzed by peptidoglycan hydrolases. The peptidoglycan hydrolases exhibit at least one enzyme domain having an enzymatic activity as listed above. In addition the peptidoglycan hydrolases may contain at least one domain suitable for binding to the peptidoglycan and supporting the enzymatic activity of the peptidoglycan hydrolase. The binding domains are typically called cell-wall binding domains (CBD).

Preferably a domain having enzymatic activity is the N-acetylglucosaminidase that hydrolyzes the glycan component of the peptidoglycan on the reducing side of GlcNAc.

An example of a peptidoglycan hydrolase exhibiting the N-acetylglucosaminidase activity is the LambdaSa2 endolysin (Pritchard et al., 2007).

Further preferably, a domain having enzymatic activity is the N-acetyl-muramidase that hydrolyzes the glycan component of the peptidoglycan on the reducing side of MurNAc., i.e. the hydrolysis of the beta-(1->4) linkages between N-acetylmuramic acid and N-acetyl-D-glucosamine residues in a peptidoglycan. Furthermore, N-acetyl-muramidase hydrolyzes N-acetyl-D-glucosamine residues in chitodextrins. The EC number of N-acetyl-muramidase is EC 3.2.1.17 with alternative names for N-acetyl-muramidase like 1,4-N-acetylmuramidase, mucopeptide, glucohydrolase, mucopeptide, N-acetylmuramoylhydrolase, muramidase, N,O-diacetylmuramidase, and peptidoglycan N-acetylmuramoylhydrolase. Examples of peptidoglycan hydrolases exhibiting the N-acetyl-muramidase activity are endolysins Cpl -1 endolysin (Garcia et al., 1987), B30 endolysin (Pritchard et al., 2004), and Cpl-7 (Garcia et al., 1990).

Further preferably, a domain having enzymatic activity is the lytic transglycosylase that hydrolyzes the same bond as muraminidases, but form N-acetyl-1,6-anhydro-muramyl intermediates during hydrolysis, i.e. the hydrolysis of the b(1 -> 4) linkages between N-acetylmuramyl and N-acetylglucosaminyl residues of the peptidoglyccan. The reaction includes forming a N-acetyl-1,6-anhydro-muramyl moiety residue during glycosidic cleavage. The EC number of lytic transglycosylase is EC 4.2.2.n1 - Peptidoglycan lytic exotransglycosylase with alternative names for lytic transglycosylase like peptidoglycan lytic exotransglycosylase and exomuramidase. Examples of peptidoglycan hydrolases exhibiting the lytic transglycosylase activity are phage lambda endolysin (Taylor and Gorazdowska, 1974) and phage Phi KZ gp144 endolysin (Paradis-Bleau et al., 2007).

Further preferably, a domain having enzymatic activity is the N-acetylmuramoyl-L-alanine amidase that hydrolyzes the amide bond between the glycan moiety (MurNAc) and the peptide moiety (L-alanine) of the cell wall. The EC number of N-acetylmuramoyl-L-alanine amidase is EC 3.5.1.28 with alternative names for N-acetylmuramoyl-L-alanine amidase like amidase, acetylmuramyl-L-alanine amidase; N-acetylmuramyl-L-alanine amidase; N-acylmuramyl-L-alanine amidase; acetylmuramoyl-alanine amidase; N-acetylmuramic acid L-alanine amidase; acetylmuramyl-alanine amidase; N-acetylmuramylalanine amidase; murein hydrolase; N-acetylmuramoyl-L-alanine amidase type I; N-acetylmuramoyl-L-alanine amidase type II. Examples of peptidoglycan hydrolases exhibiting the N-acetylmuramoyl-L-alanine amidase activity are amidase domain of the staphylococcal phage F11 endolysin (Navarre et al., 1999), LysK (Becker et al., 2009a; Donovan et al., 2009), Listeria phage endolysin Ply511 (Loessner et al., 1995b) and PlyPSA (Korndorfer et al., 2006).

Further preferably, a domain having enzymatic activity is the endopeptidase that hydrolyzes peptide bonds between two amino acids. Cleavage may occur in the stem peptide, e.g. Ply500 and Ply118 L-alanyl-D-glutamate endolysins (Loessner et al., 1995b) or in the interpeptide bridge, e.g., F11 D-alanyl-glycyl endolysin (Navarre et al., 1999). The EC number of endopeptidase is EC 3.4.24 in case of Ply118. An alternative name for endopeptidase is metallopeptidase. Examples of peptidoglycan hydrolases exhibiting the endopeptidase activity are. Ply500, Ply118, L-alanyl-D-glutamate endolysins (Loessner et al., 1995b), Ply PSA, LysK, Lambda SaII, F11 D-alanyl-glycyl endolysin (Navarre et al., 1999), B30.

The peptidoglycan degrading activity of a polypeptide having peptidoglycan hydrolase activity on Gram-negative and Gram-positive bacteria can be measured by assays well known in the art, e.g. by muralytic assays in which the outer membrane of Gram-negative bacteria is permeabilized or removed (e.g. with chloroform) to allow the putative enzyme access to the peptidoglycan layer. If the enzyme is active, degradation of the peptidoglycan layer will lead to a drop of turbidity, which can be measured photometrically (see for example Briers et al., J. Biochem. Biophys Methods 70: 531-533, (2007) or Schmelcher et al., Bacteriophage endolysins as novel antimicrobials. Schmelcher M, Donovan DM, Loessner MJ. Future Microbiol. 2012 Oct;7(10):1147-7).

The peptidoglycan hydrolase is an endolysin.

Preferred endolysins are PhiV10p30 of phage ΦV10 (Sequence analysis of Escherichia coli O157:H7 bacteriophage PhiV10 and identification of a phage-encoded immunity protein that modifies the 0157 antigen. Perry LL, SanMiguel P, Minocha U, Terekhov AI, Shroyer ML, Farris LA, Bright N, Reuhs BL, Applegate BM. FEMS Microbiol Lett. 2009 Mar;292(2):182-6); STM0907.Fels0 of phage FELS-1 (Nature. 2001 Oct 25;413(6858):852-6. Complete genome sequence of Salmonella enterica serovar Typhimurium LT2. McClelland M1, Sanderson KE, Spieth J, Clifton SW, Latreille P, Courtney L, Porwollik S, Ali J, Dante M, Du F, Hou S, Layman D, Leonard S, Nguyen C, Scott K, Holmes A, Grewal N, Mulvaney E, Ryan E, Sun H, Florea L, Miller W, Stoneking T, Nhan M, Waterston R, Wilson RK); epsilon15p25 of phage ε15 (Virology. 2007 Dec 20;369(2):234-44. Epub 2007 Sep 7. The genome of epsilon15, a serotype-converting, Group E1 Salmonella enterica-specific bacteriophage. Kropinski AM1, Kovalyova IV, Billington SJ, Patrick AN, Butts BD, Guichard JA, Pitcher TJ, Guthrie CC, Sydlaske AD, Barnhill LM, Havens KA, Day KR, Falk DR, McConnell MR); YuA20 of phage YUA (NCBI Reference Sequence: YP_001595885.1 and J Bacteriol. 2008 Feb;190(4):1429-35. The genome and structural proteome of YuA, a new Pseudomonas aeruginosa phage resembling M6. Ceyssens PJ1, Mesyanzhinov V, Sykilinda N, Briers Y, Roucourt B, Lavigne R, Robben J, Domashin A, Miroshnikov K, Volckaert G, Hertveldt K); ORF23 of phage B3 (Complete genomic sequence of bacteriophage B3, a Mu-like phage of Pseudomonas aeruginosa. Braid MD, Silhavy JL, Kitts CL, Cano RJ, Howe MM. J Bacteriol. 2004 Oct;186(19):6560-74); BcepMu22 of phage BcepMu (J Mol Biol. 2004 Jun 25;340(1):49-65. Burkholderia cenocepacia phage BcepMu and a family of Mu-like phages encoding potential pathogenesis factors. Summer EJ1, Gonzalez CF, Carlisle T, Mebane LM, Cass AM, Savva CG, LiPuma J, Young R); F116p62 of phage F116 (Gene. 2005 Feb 14;346:187-94. The genome of the Pseudomonas aeruginosa generalized transducing bacteriophage F116. Byrne M1, Kropinski AM.); STM2715.S.Fels2 of phage Fels2 (Nature. 2001 Oct 25;413(6858):852-6. Complete genome sequence of Salmonella enterica serovar Typhimurium LT2. McClelland M1, Sanderson KE, Spieth J, Clifton SW, Latreille P, Courtney L, Porwollik S, Ali J, Dante M, Du F, Hou S, Layman D, Leonard S, Nguyen C, Scott K, Holmes A, Grewal N, Mulvaney E, Ryan E, Sun H, Florea L, Miller W, Stoneking T, Nhan M, Waterston R, Wilson RK); gp76 of phage ES18 (Casjens,S.R., Gilcrease,E.B., Winn-Stapley,D.A., Schicklmaier,P., Schmieger,H., Pedulla,M.L., Ford,M.E., Houtz,J.M., Hatfull,G.F. and Hendrix,R.W. The generalized transducing Salmonella bacteriophage ES18: complete genome sequence and DNA packaging strategy J. Bacteriol. 187 (3), 1091-1104 (2005)); SPSV3_gp23 of phage SETP3 (J Med Microbiol. 2009 Jan;58(Pt 1):86-93. Characterization of bacteriophages used in the Salmonella enterica serovar Enteritidis phage-typing scheme. De Lappe N1, Doran G, O'Connor J, O'Hare C, Cormican M); phi32_17 of phage ΦECO32 (Genomic and proteomic analysis of phiEco32, a novel Escherichia coli bacteriophage. Savalia D, Westblade LF, Goel M, Florens L, Kemp P, Akulenko N, Pavlova O, Padovan JC, Chait BT, Washburn MP, Ackermann HW, Mushegian A, Gabisonia T, Molineux I, Severinov K. J Mol Biol. 2008 Mar 28;377(3):774-89); HK022p54 of phage HK022 (J Mol Biol. 2000 May 26;299(1):27-51. Genomic sequences of bacteriophages HK97 and HK022: pervasive genetic mosaicism in the lambdoid bacteriophages. Juhala RJ1, Ford ME, Duda RL, Youlton A, Hatfull GF, Hendrix RW); HK97p58 of phage HK97 (J Mol Biol. 2000 May 26;299(1):27-51. Genomic sequences of bacteriophages HK97 and HK022: pervasive genetic mosaicism in the lambdoid bacteriophages. Juhala RJ1, Ford ME, Duda RL, Youlton A, Hatfull GF, Hendrix RW); HK620p36 of phage HK620 (Nucleotide sequence of coliphage HK620 and the evolution of lambdoid phages. Clark AJ, Inwood W, Cloutier T, Dhillon TS. J Mol Biol. 2001 Aug 24;311(4):657-79); VIP0007 of phage E1 (Molecular characterization of the Salmonella enterica serovar Typhi Vi-typing bacteriophage E1. Pickard D, Thomson NR, Baker S, Wain J, Pardo M, Goulding D, Hamlin N, Choudhary J, Threfall J, Dougan G. J Bacteriol. 2008 Apr;190(7):2580-7); Sf6p62 of phage SF6 (J Mol Biol. 2004 May 28;339(2):379-94. The chromosome of Shigella flexneri bacteriophage Sf6: complete nucleotide sequence, genetic mosaicism, and DNA packaging. Casjens S1, Winn-Stapley DA, Gilcrease EB, Morona R, Kühlewein C, Chua JE, Manning PA, Inwood W, Clark AJ); R (SfVp40) of phage SFV (J Bacteriol. 2002 Apr;184(7):1974-87. Complete genomic sequence of SfV, a serotype-converting temperate bacteriophage of Shigella flexneri. Allison GE1, Angeles D, Tran-Dinh N, Verma NK); gp22 of phage BCEPC6B (Summer,E.J., Christian,B.N., Collins,J., Morrison,W., Patel,P., Wells,W., Mebane,L., Gonzalez,C.F. and Young,R.F. GenBank: AAT38381.1); Nazgul38 of phage BCEPNAZGUL (Summer,E.J., Peek,M.L., Haliburton,J.R., Hall,E., Heusinkveld,K., Simser,J., No,E.G., Gonzalez,C.F. and Young,R.F. NCBI Reference Sequence: NP_918971.2); K (P2p09) of phage P2 (Christie,G.E., Haggard-Ljungquist,E. and Calendar,R. NCBI Reference Sequence: NP_046765.1); K (Wphi09) of phage WΦ (Esposito,D., Schmidt,B.J., Bloom,F.R. and Christie,G.E. GenBank: AAN28227.1); rv5_gp085 of phage RV5 (Virol J. 2013 Mar 6;10:76. The host-range, genomics and proteomics of Escherichia coli 0157:H7 bacteriophage rV5. Kropinski AM1, Waddell T, Meng J, Franklin K, Ackermann HW, Ahmed R, Mazzocco A, Yates J 3rd, Lingohr EJ, Johnson RP); EpJS98_gp116 of phage JS98 (Zuber,S., Ngom-Bru,C., Barretto,C., Bruttin,A., Brussow,H. and Denou,E. Genome analysis of phage JS98 defines a fourth major subgroup of T4-like phages in Escherichia coli J. Bacteriol. 189 (22), 8206-8214 (2007)); gp3.5 of phage 13A (Savalia,D., Severinov,K. and Molineux,I. NCBI Reference Sequence: YP_002003950.1); gp3.5 of phage BA14 (Savalia,D., Severinov,K. and Molineux,I. GenBank: ACF15743.1); gp3.5 of phage ECODS1 (Savalia,D., Severinov,K. and Molineux,I. GenBank: ACF15800.1); CKV1F_gp16 of phage K1F (Scholl,D. and Merril,C. The Genome of Bacteriophage K1F, a T7-Like Phage That Has Acquired the Ability To Replicate on K1 Strains of Escherichia coli J. Bacteriol. 187 (24), 8499-8503 (2005)); T3p18 of phage T3 (Pajunen,M.I., Elizondo,M.R., Skurnik,M., Kieleczawa,J. and Molineux,I.J. Complete nucleotide sequence and likely recombinatorial origin of bacteriophage T3 J. Mol. Biol. 319 (5), 1115-1132); gh-1p12 of phage GH-1 (Kovalyova,I.V. and Kropinski,A.M. The complete genomic sequence of lytic bacteriophage gh-1 infecting Pseudomonas putida--evidence for close relationship to the T7 group Virology 311 (2), 305-315 (2003)); gp3.5 of phage K11 (Savalia,D., Severinov,K. and Molineux,I. NCBI Reference Sequence: YP_002003804.1); ORF12 of phage ΦCTX (Nakayama,K., Kanaya,S., Ohnishi,M., Terawaki,Y. and Hayashi,T. The complete nucleotide sequence of phi CTX, a cytotoxin-converting phage of Pseudomonas aeruginosa: implications for phage evolution and horizontal gene transfer via bacteriophages Mol. Microbiol. 31 (2), 399-419 (1999)); Bcep43-27 of phage BCEP43 (Summer,E.J., Gonzalez,C.F., Bomer,M., Carlile,T., Embry,A., Kucherka,A.M., Lee,J., Mebane,L., Morrison,W.C., Mark,L., King,M.D., LiPuma,J.J., Vidaver,A.K. and Young,R. Divergence and mosaicism among virulent soil phages of the Burkholderia cepacia complex J. Bacteriol. 188 (1), 255-268 (2006)); Bcep781-27 of phage BCEP781 (Summer,E.J., Gonzalez,C.F., Bomer,M., Carlile,T., Embry,A., Kucherka,A.M., Lee,J., Mebane,L., Morrison,W.C., Mark,L., King,M.D., LiPuma,J.J., Vidaver,A.K. and Young,R. Divergence and mosaicism among virulent soil phages of the Burkholderia cepacia complex J. Bacteriol. 188 (1), 255-268 (2006)); Bcep1-28 of phage BCEP1 (Summer,E.J., Gonzalez,C.F., Bomer,M., Carlile,T., Embry,A., Kucherka,A.M., Lee,J., Mebane,L., Morrison,W.C., Mark,L., King,M.D., LiPuma,J.J., Vidaver,A.K. and Young,R. Divergence and mosaicism among virulent soil phages of the Burkholderia cepacia complex J. Bacteriol. 188 (1), 255-268 (2006)); BcepNY3gene26 of phage BCEPNY3 (GenBank: ABR10561.1 Summer,E.J., Orchard,R.C., Attenhofer,K., Coffey,A., Gill,J.J., Gonzalez,C.F. and Young,R.); gp45 of phage ΦE12-2 (NCBI Reference Sequence: YP_001111195.1 DeShazer,D., Ronning,C.M., Brinkac,L.M. and Nierman,W.C.); gp28 of phage Φ52237 (DeShazer, D and Nierman, W.C. NCBI Reference Sequence: YP_293741.1 DeShazer,D., Ronning,C.M., Brinkac,L.M. and Nierman,W.C.); P27p30 of phage ΦP27 (Recktenwald,J. and Schmidt,H. The nucleotide sequence of Shiga toxin (Stx) 2e-encoding phage phiP27 is not related to other Stx phage genomes, but the modular genetic structure is conserved Infect. Immun. 70 (4), 1896-1908 (2002)); RB49p102 of phage RB49 (Monod,C., Repoila,F., Kutateladze,M., Tetart,F. and Krisch,H.M. The genome of the pseudo T-even bacteriophages, a diverse group that resembles T4 J. Mol. Biol. 267 (2), 237-249 (1997)); phi1-p102 of phage Φ1 (Arbiol,C., Comeau,A.M., Kutateladze,M., Adamia,R. and Krisch,H.M. Mobile regulatory cassettes mediate modular shuffling in t4-type phage genomes Genome Biol Evol 2010, 140-152 (2010)); lys (T5.040) of phage T5 (NCBI Reference Sequence: YP_006868.1 Ksenzenko,V.N., Kaliman,A.V., Krutilina,A.I. and Shlyapnikov,M.G.); YP_001956952.1 of phage 201phi2-1 (Thomas,J.A., Rolando,M.R., Carroll,C.A., Shen,P.S., Belnap,D.M., Weintraub,S.T., Serwer,P. and Hardies,S.C. Characterization of Pseudomonas chlororaphis myovirus 201varphi2-1 via genomic sequencing, mass spectrometry, and electron microscopy Virology 376 (2), 330-338 (2008)); Aehlp339 of phage Aeh1 (NCBI Reference Sequence: NP_944217.1 Petrov,V., Nolan,J., Bertrand,C., Letarov,A.V., Krisch,H.M. and Karam,J.D); YYZgp45 of phage YYZ-2008 (GenBank: ACI32381.1 Zhang,Y., Laing,C.R., Kropinski,A. and Gannon,V.J.P.).

Also preferred is the endolysin of the *Pseudomonas aeruginosa* phages ΦKZ, gp144 (J Biol Chem. 2008 Mar 14;283(11):7242-50. Structure of the bacteriophage phi KZ lytic transglycosylase gp144. Fokine A, Miroshnikov KA, Shneider MM, Mesyanzhinov VV, Rossmann MG.), and EL, EL188 (Mol Microbiol. 2007 Sep;65(5):1334-44. Muralytic activity and modular structure of the endolysins of Pseudomonas aeruginosa bacteriophages phiKZ and EL. Briers Y1, Volckaert G, Cornelissen A, Lagaert S, Michiels CW, Hertveldt K, Lavigne R), of the phage LUZ24 (NCBI Reference Sequence: YP_001671940.1) as well as of the *E. coli* phage N4gp61 (J Mol Biol. 2007 Feb 16;366(2):406-19. Coliphage N4 N-acetylmuramidase defines a new family of murein hydrolases. Stojkovic EA1, Rothman-Denes LB), STM0016 endolysin (NCBI Reference Sequence: NP_459021.1), PSP3 endolysin (NP_958065.1) and endolysin of *Salmonella enteritis* phage PVPSE1 (PVP-SE1gp146 (YP_004893953.1)).

Further preferred endolysins are Listeria phage endolysins PlyA118 (NCBI Reference Sequence: YP_008666952.1), PlyA500 (NCBI Reference Sequence: YP_001468411.1), PlyPSA (GenBank: CAC85577.1 and J Mol Biol. 2006 Dec 8;364(4):678-89. The crystal structure of the bacteriophage PSA endolysin reveals a unique fold responsible for specific recognition of Listeria cell walls. Korndörfer IP1, Danzer J, Schmelcher M, Zimmer M, Skerra A, Loessner MJ), PlyA511 (NCBI Reference Sequence: YP_001468459.1), PlyP35 (GenBank: AAY53213.1), PlyP40 (NCBI Reference Sequence: YP_002261442.1), Staphylococcal phage Phi 11 endolysin (Lytic activity of recombinant bacteriophage phi11 and phi12 endolysins on whole cells and biofilms of Staphylococcus aureus. Sass P, Bierbaum G. Appl Environ Microbiol. 2007 Jan;73(1):347-52), Phi MR11 endolysin (NCBI Reference Sequence: YP_001604156.1), LysK (The recombinant phage lysin LysK has a broad spectrum of lytic activity against clinically relevant staphylococci, including methicillin-resistant Staphylococcus aureus. O'Flaherty S, Coffey A, Meaney W, Fitzgerald GF, Ross RP.J Bacteriol. 2005 Oct;187(20):7161-4), *Clostridium perfringens* PlyS9 (WO2010003943 (A1; Bacteriophage. 2012 Apr 1;2(2):89-97. Inducible Clostridium perfringens bacteriophages ΦS9 and ΦS63: Different genome structures and a fully functional sigK intervening element. Kim KP1, Born Y, Lurz R, Eichenseher F, Zimmer M, Loessner MJ, Klumpp J.), Ply3626 (Zimmer,M., Scherer,S. and Loessner,M.J. Genomic analysis of Clostridium perfringens bacteriophage phi3626, which integrates into guaA and possibly affects sporulation J. Bacteriol. 184 (16), 4359-4368 (2002)), *Clostridium difficile:* CD27L endolysin (J Bacteriol. 2008 Oct;190(20):6734-40. Molecular characterization of a Clostridium difficile bacteriophage and its cloned biologically active endolysin. Mayer MJ1, Narbad A, Gasson MJ), Streptococcus: B30 endolysin (The bifunctional peptidoglycan lysin of Streptococcus agalactiae bacteriophage B30. Pritchard DG, Dong S, Baker JR, Engler JA. Microbiology. 2004 Jul;150(Pt 7):2079-87), phage Dp-1 encoded Pal amidase (J Biol Chem. 2004 Oct 15;279(42):43697-707. Structural and thermodynamic characterization of Pal, a phage natural chimeric lysin active against pneumococci. Varea J1, Monterroso B, Sáiz JL, López-Zumel C, Garcia JL, Laynez J, Garcia P, Menéndez M.), C1 endolysin PlyC (PlyC: a multimeric bacteriophage lysin. Nelson D, Schuch R, Chahales P, Zhu S, Fischetti VA. Proc Natl Acad Sci U S A. 2006 Jul 11;103(28):10765-70), Cpl-1 endolysin (Gene. 1990 Jan 31;86(1):81-8. Modular organization of the lytic enzymes of Streptococcus pneumoniae and its bacteriophages. Garcia P1, Garcia JL, Garcia E, Sanchez-Puelles JM, Lopez R.), PlyGBS (Antimicrob Agents Chemother. 2005 Jan;49(1):111-7. Removal of group B streptococci colonizing the vagina and oropharynx of mice with a bacteriophage lytic enzyme. Cheng Q1, Nelson D, Zhu S, Fischetti VA.), Enterococccus: PlyV12 (J Bacteriol. 2004 Jul;186(14):4808-12. Identification of a broadly active phage lytic enzyme with lethal activity against antibiotic-resistant Enterococcus faecalis and Enterococcus faecium. Yoong P1, Schuch R, Nelson D, Fischetti VA.), *Bacillus anthracis:* Phage gamma endolysin PlyG (Nature. 2002 Aug 22;418(6900):884-9. A bacteriolytic agent that detects and kills Bacillus anthracis. Schuch R, Nelson D, Fischetti VA.).

More preferred endolysins are listed as SEQ ID NO: 1 - 11.

A polypeptide for use of the present invention may comprise additionally to the peptide hydrolase at least one amino acid sequence stretch, e.g. an amino acid sequence stretch selected from the group consisting of amphipathic peptide, cationic peptide, polycationic peptide, hydrophobic peptide, or naturally occurring antimicrobial peptide, like sushi peptide and defensin. This additional at least one amino acid sequence stretch may be present at any position in the polypeptide of to the present invention, as long as it will not disrupt the peptidoglycan hydrolyzing activity, but is preferably present at the termini, i.e. in the Nand/or C-terminal region of the polypeptide of to the present invention. Such additional amino acid sequence stretch may be fused directly, or via a peptide linker, to the rest of the polypeptide, e.g. the peptidoglycan hydrolase.

More preferred are cationic and/or polycationic amino acid sequence stretches comprising at least one motive according to SEQ ID NO: 13 (KRKKRK). In particular cationic amino acid sequence stretches comprising at least 2, 3, 4, 5, 6, 7, 8, 9, 10, 11, 12, 13, 14, 15, 16 or 17 motives according to SEQ ID NO: 13 (KRKKRK) are preferred. More preferred are cationic peptide stretches comprising at least one KRK motive (lys-arg-lys), preferable at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32 or 33 KRK motives.

In another preferred embodiment of the present invention the cationic amino acid sequence stretch comprises beside the positively charged amino acid residues, in particular lysine and/or arginine residues, neutrally charged amino acid residues, in particular glycine and/or serine residues. Preferred are cationic amino acid sequence stretches consisting of about 70 % to about 100 %, or about 80 % to about 95 %, or about 85 % to about 90 % positively charged amino acid residues, in particular lysine, arginine and/or histidine residues, more preferably lysine and/or arginine residues and of about 0 % to about 30 %, or about 5 % to about 20 %, or about 10 % to about 20 % neutrally charged amino acid residues, in particular glycine and/or serine residues. Preferred are amino acid sequence stretches consisting of about 4 % to about 8 % serine residues, of about 33 % to about 36 % arginine residues and of about 56 % to about 63 % lysine residues. Especially preferred are amino acid sequence stretches comprising at least one motive according to SEQ ID NO: 14 (KRXKR), wherein X is any other amino acid than lysine, arginine and histidine. Especially preferred are polypeptide stretches comprising at least one motive according to SEQ ID NO: 15 (KRSKR). More preferred are cationic stretches comprising at least about 2, 3, 4, 5, 6, 7, 8, 9, 10, 11, 12, 13, 14, 15, 16, 17, 18, 19 or at least about 20 motives according to SEQ ID NO: 14 (KRXKR) or SEQ ID NO: 15 (KRSKR).

Also preferred are amino acid sequence stretches consisting of about 9 to about 16 % glycine residues, of about 4 to about 11 % serine residues, of about 26 to about 32 % arginine residues and of about 47 to about 55 % lysine residues. Especially preferred are amino acid sequence stretches comprising at least one motive according to SEQ ID NO: 16 (KRGSG). More preferred are cationic stretches comprising at least about 2, 3, 4, 5, 6, 7, 8, 9, 10, 11, 12, 13, 14, 15, 16, 17, 18, 19 or at least bout 20 motives according to SEQ ID NO: 16 (KRGSG).

In another preferred embodiment of the present invention such cationic amino acid sequence stretch comprises beside the positively charged amino acid residues, in particular lysine and/or arginine residues, hydrophobic amino acid residues, in particular valine, isoleucine, leucine, methionine, phenylalanine, tryptophan, cysteine, alanine, tyrosine, proline and glycine residues, more preferably alanine, valine, leucine, isoleucine, phenylalanine, and/or tryptophan residues. Preferred are cationic amino acid sequence stretches consisting of about 70 % to about 100 %, or about 80 % to about 95 %, or about 85 % to about 90 % positively charged amino acid residues, in particular lysine and/or arginine residues and of about 0 % to about 30 %, or about 5 % to about 20 %, or about 10 % to about 20 % hydrophobic amino acid residues, valine, isoleucine, leucine, methionine, phenylalanine, tryptophan, cysteine, alanine, tyrosine, proline and glycine residues, more preferably alanine, valine, leucine, isoleucine, phenylalanine, and/or tryptophan residues.

Examples for cationic and polycationic amino acid sequence stretches are listed in the following table:

**Table 2:**

| **amino acid sequence stretch** | **length** | **SEQ ID NO:** |
|---|---|---|
| KRKKRK | 6 | SEQ ID NO: 13 |
| KRKKRKKRK | 9 | SEQ ID NO: 17 |
| RRRRRRRRR | 9 | SEQ ID NO: 18 |
| KKKKKKKK | 8 | SEQ ID NO: 19 |
| KRKKRKKRKK | 10 | SEQ ID NO: 20 |
| KRKKRKKRKKRK | 12 | SEQ ID NO: 21 |
| KRKKRKKRKKRKKR | 14 | SEQ ID NO: 22 |
| KKKKKKKKKKKKKKKK | 16 | SEQ ID NO: 23 |
| KRKKRKKRKKRKKRKKRK | 18 | SEQ ID NO: 24 |
| KRKKRKKRKKRKKRKKRKK | 19 | SEQ ID NO: 25 |
| RRRRRRRRRRRRRRRRRRR | 19 | SEQ ID NO: 26 |
| KKKKKKKKKKKKKKKKKKK | 19 | SEQ ID NO: 27 |
| KRKKRKKRKRSKRKKRKKRK | 20 | SEQ ID NO: 28 |
| KRKKRKKRKRSKRKKRKKRKK | 21 | SEQ ID NO: 29 |
| KRKKRKKRKKRKKRKKRKKRK | 21 | SEQ ID NO: 30 |
| KRKKRKKRKRGSGKRKKRKKRK | 22 | SEQ ID NO: 31 |
| KRKKRKKRKRGSGSGKRKKRKKRK | 24 | SEQ ID NO: 32 |
| KRKKRKKRKKRKKRKKRKKRKKRKK | 25 | SEQ ID NO: 33 |
| KRKKRKKRKRSKRKKRKKRKRSKRKKRKKRK | 31 | SEQ ID NO: 34 |
| KRKKRKKRKRGSGSGKRKKRKKRKGSGSGKRKKRKKRK | 38 | SEQ ID NO: 35 |
| KRKKRKKRKKRKKRKKRKKRKKRKKRKKRKKRKKRKKRK | 39 | SEQ ID NO: 36 |
| KRKKRKKRKRSKRKKRKKRKRSKRKKRKKRKRSKRKKRKKRK | 42 | SEQ ID NO: 37 |

In a further aspect of the present invention at least one of the additional amino acid sequence stretches is an antimicrobial peptide, which comprises a positive net charge and around 50 % hydrophobic amino acids. The antimicrobial peptides are amphipathic with a length of about 12 to about 50 amino acid residues. The antimicrobial peptides are naturally occurring in insects, fish, plants, arachnids, vertebrates or mammals. Preferably the antimicrobial peptide may be naturally occurring in radish, silk moth, wolf spider, frog, preferably in *Xenopus laevis, Rana* frogs, more preferably in *Rana catesbeiana,* toad, preferably Asian toad *Bufo bufo gargarizans,* fly, preferably in *Drosophila,* more preferably in *Drosophila melanogaster,* in *Aedes aegypti,* in honey bee, bumblebee, preferably in *Bombus pascuorum,* flesh fly, preferably in *Sarcophaga peregrine,* scorpion, horseshoe crab, catfish, preferably in *Parasilurus asotus,* cow, pig, sheep, porcine, bovine, monkey and human.

In another preferred embodiment of the present invention the antimicrobial amino acid sequence stretches consist of about 0 % to about 5 %, or about 0 % to about 35 %, or about 10 % to about 35 % or about 15 % to about 45 %, or about 20 % to about 45 % positively charged amino acid residues, in particular lysine and/or arginine residues and of about 50 % to about 80 %, or about 60 % to about 80 %, or about 55 % to about 75 %, or about 70 % to about 90 % hydrophobic amino acid residues, valine, isoleucine, leucine, methionine, phenylalanine, tryptophan, cysteine, alanine, tyrosine, proline and glycine residues, more preferably alanine, valine, leucine, isoleucine, phenylalanine, and/or tryptophan residues.

In another preferred embodiment of the present invention the antimicrobial amino acid sequence stretches consist of about 4 % to about 58 % positively charged amino acid residues, in particular lysine and/or arginine residues and of about 33 % to about 89 % hydrophobic amino acid residues, valine, isoleucine, leucine, methionine, phenylalanine, tryptophan, cysteine, alanine, tyrosine, proline and glycine residues, more preferably alanine, valine, leucine, isoleucine, phenylalanine, and/or tryptophan residues.

Examples for antimicrobial amino acid sequences which may be used in carrying out the present invention are listed in the following table.

**Table 3:**

| **Peptide** | **Sequence** | **SEQ ID NO** |
|---|---|---|
| LL-37 | | SEQ ID NO: 38 |
| SMAP-29 | RGLRRLGRKIAHGVKKYGPTVLRIIRIAG | SEQ ID NO: 39 |
| Indolicidin | ILPWKWPWWPWRR | SEQ ID NO: 40 |
| Protegrin | RGGRLCYCRRRFCVCVGR | SEQ ID NO: 41 |
| Cecropin P1 | SWLSKTAKKLENSAKKRISEGIAIAIQGGPR | SEQ ID NO: 42 |
| Magainin | GIGKFLHSAKKFGKAFVGEIMNS | SEQ ID NO: 43 |
| Pleurocidin | GWGSFFKKAAHVGKHVGKAALTHYL | SEQ ID NO: 44 |
| Cecropin A (A. aegypti) | | SEQ ID NO: 45 |
| Cecropin A (D. melanogaster) | | SEQ ID NO: 46 |
| Buforin II | TRSSRAGLQFPVGRVHRLLRK | SEQ ID NO: 47 |
| Sarcotoxin IA | | SEQ ID NO: 48 |
| Apidaecin | ANRPVYIPPPRPPHPRL | SEQ ID NO: 49 |
| Ascaphine 5 | GIKDWIKGAAKKLIKTVASHIANQ | SEQ ID NO: 50 |
| Nigrocine 2 | GLLSKVLGVGKKVLCGVSGLVC | SEQ ID NO: 51 |
| Pseudin 1 | GLNTLKKVFQGLHEAIKLINNHVQ | SEQ ID NO: 52 |
| Ranalexin | FLGGLIVPAMICAVTKKC | SEQ ID NO: 53 |
| Melittin | GIGAVLKVLTTGLPALISWIKRKRQQ | SEQ ID NO: 54 |
| Lycotoxin 1 | IWLTALKFLGKHAAKKLAKQQLSKL | SEQ ID NO: 55 |
| Parasin 1 | KGRGKQGGKVRAKAKTRSS | SEQ ID NO: 56 |
| Buforin I | | SEQ ID NO: 57 |
| Dermaseptin 1 | ALWKTMLKKLGTMALHAGKAALGAAADTISQGTQ | SEQ ID NO: 58 |
| Bactenecin 1 | RLCRIVVIRVCR | SEQ ID NO: 59 |
| Thanatin | GSKKPVPIIYCNRRTGKCQRM | SEQ ID NO: 60 |
| Brevinin 1T | VNPIILGVLPKVCLITKKC | SEQ ID NO: 61 |
| Ranateurin 1 | SMLSVLKNLGKVGLGFVACKINIKQC | SEQ ID NO: 62 |
| Esculentin 1 | | SEQ ID NO: 63 |
| Tachyplesin | RWCFRVCYRGICYRKCR | SEQ ID NO: 64 |
| Androctonin | RSVCRQIKICRRRGGCYYKCTNRPY | SEQ ID NO: 65 |
| alphadefensin | DCYCRIPACIAGERRYGTCIYQGRLWAFCC | SEQ ID NO: 66 |
| beta-defensin | | SEQ ID NO: 67 |
| theta-defensin | GFCRCLCRRGVCRCICTR | SEQ ID NO: 68 |
| defensin (sapecin A) | | SEQ ID NO: 69 |
| Thionin (crambin) | | SEQ ID NO: 70 |
| defensin from radish | | SEQ ID NO: 71 |
| Drosomycin | | SEQ ID NO: 72 |
| Hepcidin | DTHFPICIFCCGCCHRSKCGMCCKT | SEQ ID NO: 73 |
| Bac 5 | | SEQ ID NO: 74 |
| PR-39 | | SEQ ID NO: 75 |
| Pyrrhocoricin | VDKGSYLPRPTPPRPIYNRN | SEQ ID NO: 76 |
| Histatin 5 | DSHAKRHHGYKRKFHEKHHSHRGY | SEQ ID NO: 77 |
| Macedocin | GKNGVFKTISHECHLNTWAFLATCCS | SEQ ID NO: 78 |
| Macedocin (Trunc) | GKNGVFKTISHECHLNTWAFLA | SEQ ID NO: 79 |
| D16 | ACKLKSLLKTLSKAKKKKLKTLLKALSK | SEQ ID NO: 80 |
| CPF-C1 | GFGSLLGKALRLGANVL | SEQ ID NO: 81 |
| TL-ColM | ETLTVHAPSPSTNLPSYGNGAFSLSAPHVPGAGP | SEQ ID NO: 82 |
| TM-174E | LISKGWPYLLVVVLGATIYFWGNSNG | SEQ ID NO: 83 |
| MSI-594 | GIGKFLKKAKKGIGAVLKVLTTG | SEQ ID NO: 84 |
| ECP19 | RPPQFTRAQWFAIQHISLN | SEQ ID NO: 85 |
| ECP45 | | SEQ ID NO: 86 |
| ColicinE3_1-51 (S37F) | | SEQ ID NO: 87 |
| ColicinE3_1-69 (S37F) | | SEQ ID NO: 88 |
| ColicinD_1-53 | | SEQ ID NO: 89 |

The amino acid sequence stretch may be selected for example from LL-37, SMAP-29, Indolicidin, Protegrin, Cecropin P1, Magainin, Pleurocidin, Cecropin A (A.aegypti), Cecropin A (D. melanogaster), Buforin II, Sarcotoxin IA, Apidaecin, Ascaphine 5, Nigrocine 2, Pseudin 1, Ranalexin, Melittin, Lycotoxin 1, Parasin 1, Buforin I, Dermaseptin 1, Bactenecin 1, Thanatin, Brevinin 1T, Ranateurin 1, Esculentin 1, Tachyplesin, Androctonin, alphadefensin, beta-defensin, theta-defensin, defensin (sapecin A), Thionin (crambin), defensin from radish, Drosomycin, Hepcidin, Bac 5, PR-39, Pyrrhocoricin or Histatin 5.

In a further aspect of the present invention at least one of the additional amino acid sequence stretches may be a sushi peptide which is described by Ding JL, Li P, Ho B Cell Mol Life Sci. 2008 Apr;65(7-8):1202-19. The Sushi peptides: structural characterization and mode of action against Gram-negative bacteria. Especially preferred is the sushi 1 peptide according to SEQ ID NO: 90. Preferred sushi peptides are sushi peptides S1 and S3 and multiples thereof; FASEB J. 2000 Sep;14(12):1801-13.

In a further aspect of the present invention at least one of the additional amino acid sequence stretches is a hydrophobic peptide, which comprises at least 90 % of the hydrophobic amino acid residues of valine, isoleucine, leucine, methionine, phenylalanine, tryptophan, cysteine, alanine, tyrosine, proline and/or glycine. In another preferred embodiment the hydrophobic peptide fused to the protein of the invention consists of about 90 % to about 95 %, or of about 90 % to about 100%, or of about 95 % to about 100 % of the hydrophobic amino acid residues of valine, isoleucine, leucine, methionine, phenylalanine, tryptophan, cysteine, alanine, tyrosine, proline and/or glycine.

Preferred hydrophobic peptides are Walmagh1 having the amino acid sequence according to SEQ ID NO: 91 and the hydrophobic peptide having the amino acid sequence Phe-Phe-Val-Ala-Pro (SEQ ID NO: 92).

In a further aspect of the present invention at least one of the additional amino acid sequence stretches is an amphipathic peptide, which comprises one or more of the positively charged amino acid residues of lysine, arginine and/or histidine, combined to one or more of the hydrophobic amino acid residues of valine, isoleucine, leucine, methionine, phenylalanine, tryptophan, cysteine, alanine, tyrosine, proline and/or glycine. Side chains of the amino acid residues are oriented in order that cationic and hydrophobic surfaces are clustered at opposite sides of the peptide. Preferably, more than about 30, 40, 50, 60 or 70 % of the amino acids in said peptide are positively charged amino acids. Preferably, more than about 30, 40, 50, 60 or 70 %, of the amino acid residues in said peptide are hydrophobic amino acid residues. Advantageously, the amphipathic peptide is present at the N-terminal or the C-terminal end of the polypeptide of to the present invention.

In another embodiment of the invention, the amphipathic peptide consists of at least 5, more preferably at least of 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or at least 50 amino acid residues. In a preferred embodiment at least about 30, 40, 50, 60 or 70 % of said amino acid residues of the amphipathic peptide are either arginine or lysine residues and/or at least about 30, 40, 50, 60 or 70 % of said amino acid residues of the amphipathic peptide are of the hydrophobic amino acids valine, isoleucine, leucine, methionine, phenylalanine, tryptophan, cysteine, alanine, tyrosine, proline and/or glycine.

In another preferred embodiment of the present invention the amphipathic peptide stretch comprises beside the positively charged amino acid residues, in particular lysine and/or arginine residues, hydrophobic amino acid residues, in particular valine, isoleucine, leucine, methionine, phenylalanine, tryptophan, cysteine, alanine, tyrosine, proline and glycine residues, more preferably alanine, valine, leucine, isoleucine, phenylalanine, and/or tryptophan residues. Preferred are amphipathic peptide stretches consisting of about 10 % to about 50 %, or about 20 % to about 50 %, or about 30 % to about 45 % or about 5 % to about 30 % positively charged amino acid residues, in particular lysine and/or arginine residues and of about 50 % to about 85 %, or about 50 % to about 90 %, or about 55 % to about 90 %, or about 60 % to about 90 %, or about 65 % to about 90 % hydrophobic amino acid residues, valine, isoleucine, leucine, methionine, phenylalanine, tryptophan, cysteine, alanine, tyrosine, proline and glycine residues, more preferably alanine, valine, leucine, isoleucine, phenylalanine, and/or tryptophan residues. In another preferred embodiment amphiphatic peptide stretches consisting of 12 % to about 50 % positively charged amino acid residues, in particular lysine and/or arginine residues and of about 50 % to about 85 % hydrophobic amino acid residues, valine, isoleucine, leucine, methionine, phenylalanine, tryptophan, cysteine, alanine, tyrosine, proline and glycine residues, more preferably alanine, valine, leucine, isoleucine, phenylalanine, and/or tryptophan residues.

Preferred amphipathic peptides are α4-helix of T4 lysozyme according to SEQ ID NO: 93 and WLBU2-Variant having the amino acid sequence according to SEQ ID NO: 94 and Walmagh 2 according to SEQ ID NO: 95.

The amino acid sequence stretch may be selected for example from the group of SEQ ID NO: 13-95.

In a preferred embodiment of the present invention the polypeptide of to the present invention comprises two or more amino acid sequence stretches as defined herein. If the polypeptide of to the present invention comprises more than one of these additional amino acid sequence stretches, then it preferably comprises at least two distinct amino acid sequence stretches, preferably selected from the group of amphipathic peptide, cationic peptide, polycationic peptide, hydrophobic peptide, or naturally occurring antimicrobial peptide, like sushi peptide and defensin. The two or more amino acid sequence stretches, e.g. at the N- or C-terminus of the enzyme and/or at the N- or C-terminus of the polypeptide may thus be two or more distinct cationic peptides or two or more distinct polycationic peptides or two or more distinct antimicrobial peptides or two or more distinct amphipathic peptides or two or more distinct hydrophobic peptides. The two or more amino acid sequence stretches may in the alternative also be any combination of two or more peptides selected from different representatives of the group consisting of: cationic peptide, a polycationic peptide, a hydrophobic peptide, an antimicrobial peptide, a sushi peptide, a defensin and an amphipathic peptide. For example, a cationic peptide could be combined with an antimicrobial peptide.

The optional additional amino acid sequence stretches as specified above consist preferably of at least 5, more preferably at least of 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or at least 100 amino acid residues. Especially preferred are those additional amino acid sequence stretches consisting of about 5 to about 100 amino acid residues, about 5 to about 50 or about 5 to about 30 amino acid residues. More preferred are peptide stretches consisting of about 6 to about 42 amino acid residues, about 6 to about 39 amino acid residues, about 6 to about 38 amino acid residues, about 6 to about 31 amino acid residues, about 6 to about 25 amino acid residues, about 6 to about 24 amino acid residues, about 6 to about 22 amino acid residues, about 6 to about 21 amino acid residues, about 6 to about 20 amino acid residues, about 6 to about 19 amino acid residues, about 6 to about 16 amino acid residues, about 6 to about 14 amino acid residues, about 6 to about 12 amino acid residues, about 6 to about 10 amino acid residues or about 6 to about 9 amino acid residues.

In a preferred embodiment the polypeptide of to the present invention comprises at least one, two or more amino acid sequence stretches selected from the group consisting of KRK and SEQ ID NOs: 13-95. Preferably, the polypeptide of to the present invention comprises at least one, two or more amino acid sequence stretches selected from the group consisting of KRK and SEQ ID NOs: 13-95, and an amino acid sequence selected from any one of SEQ ID NOs: 1 to 12, wherein preferably the amino acid sequence stretches, are fused to the N- and/or C-terminus of the amino acid sequence selected from the group consisting of SEQ ID NOs: 1 to 12.

More preferred polypeptides of the invention comprise an amino acid sequence selected from the group consisting of SEQ ID NO: 96-119.

The additional amino acid sequence stretches of the polypeptide comprised in the composition according to the present invention may be linked to the rest of the enzyme by intervening additional amino acid residues e.g. due to cloning reasons. Alternatively, the additional amino acid sequence stretches may be directly linked to the rest of the enzyme sequence without intervening linker sequences. The additional amino acid sequences, if more than one present in the polypeptide of to the present invention and positioned on the same terminus of the enzyme, may likewise be linked to each other by additional intervening amino acid residues or may be directly joined to each other.

Preferably, said intervening additional amino acid residues may not be recognized and/or cleaved by proteases. Preferably said additional amino acid sequences are linked to each other and/or to the enzyme by at least 1, 2, 3, 4, 5, 6, 7, 8, 9 or 10 additional intervening amino acid residues.

In a preferred embodiment the at least one additional amino acid sequence stretch is linked to the rest of the polypeptide of to the present invention, preferably at the N- or C-terminus of the polypeptide of to the present invention, by the additional intervening amino acid residues glycine and serine (Gly-Ser), glycine, serine and serine (Gly-Ser-Ser), glycine, alanine, glycine and alanine (Gly-Ala-Gly-Ala; SEQ ID NO: 120), glycine, alanine, glycine, alanine, glycine, alanine, glycine and alanine (Gly-Ala-Gly-Ala-Gly-Ala-Gly-Ala; SEQ ID NO: 121) or glycine, alanine, glycine, alanine, glycine, alanine, glycine, alanine, glycine, alanine, glycine and alanine (Gly-Ala-Gly-Ala-Gly-Ala-Gly-Ala-Gly-Ala-Gly-Ala; SEQ ID NO: 122).

Aside of the peptidoglycan hydrolase and the optional additional amino acid sequence stretches, as defined herein, the polypeptide of to the present invention may of course also comprise other amino acid sequence elements, e.g. one or more tags, e.g. a His-tag, Strep-tag, Avi-tag, Myc-tag, Gst-tag, JS-tag, cystein-tag, FLAG-tag or other tags known in the art, thioredoxin, maltose binding proteins (MBP) etc.

In this context, the polypeptide of to the present invention, preferably having the ability of degrading the peptidoglycan layer, may additional comprise a tag e.g. for purification.

Preferred is a His₆-tag, preferably at the C-terminus and/or the N-terminus of the polypeptide of to the present invention. Said tag can be linked to the polypeptide by additional amino acid residues e.g. due to cloning reasons. Preferably said tag can be linked to the protein by at least 1, 2, 3, 4, 5, 6, 7, 8, 9 or 10 additional amino acid residues. Preferably said additional amino acid residues may not be recognized and/or cleaved by proteases. In a preferred embodiment the polypeptide of to the present invention comprises a His₆-tag at its C-terminus linked to the polypeptide by the additional amino acid residues lysine and glycine (Lys-Gly) or leucine and glutamic acid (Leu-Glu). Preferably, said additional amino acid residues may be not recognized or cleaved by proteases. In another preferred embodiment the polypeptide of to the present invention comprises a His₆-tag at its N-terminus linked to the polypeptide by the additional amino acid residues lysine and glycine (Lys-Gly) or leucine and glutamic acid (Leu-Glu). In another preferred embodiment the polypeptide comprises a His₆-tag at its Nand C-terminus linked to the polypeptide by the additional amino acid residues lysine and glycine (Lys-Gly) or leucine and glutamic acid (Leu-Glu). A preferred polypeptide of the invention is listed in SEQ ID NO: 123.

The polypeptide and/or the pharmaceutical composition for use of the present invention may be administered to a subject in an effective amount, wherein the subject may be a human being or an animal, preferably a livestock or companion animal. In particular, the polypeptide and/or the pharmaceutical composition of the present invention may be used for the treatment of chronic bacterial infections caused by Gram-negative and/or Gram-positive bacteria. The treatment may comprise the treatment of infections of the skin, of soft tissues, the respiratory system, the lung, the digestive tract, the eye, the ear, the teeth, the nasopharynx, the mouth, the bones, the vagina, of wounds of bacteraemia and/or endocarditis, e.g. caused by Gram-negative and/or Gram-positive bacteria, in particular by the bacteria as mentioned herein.

The dosage and route of administration used for the treatment according to the present invention depends on the specific disease and/or site of infection to be treated. The route of administration may be for example oral, topical, nasopharyngeal, parenteral, intravenous, rectal or any other route of administration.

For application of the polypeptide and/or the pharmaceutical composition for use of the present invention to a site of infection a formulation may be used that protects the active compounds from environmental influences such as proteases, oxidation, immune response etc., until it reaches the site of infection. Therefore, the formulation may be capsule, dragee, pill, powder, suppository, emulsion, suspension, gel, lotion, cream, salve, injectable solution, syrup, spray, inhalant or any other medical reasonable galenic formulation. Preferably, the galenic formulation may comprise suitable carriers, stabilizers, flavourings, buffers or other suitable reagents. For example, for topical application the formulation may be a lotion, cream, gel, salve or plaster, for nasopharyngeal application the formulation may be saline solution to be applied via a spray to the nose. For oral administration in case of the treatment of a specific infection site e.g. in the intestine, it can be necessary to protect polypeptide of to the present invention from the harsh digestive environment of the gastrointestinal tract until the site of infection is reached.

In one aspect of the present invention the subject suffering from a chronic bacterial infection caused by persister bacteria has been treated with antibiotics whereby the symptoms of the bacterial infection has been reduced or cured but the bacterial infection reoccurred after offsetting the antibiotics.

Preferably, the polypeptide and/or the pharmaceutical composition for use of the present invention is used for the treatment of chronic bacterial infections caused by persister bacteria, wherein the infection is caused by multiresistant or antibiotics tolerant bacterial strains, in particular by strains resistant or tolerant against one or more antibiotics selected from the following group: beta-lactams, aminoglycosides, fluoroquinolones, macrolides, novobiocin, rifampicin, oxazolidinones, fusidic acid, mupirocin, pleuromutilins, daptomycin, vancomycin, tetracyclines, sulfonamides, chloramphenicol, trimetoprim, fosfomycin, cycloserine, polymyxin, streptomycin, tetracycline, cephalothin, gentamicin, cefotaxime, cephalosporin, ceftazidime or imipenem. Furthermore, the polypeptide and/or the pharmaceutical composition of the present invention can be used for treatment by administering in combination with conventional antibacterial agents, such as antibiotics.

The polypeptide and/or the pharmaceutical composition of the present invention is used as a medicament for the treatment of a chronic bacterial infection caused by persister bacteria and wherein the chronic bacterial infection is a chronic bacterial infection with *Pseudomonas, Escherichia, Staphylococcus aureus, Acinetobacter, Mycobacterium avium, Mycobacterium tuberculosis, Listeria monocytogenes* and/or *Salmonella* bacteria.

In a specific embodiment of the present invention the polypeptide and/or the pharmaceutical composition for use of the present invention is used as a medicament for the treatment of a chronic bacterial infection caused by Listeria monocytogenes, in particular Granulomatosis infantiseptica (listeriosis of newborns), mononucleosis, conjunctivitis, meningitis, granulomatosis septica and the listeriosis of pregnant women.

In another specific embodiment of the present invention the polypeptide and/or the pharmaceutical composition for use of the present invention is used in the manufacture of a medicament for the treatment of a chronic bacterial infection caused by persister bacteria, and wherein the chronic bacterial infection is a chronic bacterial infection with Staphylococcus aureus, in particular infections of the skin like pyoderma, particularly folliculitis, furuncle, carbuncle, abscesses of the sweat glands and pemphigus, and like scaled skin syndrome. The scaled skin syndrome can appear in three clinical pictures: dermatitis exfoliativa, impetigo bullosa and scarlatiniform erythroderma. Moreover chronic bacterial infection caused by Staphylococcus aureus are Staphylococcus pneumonia, hospitalism, in particular surgical wound infections, mastitis puerperalis and enterokolitis, and food poisonings.

In another specific embodiment of the present invention the polypeptide and/or the pharmaceutical composition for use of the present invention is used in the manufacture of a medicament for the treatment of a chronic bacterial infection caused by persister bacteria, and wherein the chronic bacterial infection is a chronic bacterial infection with Mycobacterium avium, or Mycobacterium tuberculosis, in particular tuberculosis.

In another specific embodiment of the present invention the polypeptide and/or the pharmaceutical composition for use of the present invention is used in the manufacture of a medicament for the treatment of a chronic bacterial infection caused by persister bacteria, and wherein the chronic bacterial infection is a chronic bacterial infection with E. coli , in particular for urinary tract infections, diseases of the digestive tract like Morbus Crohn and Colitis ulcerosa.

In another specific embodiment of the present invention the polypeptide and/or the pharmaceutical composition for use of the present invention is used in the manufacture of a medicament for the treatment of a chronic bacterial infection caused by persister bacteria, and wherein the chronic bacterial infection is a chronic bacterial infection with Pseudomonas, in particular urinary tract infections, respiratory system infections, dermatitis, soft tissue infections, bacteraemia and a variety of systemic infections, particularly in victims of severe burns, and also in cancer and AIDS patients who are immuno-compromised.

In another specific embodiment of the present invention the polypeptide and/or the pharmaceutical composition for use of the present invention is used in the manufacture of a medicament for the treatment of a chronic bacterial infection caused by persister bacteria, and wherein the chronic bacterial infection is a chronic bacterial infection with Acinetobacter, e.g. Acinetobacter baumanii, in particular wound infections, lung infections and meningitis (often also caused by A. lwoffii).

The polypeptide for use of to the present invention can be produced by standard means known in the art, e.g. by recombinant expression of nucleic acids encoding the respective polypeptide in appropriate host cells. If the polypeptide for use of to the present invention comprises for example additionally amino acid sequence stretches or tags, such fusion proteins may be produced by linking the required individual nucleic acid sequences using standard cloning techniques as described e.g. by Sambrook et al. 2001, Molecular Cloning: A Laboratory Manual. Such a polypeptide may be produced likewise with methods known in the art, e.g., in recombinant DNA expression systems.

It has to be noted that the polypeptide for use of to the present invention having peptidoglycan hydrolase activity and thus the activity of degrading the peptidoglycan layer of bacteria can be assembled like using a tool box, i.e. any additional amino acid sequence stretch and antimicrobial peptide disclosed above may be included in the polypeptide for use of the present invention. Consequently, it is possible to construct a suitable polypeptide for any bacteria which should be eliminated.

### Example 1:

Persister killing - Planktonic persister cells were isolated from a stationary phase culture as described e.g. in Chen et al., 2011(Chen X, Zhang M, Zhou C, Kallenbach NR, Ren D. 2011. Control of bacterial persister cells by Trp/Arg-containing antimicrobial peptides. Appl Environ Microbiol 77:4878-4885.). Briefly, an overnight culture of P. aeruginosa PA14 and PA1255 (a cystic fibrosis isolate) was inoculated each in 50 ml 1:20 Trypticase Soy Broth (TSB). After 48h of growth at 37°C, the culture was treated with ofloxacin at a final concentration of 10 µg/ml (5xMIC) for 5 hours at 37°C, while shaking at 200 rpm. The persister cells surviving this antibiotic treatment were isolated by centrifugation (5250g, 15 min, 4°C) and the cell pellet was washed twice with 20 mM HEPES buffer. Prior to treatment, cells were resuspended in the same volume of 20 mM HEPES buffer. A persister killing assay was performed as described e.g. by De Groote et al., 2009 (De Groote VN, Verstraeten N, Fauvart M, Kint CI, Verbeeck AM, Beullens S, Cornelis P, Michiels J. 2009. Novel persistence genes in Pseudomonas aeruginosa identified by high-throughput screening. FEMS Microbiol Lett 297:73-79). Briefly, a volume of 100 µl of the isolated persister fraction was mixed with polypeptides comprising peptidoglycan hydrolase or ciprofloxacin (final concentrations of 10x or 30xMIC against the respective strains), in the absence or presence of 0.5 mM EDTA-Na2 (final concentration) up to a final volume of 200 µl. Control treatments with 20mM HEPES buffer, 0.5 mM EDTA or ofloxacin (30x and 10x MIC) were performed in parallel. The mixtures were shaken (200 rpm) for 1h at 37°C. Treated persisters where washed twice with 20 mM HEPES buffer and appropriate dilutions were plated on TSB agar plates and incubated at 37°C. The number of persisters was determined after 72h incubation to allow complete resuscitation of all surviving persisters. Each experiment was independently repeated at least 3 times.

### Example 2: Materials and methods for examples 3 to 6

### Isolation of persister fraction

Planktonic persister cells were isolated from a stationary-phase culture as described previously (Liebens V, Defraine V, Van der Leyden A, De Groote VN, Fierro C, Beullens S, Verstraeten N, Kint C, Jans A, Frangipani E, Visa P, Marchal K, Versées W, Fauvart M, Michiels J. 2014. A putative de-N-acetylase of the PIG-L superfamily affects fluoroquinolone tolerance in Pseudomonas aeruginosa. Pathogen. Dis. 71:39-54.) with minor modifications. An overnight culture of P. *aeruginosa* PA14, PA1255 (a cystic fibrosis isolate), *Escherichia coli* UTI-89 (isolate from chronic urinary tract infection) and *Acinetobacter baumannii* RUH134 (reference strain for global clone 2) was diluted 100 x in 50 ml 1:20 Trypticase soy broth (TSB). After overnight growth at 37°C, the cultures were treated with either 10 µg/ml ofloxacin *(P. aeruginosa* strains), 200 µg/ml tobramycin (A. *baumannii*) or 200 µg/ml amikacin (*E. coli*) for 5h at 37°C, while shaking at 200 rpm. The antibiotic concentrations were chosen to allow only persister cells to survive, based on previous time-kill curves. The persister fractions were isolated by centrifugation (5,250 × g for 15 min at 4°C), and the cell pellet was washed twice with 20 mM HEPES (pH 7.4). Prior to treatment, the cells were resuspended in a ten-fold reduced volume of 20 mM HEPES (pH 7.4) to concentrate the persisters.

### Persister killing assay

A persister killing assay was performed as described previously (Liebens et al., 2014), with minor modifications. A volume of 100 µl of the isolated persister fraction was mixed with either the polypeptide according to SEQ ID NO: 124, 125, 126 or ciprofloxacin (final concentrations of lOx or 30xMIC against the respective strains), in the absence or presence of 0.5 mM EDTA-Na₂ (final concentration) up to a final volume of 200 µl. Control treatments with 20 mM HEPES (pH 7.4), 0.5 mM EDTA, or ofloxacin/tobramycin/amikacin (30x and 10×MIC) were performed in parallel. The mixtures were shaken (200 rpm) for 1h at 37°C. The treated persister cells were washed twice with 20 mM HEPES (pH 7.4), and the appropriate dilutions were plated on TSB plates and incubated at 37°C. The number of persisters was determined after 72h of incubation to allow for complete resuscitation of all surviving persisters.

### Example 3:

The bactericidal activity of the polypeptide according to SEQ ID NO: 126 was tested against isolated persister fractions of *P. aeruginosa* strains. Persister fractions of two different strains (PA14 and PA1255, an isolate of a cystic fibrosis patient) were collected after 5h exposure to 5× MIC ofloxacin. The lack of killing after further exposure of the isolated fractions to increased ofloxacin concentrations (10x and 30xMIC) confirmed the persistent (antibiotictolerant) nature of both isolated persister fractions. The persisters were incubated with different concentrations of the polypeptide according to SEQ ID NO: 126 or ciprofloxacin (10x MIC and 30xMIC) in the absence or presence of 0.5 mM EDTA. The polypeptide according to SEQ ID NO: 126 (10x and 30xMIC) completely eradicated the remaining persisters in the presence of 0.5 mM EDTA, whereas similar doses of ciprofloxacin (with and without EDTA) or 0.5 mM EDTA alone did not affect the persisters. In the absence of EDTA, a 30xMIC dose of the polypeptide according to SEQ ID NO: 126 reduced the persister fractions of PA14 and PA1255 severely. In summary, the overall activity of the polypeptide according to SEQ ID NO: 126 against these persister fractions is extremely pronounced.

### Example 4:

The killing activity of the polypeptide according to SEQ ID NO: 126 (30xMIC) was evaluated against A. *baumannii* RUH persisters. Those were isolated after a 5h treatment with tobramycin. Aside of the polypeptide according to SEQ ID NO: 126, also the effect of the polypeptide according to SEQ ID NO: 125 (30xMIC) was evaluated. Ciprofloxacin (30xMIC) was used as a control antibiotic. Both polypeptides, i.e. the polypeptide according to SEQ ID NO: 126 as well as the polypeptide according to SEQ ID NO: 125, and ciprofloxacin were tested in the absence and presence of 0.5 mM EDTA. Tobramycin (30xMIC) and 0.5 mM EDTA were added as a control. The polypeptide according to SEQ ID NO: 126 (30xMIC) reduced the number of persisters with approximately 1.75 log units, whereas the polypeptide according to SEQ ID NO: 126 (30xMIC) with 0.5 mM EDTA resulted in a complete eradication (>2.5 log in comparison to 0.5 mM EDTA/Tobramycin control). Whereas the polypeptide according to SEQ ID NO: 125 (30xMIC) alone did not strongly affect persisters, a strong reduction (approximately 1.8 log in comparison to the 0.5 mM EDTA/Tobramycin control) is observed in the presence of 0.5 mM EDTA.

### Example 5:

The killing effect of the polypeptide according to SEQ ID NO: 124 (comprising a fusion of Sarcotoxin IA (SEQ ID NO: 48) and KZ144 (SEQ ID NO: 11), active against vegetative cells of *E. coli)* was analyzed against the isolated persister fraction of *E. coli.* An isolate of a chronic urinary tract infection was used to isolate the persister fraction upon 5h amikacin exposure. A final concentration corresponding to 10xMIC was used, both in the absence or the presence of 0.5 mM EDTA. 0.5 mM EDTA was included as control. Ciprofloxacin (10xMIC) was tested as comparison antibiotic. The isolated persister fraction (approximately 10² persisters/ml) was significantly smaller in comparison to *P.aeruginosa* and *A*. *baumannii* persister fractions. The control antibiotic ciprofloxacin (10xMIC) (with and without EDTA) and the polypeptide according to SEQ ID NO: 124 (10xMIC) without 0.5 mM EDTA did not completely kill persisters. However, combination of the polypeptide according to SEQ ID NO: 124 (10xMIC) with 0.5 mM EDTA completely eradicated the persisters.

### Example 6:

The anti-persister effect of the polypeptide according to SEQ ID NO: 124 was evaluated against persisters isolated from *P. aeruginosa* PA14 upon exposure to ofloxacin. The persisters were treated with the polypeptide according to SEQ ID NO: 124 with a final concentration equivalent to 30xMIC against *P. aeruginosa* PA14, both in the absence and the presence of 0.5 mM EDTA. 0.5 mM EDTA and ofloxacin (30xMIC) were included as a control. Ciprofloxacin was included as comparison antibiotic. In comparison to the 0.5 mM EDTA control, the polypeptide according to SEQ ID NO: 124 (30xMIC) reduced the number of persisters with approximately 2.3 log units. When 0.5 mM EDTA is added to the polypeptide according to SEQ ID NO: 124 (30xMIC), a complete eradication is achieved (approximately 5 log units).

## Claims

1. A polypeptide comprising a peptidoglycan hydrolase for use as a medicament for the treatment of chronic bacterial infections caused by persister bacteria, wherein the peptidoglycan hydrolase is an endolysin and wherein the chronic bacterial infection is a chronic bacterial infection with *Pseudomonas, Escherichia, Staphylococcus aureus, Acinetobacter, Mycobacterium avium, Mycobacterium tuberculosis, Listeria monocytogenes* and/or *Salmonella* bacteria, and wherein the endolysin is capable of degrading the bacterial peptidoglycan of respective *Pseudomonas, Escherichia, Staphylococcus aureus, Acinetobacter, Mycobacterium avium, Mycobacterium tuberculosis, Listeria monocytogenes* or *Salmonella* bacteria.

2. The polypeptide for use according to claim 1, wherein the peptidoglycan hydrolase comprises an endopeptidase, N-acetyl-muramoyl-L-alanine-amidase, N-acetylmuramidase, N-acetyl-glucosaminidase and/or lytic transglycosylase.

3. The polypeptide for use according to claim 1 or 2, wherein the endolysin is PhiV10p30 of phage ΦV10 STM0907.Fels0 of phage FELS-1, epsilon15p25 of phage ε15, YuA20 of phage YUA, ORF23 of phage B3, BcepMu22 of phage BcepMu, F116p62 of phage F116, STM2715.S.Fels2 of phage Fels2, gp76 of phage ES18, SPSV3_gp23 of phage SETP3, phi32_17 of phage ΦECO32, HK022p54 of phage HK022, HK97p58 of phage HK97, HK620p36 of phage HK620, VIP0007 of phage El, Sf6p62 of phage SF6, R (SfVp40) of phage SFV, gp22 of phage BCEPC6B, K (P2p09) of phage P2, K (Wphi09) of phage WΦ, rv5_gp085 of phage RV5, EpJS98_gp116 of phage JS98, gp3.5 of phage 13A, gp3.5 of phage BA14, gp3.5 of phage ECODS1, CKV1F_gp16 of phage K1F, T3p18 of phage T3, gh-1p12 of phage GH-1, gp3.5 of phage K11, ORF12 of phage ΦCTX, Bcep43-27 of phage BCEP43, Bcep781-27 of phage BCEP781, Bcep1-28 of phage BCEP1, BcepNY3gene26 of phage BCEPNY3, gp45 of phage ΦE12-2, gp28 of phage Φ52237, P27p30 of phage ΦP27, RB49p102 of phage RB49, phil-p102 of phage Φ1, lys (T5.040) of phage T5, YP_001956952.1 of phage 201phi2-1, Aehlp339 of phage Aeh1, YYZgp45 of phage YYZ-2008, gp144 of phage ΦKZ, EL188 of phage EL, YP_001671940.1 of the phage LUZ24, N4 N-acetylmuramidase of phage N4gp61, STM0016 endolysin, PSP3 endolysin, PVP-SE1gp146 of phage PVPSE1, PlyA118, PlyA500, PlyPSA, PlyA511, PlyP35, PlyP40, Phi 11, Phi MR11, LysK, PlyS9, Ply3626, CD27L, B30, phage Dp-1 encoded Pal amidase, C1 endolysin PlyC, Cpl-1 endolysin, PlyGBS, PlyV12, Phage gamma endolysin PlyG or an endolysin having an amino acid sequence selected from the group of SEQ ID NO: 1-11.

4. The polypeptide for use according to any one of claims 1 to 3 further comprising an amino acid sequence stretch.

5. The polypeptide for use according to claim 4, wherein the amino acid sequence stretch is LL-37, SMAP-29, Indolicidin, Protegrin, Cecropin P1, Magainin, Pleurocidin, Cecropin A (A.aegypti), Cecropin A (D. melanogaster), Buforin II, Sarcotoxin IA, Apidaecin, Ascaphine 5, Nigrocine 2, Pseudin 1, Ranalexin, Melittin, Lycotoxin 1, Parasin 1, Buforin I, Dermaseptin 1, Bactenecin 1, Thanatin, Brevinin 1T, Ranateurin 1, Esculentin 1, Tachyplesin, Androctonin, alpha-defensin, beta-defensin, theta-defensin, defensin (sapecin A), Thionin (crambin), defensin from radish, Drosomycin, Hepcidin, Bac 5, PR-39, Pyrrhocoricin or Histatin 5.

6. The polypeptide for use according to claim 4, wherein the amino acid sequence stretch has an amino acid sequence selected from the group of SEQ ID NO: 13-95.

7. The polypeptide for use according to any one of claims 1 to 6, wherein the bacterial infection is an infection of the skin, of soft tissues, the respiratory system, the lung, the digestive tract, the eye, the ear, the teeth, the nasopharynx, the mouth, the bones, and/or the vagina.

8. The polypeptide for use according to any one of claims 1 to 7, wherein the chronic bacterial infection is a chronic bacterial infection with *Pseudomonas aeruginosa, Escherichia coli* and/or *Acinetobacter baumannii* bacteria.

9. The polypeptide for use according to any one of claims 1 to 7, wherein the chronic bacterial infection is a chronic bacterial infection with *Pseudomonas aeruginosa.*

10. The polypeptide for use according to any one of claims 1 to 7, wherein the chronic bacterial infection is a chronic bacterial infection with *Escherichia coli.*

11. The polypeptide for use according to any one of claims 1 to 7, wherein the chronic bacterial infection is a chronic bacterial infection with *Acinetobacter baumannii.*

12. A pharmaceutical composition comprising a polypeptide according to any one of claims 1 to 11 for use as a medicament for the treatment of chronic bacterial infections caused by persister bacteria, wherein the chronic bacterial infection is a chronic bacterial infection with *Pseudomonas, Escherichia, Staphylococcus aureus, Acinetobacter, Mycobacterium avium, Mycobacterium tuberculosis, Listeria monocytogenes* and/or *Salmonella* bacteria.

13. The pharmaceutical composition for use according to claim 12, wherein the chronic bacterial infection is a chronic bacterial infection with *Pseudomonas aeruginosa, Escherichia coli* and/or *Acinetobacter baumannii* bacteria.

14. The pharmaceutical composition for use according to claim 12 or 13, wherein the bacterial infection is an infections of the skin, of soft tissues, the respiratory system, the lung, the digestive tract, the eye, the ear, the teeth, the nasopharynx, the mouth, the bones, and/or the vagina.

## Patentansprüche

1. Polypeptid umfassend eine Peptidoglycanhydrolase zur Verwendung als Medikament zur Behandlung chronischer bakterieller Infektionen verursacht durch Persisterbakterien,
2. wobei die Peptidoglycanhydrolase ein Endolysin ist und
3. wherein the chronic bacterial infection is a chronic bacterial infection with *Pseudomonas, Escherichia, Staphylococcus aureus, Acinetobacter, Mycobacterium avium, Mycobacterium tuberculosis, Listeria monocytogenes* and/or *Salmonella* bacteria, and
4. wobei die chronische bakterielle Infektion eine chronische bakterielle Infektion mit *Pseudomonas-, Escherichia-, Staphylococcus aureus-, Acinetobacter-, Mycobacterium avium-, Mycobacterium tuberculosis-, Listeria monocytogenes*und/oder *Salmonella-Bakterien* ist, und
5. wobei das Endolysin in der Lage ist, das bakterielle Peptidoglycan der jeweiligen *Pseudomonas-, Escherichia-, Staphylococcus aureus-, Acinetobacter-, Mycobacterium avium-, Mycobacterium tuberculosis-, Listeria monocytogenes*oder *Salmonella-Bakterien* abzubauen.

2. Polypeptid zur Verwendung nach Anspruch 1, wobei die Peptidoglycan-Hydrolase eine Endopeptidase, N-Acetyl-Muramoyl-L-Alanin-Amidase, N-Acetyl-Muramidase, N-Acetyl-Glucosaminidase und/oder lytische Transglycosylase umfasst

3. Polypeptid zur Verwendung nach Anspruch 1 oder 2, wobei das Endolysin PhiV10p30 des Phagen ΦV10 STM0907,Fels0 des Phagen FELS-1, epsilon15p25 des Phagen ε15, YuA20 des Phagen YUA, ORF23 des Phagen B3, BcepMu22 des Phagen BcepMu, F116p62 des Phagen F116, STM2715.S.Fels2 des Phagen Fels2, gp76 des Phagen ES18, SPSV3_gp23 des Phagen SETP3, phi32_17 des Phagen ΦECO32, HK022p54 des Phagen HK022, HK97p58 des Phagen HK97, HK620p36 des Phagen HK620, VIP0007 des Phagen E1, Sf6p62 des Phagen SF6, R (SfVp40) des Phagen SFV, gp22 des Phagen BCEPC6B, K (P2p09) des Phagen P2, K (Wphi09) des Phagen WΦ, rv5_gp085 des Phagen RV5, EpJS98_gp116 des Phagen JS98, gp3.5 des Phagen 13A, gp3.5 des Phagen BA14, gp3.5 des Phagen ECODS1, CKV1F_gp16 des Phagen K1F, T3p18 des Phagen T3, gh-1p12 des Phagen GH-1, gp3.5 des Phagen K11, ORF12 des Phagen ΦCTX, Bcep43-27 des Phagen BCEP43, Bcep781-27 des Phagen BCEP781, Bcepl-28 des Phagen BCEP1, BcepNY3gene26 des Phagen BCEPNY3, gp45 des Phagen ΦE12-2, gp28 des Phagen Φ52237, P27p30 des Phagen ΦP27, RB49p102 des Phagen RB49, phil-p102 des Phagen Φ1, lys (T5.040) des Phagen T5, YP_001956952.1 des Phagen 201phi2-1, Aehlp339 des Phagen Aeh1, YYZgp45 des Phagen YYZ-2008, gp144 des Phagen ΦKZ, EL188 des Phagen EL, YP_001671940.1 des Phagen LUZ24, N4 N-acetylmuramidase des Phagen N4gp61, STM0016 endolysin, PSP3 endolysin, PVP-SE1gp146 des Phagen PVPSE1, PlyA118, PlyA500, PlyPSA, PlyA511, PlyP35, PlyP40, Phi 11, Phi MR11, LysK, PlyS9, Ply3626, CD27L, B30, des Phagen Dp-1 codierte Pal amidase, C1 endolysin PlyC, Cpl-1 endolysin, PlyGBS, PlyV12, Phagen gamma endolysin PlyG oder ein Endolysin mit einer Aminosäuresequenz ausgewählt aus der Gruppe von SEQ ID NO: 1-11 ist.

4. Polypeptid zur Verwendung nach einem der Ansprüche 1 bis 3, das ferner einen Aminosäuresequenzabschnitt umfasst.

5. Polypeptid zur Verwendung nach Anspruch 4, wobei der Aminosäuresequenzabschnitt LL-37, SMAP-29, Indolicidin, Protegrin, Cecropin P1, Magainin, Pleurocidin, Cecropin A (A. aegypti), Cecropin A (D. melanogaster), Buforin II, Sarcotoxin IA, Apidaecin, Ascaphine 5, Nigrocine 2, Pseudin 1, Ranalexin, Melittin, Lycotoxin 1, Parasin 1, Buforin I, Dermaseptin 1, Bactenecin 1, Thanatin, Brevinin 1T, Ranateurin 1, Esculentin 1, Tachyplesin, Androctonin, Alpha-Defensin, Beta-Defensin, Theta-Defensin, Defensin (Sapecin A), Thionin (Crambin), Defensin aus Rettich, Drosomycin, Hepcidin, Bac 5, PR-39, Pyrrhocoricin oder Histatin 5 ist.

6. Polypeptid zur Verwendung nach Anspruch 4, wobei der Aminosäuresequenzabschnitt eine Aminosäuresequenz aufweist, die aus der Gruppe SEQ ID NO: 13-95 ausgewählt ist.

7. Polypeptid zur Verwendung nach einem der Ansprüche 1 bis 6, wobei die bakterielle Infektion eine Infektion der Haut, der Weichteile, des Atmungssystems, der Lunge, des Verdauungstrakts, des Auges, des Ohrs, der Zähne, der Nasopharynx, des Mundes, der Knochen und/oder der Vagina ist.

8. Polypeptid zur Verwendung nach einem der Ansprüche 1 bis 7, wobei die chronische bakterielle Infektion eine chronische bakterielle Infektion mit *Pseudomonas aeruginosa-, Escherichia coli-* und/oder *Acinetobacter baumannii-Bakterien* ist.

9. Polypeptid zur Verwendung nach einem der Ansprüche 1 bis 7, wobei die chronische bakterielle Infektion eine chronische bakterielle Infektion mit *Pseudomonas aeruginosa* ist.

10. Polypeptid zur Verwendung nach einem der Ansprüche 1 bis 7, wobei die chronische bakterielle Infektion eine chronische bakterielle Infektion mit *Escherichia coli* ist

11. Polypeptid zur Verwendung nach einem der Ansprüche 1 bis 7, wobei die chronische bakterielle Infektion eine chronische bakterielle Infektion mit *Acinetobacter baumannii* ist

12. Pharmazeutische Zusammensetzung umfassend ein Polypeptid nach einem der Ansprüche 1 bis 11 zur Verwendung als Medikament zur Behandlung chronischer bakterieller Infektionen verursacht durch Persister-Bakterien, wobei die chronische bakterielle Infektion eine chronische bakterielle Infektion mit *Pseudomonas-, Escherichia-, Staphylococcus aureus-, Acinetobacter-, Mycobacterium avium-, Mycobacterium tuberculosis-, Listeria monocytogenes-* und/oder *Salmonella-Bakterien* ist.

13. Pharmazeutische Zusammensetzung zur Verwendung nach Anspruch 12, wobei die chronische bakterielle Infektion eine chronische bakterielle Infektion mit *Pseudomonas aeruginosa-, Escherichia coli-* und/oder *Acinetobacter baumannii-Bakterien* ist.

14. Pharmazeutische Zusammensetzung zur Verwendung nach Anspruch 12 oder 13, wobei die bakterielle Infektion eine Infektion der Haut, der Weichteile, des Atmungssystems, der Lunge, des Verdauungstrakts, des Auges, des Ohrs, der Zähne, des Nasopharynx, des Mundes, der Knochen und/oder der Vagina ist.

## Revendications

1. Polypeptide comprenant une peptidoglycane hydrolase pour utilisation comme médicament pour le traitement d'infections bactériennes chroniques provoquées par des bactéries persistantes, laquelle peptidoglycane hydrolase étant une endolysine et laquelle infection bactérienne chronique étant une infection bactérienne chronique par des bactéries *Pseudomonas, Escherichia, Staphylococcus aureus, Acinetobacter, Mycobacterium avium, Mycobacterium tuberculosis, Listeria monocytogenes* et/ou *Salmonella,* et laquelle endolysine étant capable de dégrader le peptidoglycane bactérien des bactéries *Pseudomonas, Escherichia, Staphylococcus aureus, Acinetobacter, Mycobacterium avium, Mycobacterium tuberculosis, Listeria monocytogenes* ou *Salmonella* respectives.

2. Polypeptide pour utilisation selon la revendication 1, dans lequel la peptidoglycane hydrolase comprend une endopeptidase, N-acétyl-myramoyl-L-alanine-amidase, N-acétyl-muramidase, N-acétyl-glucosaminidase et/ou transglycosylase lytique.

3. Polypeptide pour utilisation selon la revendication 1 ou 2, dans lequel l'endolysine est PhiV10p30 du phage ΦV10, STM0907.Fels0 du phage FELS-1, epsilon15p25 du phage ε15, YuA20 du phage YUA, ORF23 du phage B3, BcepMu22 du phage BcepMu, F116p62 du phage F116, STM2715.S.Fels2 du phage Fels2, gp76 du phage ES18, SPSV3_gp23 du phage SETP3, phi32_17 du phage ΦECO32, HK022p54 du phage HK022, HK97p58 du phage HK97, HK620p36 du phage HK620, VIP0007 du phage El, Sf6p62 du phage SF6, R (SfVp40) du phage SFV, gp22 du phage BCEPC6B, K (P2p09) du phage P2, K (Wphi09) du phage WΦ, rv5_gp085 du phage RV5, EpJS98_gp116 du phage JS98, gp3.5 du phage 13A, gp3.5 du phage BA14, gp3.5 du phage ECODS1, CKV1F_gp16 du phage K1F, T3p18 du phage T3, gh-1p12 du phage GH-1, gp3.5 du phage K11, ORF12 du phage ΦCTX, Bcep43-27 du phage BCEP43, Bcep781-27 du phage BCEP781, Bcepl-28 du phage BCEP1, BcepNY3gene26 du phage BCEPNY3, gp45 du phage ΦE12-2, gp28 du phage Φ52237, P27p30 du phage ΦP27, RB49p102 du phage RB49, phil-p102 du phage Φ1, lys (T5.040) du phage T5, YP 001956952.1 du phage 201phi2-1, Aehlp339 du phage Aeh1, YYZgp45 du phage YYZ-2008, gp144 du phage ΦKZ, EL188 du phage EL, YP_001671940.1 du phage LUZ24, N4 N-acétylmuramidase du phage N4gp61, endolysine STM0016, endolysine PSP3, PVP-SE1gp146 du phage PVPSE1, PlyA118, PlyA500, PlyPSA, PlyA511, PlyP35, PlyP40, Phi 11, Phi MR11, LysK, PlyS9, Ply3626, CD27L, B30, amidase Pal codée par le phage Dp-1, endolysine PlyC de C1, endolysine Cpl-1, PlyGBS, PlyV12, endolysine PlyG du phage gamma ou une endolysine ayant une séquence d'acides aminés sélectionnée dans le groupe des SEQ ID NO : 1-11.

4. Polypeptide pour utilisation selon l'une quelconque des revendications 1 à 3, comprenant en outre une extension de séquence d'acides aminés.

5. Polypeptide pour utilisation selon la revendication 4, dans lequel l'extension de séquence d'acides aminés est LL-37, SMAP-29, Indolicidine, Protégrine, Cécropine P1, Magainine, Pleurocidine, Cécropine A (A. aegypti), Cécropine A (D. melanogaster), Buforine II, Sarcotoxine IA, Apidaécine, Ascaphine 5, Nigrocine 2, Pseudine 1, Ranalexine, Mélittine, Lycotoxine 1, Parasine 1, Buforine I, Dermaseptine 1, Bacténécine 1, Thanatine, Brévinine 1T, Ranateurine 1, Esculentine 1, Tachyplésine, Androctonine, alpha-défensine, bêta-défensine, thêta-défensine, défensine (sapécine A), Thionine (crambine), défensine de radis, Drosomycine, Hepcidine, Bac 5, PR-39, Pyrrhocoricine ou Histatine 5.

6. Polypeptide pour utilisation selon la revendication 4, dans lequel l'extension de séquence d'acides aminés a une séquence d'acides aminés sélectionnée dans le groupe des SEQ ID NO : 13-95.

7. Polypeptide pour utilisation selon l'une quelconque des revendications 1 à 6, laquelle infection bactérienne étant une infection de la peau, des tissus mous, du système respiratoire, du poumon, du tube digestif, de l'œil, de l'oreille, des dents, du nasopharynx, de la bouche, des os, et/ou du vagin.

8. Polypeptide pour utilisation selon l'une quelconque des revendications 1 à 7, laquelle infection bactérienne chronique étant une infection bactérienne chronique par des bactéries *Pseudomonas aeruginosa, Escherichia coli* et/ou *Acinetobacter baumannii.*

9. Polypeptide pour utilisation selon l'une quelconque des revendications 1 à 7, laquelle infection bactérienne chronique étant une infection bactérienne chronique par *Pseudomonas aeruginosa.*

10. Polypeptide pour utilisation selon l'une quelconque des revendications 1 à 7, laquelle infection bactérienne chronique étant une infection bactérienne chronique par *Escherichia coli.*

11. Polypeptide pour utilisation selon l'une quelconque des revendications 1 à 7, laquelle infection bactérienne chronique étant une infection bactérienne chronique par *Acinetobacter baumannii.*

12. Composition pharmaceutique comprenant un polypeptide selon l'une quelconque des revendications 1 à 11 pour utilisation comme médicament pour le traitement d'infections bactériennes chroniques provoquées par des bactéries persistantes, laquelle infection bactérienne chronique étant une infection bactérienne chronique par des bactéries *Pseudomonas, Escherichia, Staphylococcus aureus, Acinetobacter, Mycobacterium avium, Mycobacterium tuberculosis, Listeria monocytogenes* et/ou *Salmonella.*

13. Composition pharmaceutique pour utilisation selon la revendication 12, laquelle infection bactérienne chronique étant une infection bactérienne chronique par des bactéries *Pseudomonas aeruginosa, Escherichia coli* et/ou *Acinetobacter baumannii.*

14. Composition pharmaceutique pour utilisation selon la revendication 12 ou 13, laquelle infection bactérienne étant une infection de la peau, des tissus mous, du système respiratoire, du poumon, du tube digestif, de l'œil, de l'oreille, des dents, du nasopharynx, de la bouche, des os, et/ou du vagin.


### SEQUENCE LISTING

<110> Lysando AG
<120> Pharmaceutical Composition against Chronic Bacterial Infections
<130> LYS-026 PCT
<150> EP14163927.8
   <151> 2014-04-08
<160> 126
<170> PatentIn version 3.5
<210> 1
   <211> 494
   <212> PRT
   <213> unknown
<220>
   <223> Lysk endolysin
<400> 1
   Ala Lys Thr Gln Ala Glu Ile Asn Lys Arg Leu Asp Ala Tyr Ala Lys
   1               5                   10                  15
   Gly Thr Val Asp Ser Pro Tyr Arg Val Lys Lys Ala Thr Ser Tyr Asp
               20                  25                  30
   Pro Ser Phe Gly Val Met Glu Ala Gly Ala Ile Asp Ala Asp Gly Tyr
           35                  40                  45
   Tyr His Ala Gln Cys Gln Asp Leu Ile Thr Asp Tyr Val Leu Trp Leu
       50                  55                  60
   Thr Asp Asn Lys Val Arg Thr Trp Gly Asn Ala Lys Asp Gln Ile Lys
   65                  70                  75                  80
   Gln Ser Tyr Gly Thr Gly Phe Lys Ile His Glu Asn Lys Pro Ser Thr
                   85                  90                  95
   Val Pro Lys Lys Gly Trp Ile Ala Val Phe Thr Ser Gly Ser Tyr Glu
               100                 105                 110
   Gln Trp Gly His Ile Gly Ile Val Tyr Asp Gly Gly Asn Thr Ser Thr
           115                 120                 125
   Phe Thr Ile Leu Glu Gln Asn Trp Asn Gly Tyr Ala Asn Lys Lys Pro
       130                 135                 140
   Thr Lys Arg Val Asp Asn Tyr Tyr Gly Leu Thr His Phe Ile Glu Ile
   145                 150                 155                 160
   Pro Val Lys Ala Gly Thr Thr Val Lys Lys Glu Thr Ala Lys Lys Ser
                   165                 170                 175
   Ala Ser Lys Thr Pro Ala Pro Lys Lys Lys Ala Thr Leu Lys Val Ser
               180                 185                 190
   Lys Asn His Ile Asn Tyr Thr Met Asp Lys Arg Gly Lys Lys Pro Glu
           195                 200                 205
   Gly Met Val Ile His Asn Asp Ala Gly Arg Ser Ser Gly Gln Gln Tyr
       210                 215                 220
   Glu Asn Ser Leu Ala Asn Ala Gly Tyr Ala Arg Tyr Ala Asn Gly Ile
   225                 230                 235                 240
   Ala His Tyr Tyr Gly Ser Glu Gly Tyr Val Trp Glu Ala Ile Asp Ala
                   245                 250                 255
   Lys Asn Gln Ile Ala Trp His Thr Gly Asp Gly Thr Gly Ala Asn Ser
               260                 265                 270
   Gly Asn Phe Arg Phe Ala Gly Ile Glu Val Cys Gln Ser Met Ser Ala
           275                 280                 285
   Ser Asp Ala Gln Phe Leu Lys Asn Glu Gln Ala Val Phe Gln Phe Thr
       290                 295                 300
   Ala Glu Lys Phe Lys Glu Trp Gly Leu Thr Pro Asn Arg Lys Thr Val
   305                 310                 315                 320
   Arg Leu His Met Glu Phe Val Pro Thr Ala Cys Pro His Arg Ser Met
                   325                 330                 335
   Val Leu His Thr Gly Phe Asn Pro Val Thr Gln Gly Arg Pro Ser Gln
               340                 345                 350
   Ala Ile Met Asn Lys Leu Lys Asp Tyr Phe Ile Lys Gln Ile Lys Asn
           355                 360                 365
   Tyr Met Asp Lys Gly Thr Ser Ser Ser Thr Val Val Lys Asp Gly Lys
       370                 375                 380
   Thr Ser Ser Ala Ser Thr Pro Ala Thr Arg Pro Val Thr Gly Ser Trp
   385                 390                 395                 400
   Lys Lys Asn Gln Tyr Gly Thr Trp Tyr Lys Pro Glu Asn Ala Thr Phe
                   405                 410                 415
   Val Asn Gly Asn Gln Pro Ile Val Thr Arg Ile Gly Ser Pro Phe Leu
               420                 425                 430
   Asn Ala Pro Val Gly Gly Asn Leu Pro Ala Gly Ala Thr Ile Val Tyr
           435                 440                 445
   Asp Glu Val Cys Ile Gln Ala Gly His Ile Trp Ile Gly Tyr Asn Ala
       450                 455                 460
   Tyr Asn Gly Asn Arg Val Tyr Cys Pro Val Arg Thr Cys Gln Gly Val
   465                 470                 475                 480
   Pro Pro Asn Gln Ile Pro Gly Val Ala Trp Gly Val Phe Lys
                   485                 490
<210> 2
   <211> 488
   <212> **PRT**
   <213> unknown
<220>
   <223> PlySK1249 endolysin
<400> 2
   Gly Lys His Leu Val Ile Cys Gly His Gly Gln Gly Arg Thr Thr Tyr
   1               5                   10                  15
   Asp Pro Gly Ala Val Asn Ala Lys Leu Gly Ile Thr Glu Ala Gly Lys
               20                  25                  30
   Val Arg Glu Leu Ala Lys Leu Met Ser Lys Tyr Ser Gly Gln Gln Ile
           35                  40                  45
   Asp Phe Ile Thr Glu Gln Asn Val Tyr Asp Tyr Arg Ser Ile Thr Ser
       50                  55                  60
   Ile Gly Lys Gly Tyr Asp Ser Ile Thr Glu Leu His Phe Asn Ala Phe
   65                  70                  75                  80
   Asn Gly Ser Ala Lys Gly Thr Glu Val Leu Ile Gln Ser Ser Leu Glu
                   85                  90                  95
   Ala Asp Lys Glu Asp Met Ala Ile Leu Ser Leu Leu Ser Arg Tyr Phe
               100                 105                 110
   Gln Asn Arg Gly Ile Lys Lys Val Asp Trp Leu Tyr Asn Ala Asn Gln
           115                 120                 125
   Ala Ala Ser Arg Gly Tyr Thr Tyr Arg Leu Val Glu Ile Ala Phe Ile
       130                 135                 140
   Asp Asn Glu Gln Asp Met Ala Ile Phe Glu Thr Lys Lys Glu Asp Ile
   145                 150                 155                 160
   Ala Lys Gly Leu Val Ser Ala Ile Thr Gly Val Glu Val Lys Thr Ile
                   165                 170                 175
   Val Pro Ser Thr Pro Ser Ser Thr Val Gly Ser Ser Gly Thr Pro Ser
               180                 185                 190
   Lys Pro Ile Tyr Leu Val Gly Asp Ser Leu Arg Val Leu Pro His Ala
           195                 200                 205
   Thr His Tyr Gln Thr Gly Gln Lys Ile Ala Asn Trp Val Lys Gly Arg
       210                 215                 220
   Thr Tyr Lys Ile Leu Gln Glu Lys Asn Val His Gln Ser Asn Ser Leu
   225                 230                 235                 240
   Arg Ala Tyr Leu Leu Asp Gly Ile Lys Ser Trp Val Leu Glu Gln Asp
                   245                 250                 255
   Val Glu Gly Thr Thr Lys Gly His Ser Glu Gln Thr Tyr Gln Ala Gln
               260                 265                 270
   Lys Gly Asp Thr Tyr Tyr Gly Ile Ala Arg Lys Phe Gly Leu Thr Val
           275                 280                 285
   Asp Ala Leu Leu Ala Val Asn Gly Leu Lys Lys Thr Asp Ile Leu Arg
       290                 295                 300
   Val Gly Gln Thr Leu Lys Val Asn Ala Ala Ser Arg Ile Thr Thr Ala
   305                 310                 315                 320
   Ile Pro Thr Ser Val Ala Ser Arg Val Val Ala Ser Ala Leu Ser Lys
                   325                 330                 335
   Val Gly Gln Lys Val Thr Val Pro Ser Asn Pro Tyr Gly Gly Gln Cys
               340                 345                 350
   Val Ala Leu Val Asp Lys Ile Val Gln Glu Leu Thr Asp Lys Asn Met
           355                 360                 365
   Ser Tyr Thr Asn Ala Ile Asp Cys Leu Lys Lys Ala Lys Ser Asn Gly
   370 375 380
   Phe Gln Val Ile Tyr Asp Ala Trp Gly Val Asn Pro Lys Ala Gly Asp
   385 390 395 400
   Phe Tyr Val Ile Glu Thr Asp Gly Leu Val Tyr Gly His Ile Gly Val
   405 410 415
   Cys Val Thr Asp Ser Asp Gly Lys Ser Ile Asp Gly Val Glu Gln Asn
   420 425 430
   Ile Asp Gly Tyr Ser Asp His Asn Lys Asn Gly Ile Asn Asp Gln Leu
   435 440 445
   Glu Ile Gly Gly Gly Gly Ile Thr Arg Arg Val Lys Arg Gln Trp Met
   450 455 460
   Ala Asp Gly Ser Leu Tyr Asp Ser Thr Gly Thr Val Lys Leu Gly Lys
   465 470 475 480
   Val Val Gly Trp Phe Arg Ile Ser
   485
<210> 3
   <211> 467
   <212> **PRT**
   <213> unknown
<220>
   <223> lambdaSa21ys
<400> 3
   Glu Ile Asn Thr Glu Ile Ala Ile Ala Trp Met Ser Ala Arg Gln Gly
   1               5                   10                  15
   Lys Val Ser Tyr Ser Met Asp Tyr Arg Asp Gly Pro Asn Ser Tyr Asp
               20                  25                  30
   Cys Ser Ser Ser Val Tyr Tyr Ala Leu Arg Ser Ala Gly Ala Ser Ser
           35                  40                  45
   Ala Gly Trp Ala Val Asn Thr Glu Tyr Met His Asp Trp Leu Ile Lys
       50                  55                  60
   Asn Gly Tyr Glu Leu Ile Ala Glu Asn Val Asp Trp Asn Ala Val Arg
   65                  70                  75                  80
   Gly Asp Ile Ala Ile Trp Gly Met Arg Gly His Ser Ser Gly Ala Gly
                   85                  90                  95
   Gly His Val Val Met Phe Ile Asp Pro Glu Asn Ile Ile His Cys Asn
               100                 105                 110
   Trp Ala Asn Asn Gly Ile Thr Val Asn Asn Tyr Asn Gln Thr Ala Ala
           115                 120                 125
   Ala Ser Gly Trp Met Tyr Cys Tyr Val Tyr Arg Leu Lys Ser Gly Ala
       130                 135                 140
   Ser Thr Gln Gly Lys Ser Leu Asp Thr Leu Val Lys Glu Thr Leu Ala
   145                 150                 155                 160
   Gly Asn Tyr Gly Asn Gly Glu Ala Arg Lys Ala Val Leu Gly Asn Gln
                   165                 170                 175
   Tyr Glu Ala Val Met Ser Val Ile Asn Gly Lys Thr Thr Thr Asn Gln
               180                 185                 190
   Lys Thr Val Asp Gln Leu Val Gln Glu Val Ile Ala Gly Lys His Gly
           195                 200                 205
   Asn Gly Glu Ala Arg Lys Lys Ser Leu Gly Ser Gln Tyr Asp Ala Val
       210                 215                 220
   Gln Lys Arg Val Thr Glu Leu Leu Lys Lys Gln Pro Ser Glu Pro Phe
   225                 230                 235                 240
   Lys Ala Gln Glu Val Asn Lys Pro Thr Glu Thr Lys Thr Ser Gln Thr
                   245                 250                 255
   Glu Leu Thr Gly Gln Ala Thr Ala Thr Lys Glu Glu Gly Asp Leu Ser
               260                 265                 270
   Phe Asn Gly Thr Ile Leu Lys Lys Ala Val Leu Asp Lys Ile Leu Gly
           275                 280                 285
   Asn Cys Lys Lys His Asp Ile Leu Pro Ser Tyr Ala Leu Thr Ile Leu
       290                 295                 300
   His Tyr Glu Gly Leu Trp Gly Thr Ser Ala Val Gly Lys Ala Asp Asn
   305                 310                 315                 320
   Asn Trp Gly Gly Met Thr Trp Thr Gly Gln Gly Asn Arg Pro Ser Gly
                   325                 330                 335
   Val Thr Val Thr Gln Gly Ser Ala Arg Pro Ser Asn Glu Gly Gly His
   340 345 350
   Tyr Met His Tyr Ala Ser Val Asp Asp Phe Leu Thr Asp Trp Phe Tyr
   355 360 365
   Leu Leu Arg Ala Gly Gly Ser Tyr Lys Val Ser Gly Ala Lys Thr Phe
   370 375 380
   Ser Glu Ala Ile Lys Gly Met Phe Lys Val Gly Gly Ala Val Tyr Asp
   385 390 395 400
   Tyr Ala Ala Ser Gly Phe Asp Ser Tyr Ile Val Gly Ala Ser Ser Arg
   405 410 415
   Leu Lys Ala Ile Glu Ala Glu Asn Gly Ser Leu Asp Lys Phe Asp Lys
   420 425 430
   Ala Thr Asp Ile Gly Asp Gly Ser Lys Asp Lys Ile Asp Ile Thr Ile
   435 440 445
   Glu Gly Ile Glu Val Thr Ile Asn Gly Ile Thr Tyr Glu Leu Thr Lys
   450 455 460
   Lys Pro Val
   465
<210> 4
   <211> 340
   <212> PRT
   <213> unknown
<220>
   <223> Ply511 endolysin
<400> 4
   Val Lys Tyr Thr Val Glu Asn Lys Ile Ile Ala Gly Leu Pro Lys Gly
   1               5                   10                  15
   Lys Leu Lys Gly Ala Asn Phe Val Ile Ala His Glu Thr Ala Asn Ser
               20                  25                  30
   Lys Ser Thr Ile Asp Asn Glu Val Ser Tyr Met Thr Arg Asn Trp Lys
           35                  40                  45
   Asn Ala Phe Val Thr His Phe Val Gly Gly Gly Gly Arg Val Val Gln
       50                  55                  60
   Val Ala Asn Val Asn Tyr Val Ser Trp Gly Ala Gly Gln Tyr Ala Asn
   65                  70                  75                  80
   Ser Tyr Ser Tyr Ala Gln Val Glu Leu Cys Arg Thr Ser Asn Ala Thr
                   85                  90                  95
   Thr Phe Lys Lys Asp Tyr Glu Val Tyr Cys Gln Leu Leu Val Asp Leu
               100                 105                 110
   Ala Lys Lys Ala Gly Ile Pro Ile Thr Leu Asp Ser Gly Ser Lys Thr
           115                 120                 125
   Ser Asp Lys Gly Ile Lys Ser His Lys Trp Val Ala Asp Lys Leu Gly
       130                 135                 140
   Gly Thr Thr His Gln Asp Pro Tyr Ala Tyr Leu Ser Ser Trp Gly Ile
   145                 150                 155                 160
   Ser Lys Ala Gln Phe Ala Ser Asp Leu Ala Lys Val Ser Gly Gly Gly
                   165                 170                 175
   Asn Thr Gly Thr Ala Pro Ala Lys Pro Ser Thr Pro Ala Pro Lys Pro
               180                 185                 190
   Ser Thr Pro Ser Thr Asn Leu Asp Lys Leu Gly Leu Val Asp Tyr Met
           195                 200                 205
   Asn Ala Lys Lys Met Asp Ser Ser Tyr Ser Asn Arg Asp Lys Leu Ala
       210                 215                 220
   Lys Gln Tyr Gly Ile Ala Asn Tyr Ser Gly Thr Ala Ser Gln Asn Thr
   225                 230                 235                 240
   Thr Leu Leu Ser Lys Ile Lys Gly Gly Ala Pro Lys Pro Ser Thr Pro
                   245                 250                 255
   Ala Pro Lys Pro Ser Thr Ser Thr Ala Lys Lys Ile Tyr Phe Pro Pro
               260                 265                 270
   Asn Lys Gly Asn Trp Ser Val Tyr Pro Thr Asn Lys Ala Pro Val Lys
           275                 280                 285
   Ala Asn Ala Ile Gly Ala Ile Asn Pro Thr Lys Phe Gly Gly Leu Thr
       290                 295                 300
   Tyr Thr Ile Gln Lys Asp Arg Gly Asn Gly Val Tyr Glu Ile Gln Thr
   305                 310                 315                 320
   Asp Gln Phe Gly Arg Val Gln Val Tyr Gly Ala Pro Ser Thr Gly Ala
   325 330 335
   Val Ile Lys Lys
   340
<210> 5
   <211> 338
   <212> **PRT**
   <213> unknown
<220>
   <223> Cpl-1 endolysin
<400> 5
   Val Lys Lys Asn Asp Leu Phe Val Asp Val Ser Ser His Asn Gly Tyr
   1               5                   10                  15
   Asp Ile Thr Gly Ile Leu Glu Gln Met Gly Thr Thr Asn Thr Ile Ile
               20                  25                  30
   Lys Ile Ser Glu Ser Thr Thr Tyr Leu Asn Pro Cys Leu Ser Ala Gln
           35                  40                  45
   Val Glu Gln Ser Asn Pro Ile Gly Phe Tyr His Phe Ala Arg Phe Gly
       50                  55                  60
   Gly Asp Val Ala Glu Ala Glu Arg Glu Ala Gln Phe Phe Leu Asp Asn
   65                  70                  75                  80
   Val Pro Met Gln Val Lys Tyr Leu Val Leu Asp Tyr Glu Asp Asp Pro
                   85                  90                  95
   Ser Gly Asp Ala Gln Ala Asn Thr Asn Ala Cys Leu Arg Phe Met Gln
               100                 105                 110
   Met Ile Ala Asp Ala Gly Tyr Lys Pro Ile Tyr Tyr Ser Tyr Lys Pro
           115                 120                 125
   Phe Thr His Asp Asn Val Asp Tyr Gln Gln Ile Leu Ala Gln Phe Pro
       130                 135                 140
   Asn Ser Leu Trp Ile Ala Gly Tyr Gly Leu Asn Asp Gly Thr Ala Asn
   145                 150                 155                 160
   Phe Glu Tyr Phe Pro Ser Met Asp Gly Ile Arg Trp Trp Gln Tyr Ser
                   165                 170                 175
   Ser Asn Pro Phe Asp Lys Asn Ile Val Leu Leu Asp Asp Glu Glu Asp
               180                 185                 190
   Asp Lys Pro Lys Thr Ala Gly Thr Trp Lys Gln Asp Ser Lys Gly Trp
   195 200 205
   Trp Phe Arg Arg Asn Asn Gly Ser Phe Pro Tyr Asn Lys Trp Glu Lys
   210 215 220
   Ile Gly Gly Val Trp Tyr Tyr Phe Asp Ser Lys Gly Tyr Cys Leu Thr
   225 230 235 240
   Ser Glu Trp Leu Lys Asp Asn Glu Lys Trp Tyr Tyr Leu Lys Asp Asn
   245 250 255
   Gly Ala Met Ala Thr Gly Trp Val Leu Val Gly Ser Glu Trp Tyr Tyr
   260 265 270
   Met Asp Asp Ser Gly Ala Met Val Thr Gly Trp Val Lys Tyr Lys Asn
   275 280 285
   Asn Trp Tyr Tyr Met Thr Asn Glu Arg Gly Asn Met Val Ser Asn Glu
   290 295 300
   Phe Ile Lys Ser Gly Lys Gly Trp Tyr Phe Met Asn Thr Asn Gly Glu
   305 310 315 320
   Leu Ala Asp Asn Pro Ser Phe Thr Lys Glu Pro Asp Gly Leu Ile Thr
   325 330 335
   Val Ala
<210> 6
   <211> 327
   <212> PRT
   <213> unknown
<220>
   <223> OBPgp279
<400> 6
   Lys Asn Ser Glu Lys Asn Ala Ser Ile Ile Met Ser Ile Gln Arg Thr
   1 5 10 15
   Leu Ala Ser Leu Ser Leu Tyr Gly Gly Arg Ile Asp Gly Leu Phe Gly
   20 25 30
   Glu Lys Cys Arg Gly Ala Ile Ile Leu Met Leu Asn Lys Val Tyr Pro
   35 40 45
   Asn Phe Ser Thr Asn Lys Leu Pro Ser Asn Thr Tyr Glu Ala Glu Ser
       50                  55                  60
   Val Phe Thr Phe Leu Gln Thr Ala Leu Ala Gly Val Gly Leu Tyr Thr
   65                  70                  75                  80
   Ile Thr Ile Asp Gly Lys Trp Gly Gly Thr Ser Gln Gly Ala Ile Asp
                   85                  90                  95
   Ala Leu Val Lys Ser Tyr Arg Gln Ile Thr Glu Ala Glu Arg Ala Gly
               100                 105                 110
   Ser Thr Leu Pro Leu Gly Leu Ala Thr Val Met Ser Lys His Met Ser
           115                 120                 125
   Ile Glu Gln Leu Arg Ala Met Leu Pro Thr Asp Arg Gln Gly Tyr Ala
       130                 135                 140
   Glu Val Tyr Ile Asp Pro Leu Asn Glu Thr Met Asp Ile Phe Glu Ile
   145                 150                 155                 160
   Asn Thr Pro Leu Arg Ile Ala His Phe Met Ala Gln Ile Leu His Glu
                   165                 170                 175
   Thr Ala Cys Phe Lys Tyr Thr Glu Glu Leu Ala Ser Gly Lys Ala Tyr
               180                 185                 190
   Glu Gly Arg Ala Asp Leu Gly Asn Thr Arg Pro Gly Asp Gly Pro Leu
           195                 200                 205
   Phe Lys Gly Arg Gly Leu Leu Gln Ile Thr Gly Arg Leu Asn Tyr Val
       210                 215                 220
   Lys Cys Gln Val Tyr Leu Arg Glu Lys Leu Lys Asp Pro Thr Phe Asp
   225                 230                 235                 240
   Ile Thr Ser Ser Val Thr Cys Ala Gln Gln Leu Ser Glu Ser Pro Leu
                   245                 250                 255
   Leu Ala Ala Leu Ala Ser Gly Tyr Phe Trp Arg Phe Ile Lys Pro Lys
               260                 265                 270
   Leu Asn Glu Thr Ala Asp Lys Asp Asp Ile Tyr Trp Val Ser Val Tyr
           275                 280                 285
   Val Asn Gly Tyr Ala Lys Gln Ala Asn Pro Tyr Tyr Pro Asn Arg Asp
       290                 295                 300
   Lys Glu Pro Asn His Met Lys Glu Arg Val Gln Met Leu Ala Val Thr
   305 310 315 320
   Lys Lys Ala Leu Gly Ile Val
   325
<210> 7
   <211> 291
   <212> PRT
   <213> unknown
<220>
   <223> EL188 endolysin
<400> 7
   Asn Phe Arg Thr Lys Asn Gly Tyr Arg Asp Leu Gln Ala Leu Val Lys
   1 5 10 15
   Glu Leu Gly Leu Tyr Thr Gly Gln Ile Asp Gly Val Trp Gly Lys Gly
   20 25 30
   Thr Ser Ser Ser Thr Glu Thr Leu Leu Arg Gly Tyr Ala Glu Val Val
   35 40 45
   Gly Lys Asn Thr Gly Gly Ile Gly Leu Pro Thr Thr Ser Asp Ala Ser
   50 55 60
   Gly Tyr Asn Val Ile Thr Ala Leu Gln Arg Asn Leu Ala Phe Leu Gly
   65 70 75 80
   Leu Tyr Ser Leu Thr Val Asp Gly Ile Trp Gly Asn Gly Thr Leu Ser
   85 90 95
   Gly Leu Asp Lys Ala Phe Glu Val Tyr Lys Glu Arg Tyr Arg Thr Pro
   100 105 110
   Thr Tyr Asp Ile Ala Trp Ser Gly Lys Val Ser Pro Ala Phe Thr Ala
   115 120 125
   Lys Val Lys Asp Trp Cys Gly Val His Val Pro Asn His Arg Ala Pro
   130 135 140
   His Trp Leu Met Ala Cys Met Ala Phe Glu Thr Gly Gln Thr Phe Ser
   145 150 155 160
   Pro Ser Ile Lys Asn Ala Ala Gly Ser Glu Ala Tyr Gly Leu Ile Gln
   165 170 175
   Phe Met Ser Pro Ala Ala Asn Asp Leu Asn Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Phe His Pro Ala Ser Val Gly Lys Lys Ala Asp Glu
   225                 230                 235                 240
   Val Leu Phe Leu Gln Gly Ser Lys Ala Tyr Leu Gln Asn Lys Gly Phe
                   245                 250                 255
   Asp Val Asp Lys Asp Gly Lys Ile Thr Leu Gly Glu Ile Ser Ser Thr
               260                 265                 270
   Leu Tyr Thr Thr Tyr Tyr Lys Gly Leu Leu Pro Glu Asn Arg His Val
           275                 280                 285
   Ile Ser Tyr
       290
<210> 8
   <211> 235
   <212> **PRT**
   <213> unknown
<220>
   <223> PVP-SE1gp146
<400> 8
   Asn Ala Ala Ile Ala Glu Ile Gln Arg Met Leu Ile Glu Gly Gly Phe
   1               5                   10                  15
   Ser Val Gly Lys Ser Gly Ala Asp Gly Leu Tyr Gly Pro Ala Thr Lys
               20                  25                  30
   Ala Ala Leu Gln Lys Cys Ile Ala Gln Ala Thr Ser Gly Asn Asn Lys
           35                  40                  45
   Gly Gly Thr Leu Lys Leu Thr Gln Ala Gln Leu Asp Lys Ile Phe Pro
       50                  55                  60
   Val Gly Ala Ser Ser Gly Arg Asn Ala Lys Phe Leu Lys Pro Leu Asn
   65                  70                  75                  80
   Asp Leu Phe Glu Lys Thr Glu Ile Asn Thr Val Asn Arg Val Ala Gly
                   85                  90                  95
   Phe Leu Ser Gln Ile Gly Val Glu Ser Ala Glu Phe Arg Tyr Val Arg
               100                 105                 110
   Glu Leu Gly Asn Asp Ala Tyr Phe Asp Lys Tyr Asp Thr Gly Pro Ile
           115                 120                 125
   Ala Glu Arg Leu Gly Asn Thr Pro Gln Lys Asp Gly Asp Gly Ala Lys
       130                 135                 140
   Tyr Lys Gly Arg Gly Leu Ile Gln Val Thr Gly Leu Ala Asn Tyr Lys
   145                 150                 155                 160
   Ala Cys Gly Lys Ala Leu Gly Leu Asp Leu Val Asn His Pro Glu Leu
                   165                 170                 175
   Leu Glu Gln Pro Glu Tyr Ala Val Ala Ser Ala Gly Trp Tyr Trp Asp
               180                 185                 190
   Thr Arg Asn Ile Asn Ala Ala Cys Asp Ala Asp Asp Ile Val Lys Ile
           195                 200                 205
   Thr Lys Leu Val Asn Gly Gly Thr Asn His Leu Ala Glu Arg Thr Ala
       210                 215                 220
   Tyr Tyr Lys Lys Ala Lys Ser Val Leu Thr Ser
   225                 230                 235
<210> 9
   <211> 263
   <212> **PRT**
   <213> unknown
<220>
   <223> Phage003/001_gp110
<400> 9
   Ala Ile Leu Lys Leu Gly Asn Arg Gly Ser Glu Val Lys Ala Leu Gln
   1               5                   10                  15
   Gln Ser Leu Asn Lys Ile Gly Phe Ser Leu Thr Ala Asp Gly Ile Phe
               20                  25                  30
   Gly Lys Ala Thr Glu Asn Ala Val Lys Ser Val Gln Ala Gly Ala Gly
           35                  40                  45
   Leu Val Ile Asp Gly Ile Ala Gly Pro Lys Thr Phe Tyr Ala Ile Arg
       50                  55                  60
   Asn Ala Gly Asp Ala His Gln Glu His Leu Thr Glu Ala Asp Leu Val
   65                  70                  75                  80
   Asp Ala Ala Arg Glu Leu Gly Val Glu Leu Ala Ser Met Lys Ala Val
                   85                  90                  95
   Asn Gln Val Glu Ser Arg Gly Thr Gly Phe Thr Lys Thr Gly Lys Ile
               100                 105                 110
   Lys Thr Leu Phe Glu Arg His Ile Met Tyr Lys Lys Val Thr Ala Lys
           115                 120                 125
   Phe Gly Gln Ala Arg Ala Asn Ala Leu Tyr Gln Leu Tyr Pro Thr Leu
       130                 135                 140
   Val Asn Pro Asn Ser Gly Gly Tyr Ile Gly Gly Asp Ala Glu Leu Glu
   145                 150                 155                 160
   Arg Leu Gln Gly Ala Ile Ala Leu Asp Glu Asp Cys Ala Tyr Glu Ser
                   165                 170                 175
   Ala Ser Tyr Gly Leu Phe Gln Ile Met Gly Phe Asn Cys Gln Ile Cys
               180                 185                 190
   Gly Tyr Ser Asn Ala Lys Glu Met Phe Thr Asp Phe Leu Thr Gly Glu
           195                 200                 205
   Arg Ala His Leu Leu Ala Phe Val Lys Phe Ile Lys Ala Asp Ala Asn
       210                 215                 220
   Met Trp Lys Ala Leu Lys Asn Lys Asn Trp Ala Glu Phe Ala Arg Arg
   225                 230                 235                 240
   Tyr Asn Gly Pro Ala Tyr Ala Lys Asn Gln Tyr Asp Thr Lys Leu Ala
                   245                 250                 255
   Ala Ala Tyr Lys Ser Phe Cys
               260
<210> 10
   <211> 279
   <212> PRT
   <213> unknown
<220>
   <223> Smi01 endolysin
<400> 10
   Glu Tyr Asp Met Ile Leu Lys Phe Gly Ser Lys Gly Asp Ala Val Ala
   1               5                   10                  15
   Thr Leu Gln Lys Gln Leu Ala Lys Met Gly Tyr Lys Gly Val Lys Asp
               20                  25                  30
   Lys Pro Leu Ser Val Asp Gly His Phe Gly Glu Ser Thr Glu Phe Ala
           35                  40                  45
   Val Ile Gln Leu Gln Arg Lys Phe Gly Leu Val Ala Asp Gly Lys Val
       50                  55                  60
   Gly Asp Lys Thr Arg Gln Ala Leu Ala Gly Asp Ser Val Ser Lys Phe
   65                  70                  75                  80
   Leu Lys Asp Glu Asp Tyr Lys Lys Ala Ala Ile Arg Leu Lys Val Pro
                   85                  90                  95
   Glu Leu Val Ile Arg Val Phe Gly Ala Val Glu Gly Leu Gly Val Gly
               100                 105                 110
   Phe Leu Pro Asn Gly Lys Ala Lys Ile Leu Phe Glu Arg His Arg Met
           115                 120                 125
   Tyr Phe Tyr Leu Cys Gln Ala Leu Gly Lys Thr Phe Ala Asn Ser Gln
       130                 135                 140
   Val Lys Ile Thr Pro Asn Ile Val Asn Thr Leu Thr Gly Gly Tyr Lys
   145                 150                 155                 160
   Gly Asp Ala Ala Glu Tyr Thr Arg Leu Ser Met Ala Ile Asn Ile His
                   165                 170                 175
   Lys Glu Ser Ala Leu Met Ser Thr Ser Trp Gly Gln Phe Gln Ile Met
               180                 185                 190
   Gly Glu Asn Trp Lys Asp Leu Gly Tyr Ser Ser Val Gln Glu Phe Val
           195                 200                 205
   Asp Gln Gln Gln Leu Asn Glu Gly Asn Gln Leu Glu Ala Phe Ile Arg
       210                 215                 220
   Phe Ile Glu Trp Lys Pro Gly Leu Leu Glu Ala Leu Arg Lys Gln Asp
   225                 230                 235                 240
   Trp Asp Thr Val Phe Thr Leu Tyr Asn Gly Lys Asn Tyr Lys Lys Leu
   245 250 255
   Gly Tyr Gln Ala Lys Phe Gln Lys Glu Trp Asp His Leu Glu Pro Ile
   260 265 270
   Tyr Arg Glu Lys Thr Ala Ala
   275
<210> 11
   <211> 259
   <212> PRT
   <213> artificial
<220>
   <223> KZ144 endolysin
<400> 11
   Lys Val Leu Arg Lys Gly Asp Arg Gly Asp Glu Val Ser Gln Leu Gln
   1               5                   10                  15
   Thr Leu Leu Asn Leu Ser Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Ser Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Thr Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Ile Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Ala Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
   180 185 190
   Thr Asp Leu Tyr Leu Ala His Phe Phe Gly Pro Gly Ala Ala Arg Arg
   195 200 205
   Phe Leu Thr Thr Gly Gln Asn Glu Leu Ala Ala Thr His Phe Pro Lys
   210 215 220
   Glu Ala Gln Ala Asn Pro Ser Ile Phe Tyr Asn Lys Asp Gly Ser Pro
   225 230 235 240
   Lys Thr Ile Gln Glu Val Tyr Asn Leu Met Asp Gly Lys Val Ala Ala
   245 250 255
   His Arg Lys
<210> 12
   <211> 244
   <212> **PRT**
   <213> artificial
<220>
   <223> Lysostaphin
<400> 12
   Ala His Glu His Ser Ala Gln Trp Leu Asn Asn Tyr Lys Lys Gly Tyr
   1 5 10 15
   Gly Tyr Gly Pro Tyr Pro Leu Gly Ile Asn Gly Gly Met His Tyr Gly
   20 25 30
   Val Asp Phe Phe Met Asn Ile Gly Thr Pro Val Lys Ala Ile Ser Ser
   35 40 45
   Gly Lys Ile Val Glu Ala Gly Trp Ser Asn Tyr Gly Gly Gly Asn Gln
   50 55 60
   Ile Gly Leu Ile Glu Asn Asp Gly Val His Arg Gln Trp Tyr Met His
   65 70 75 80
   Leu Ser Lys Tyr Asn Val Lys Val Gly Asp Tyr Val Lys Ala Gly Gln
   85 90 95
   Ile Ile Gly Trp Ser Gly Ser Thr Gly Tyr Ser Thr Ala Pro His Leu
   100 105 110
   His Phe Gln Arg Met Val Asn Ser Phe Ser Asn Ser Thr Ala Gln Asp
           115                 120                 125
   Pro Met Pro Phe Leu Lys Ser Ala Gly Tyr Gly Lys Ala Gly Gly Thr
       130                 135                 140
   Val Thr Pro Thr Pro Asn Thr Gly Trp Lys Thr Asn Lys Tyr Gly Thr
   145                 150                 155                 160
   Leu Tyr Lys Ser Glu Ser Ala Ser Phe Thr Pro Asn Thr Asp Ile Ile
                   165                 170                 175
   Thr Arg Thr Thr Gly Pro Phe Arg Ser Met Pro Gln Ser Gly Val Leu
               180                 185                 190
   Lys Ala Gly Gln Thr Ile His Tyr Asp Glu Val Met Lys Gln Asp Gly
           195                 200                 205
   His Val Trp Val Gly Tyr Thr Gly Asn Ser Gly Gln Arg Ile Tyr Leu
       210                 215                 220
   Pro Val Arg Thr Trp Asn Lys Ser Thr Asn Thr Leu Gly Val Leu Trp
   225                 230                 235                 240
   Gly Thr Ile Lys
<210> 13
   <211> 6
   <212> PRT
   <213> artificial
<220>
   <223> synthetic sequence
<400> 13
   Lys Arg Lys Lys Arg Lys
   1               5
<210> 14
   <211> 5
   <212> PRT
   <213> artificial
<220>
   <223> synethtic sequence
<220>
   <221> misc_feature
   <222> (3)..(3)
   <223> Xaa can be any naturally occurring amino acid
<400> 14
   Lys Arg Xaa Lys Arg
   1 5
<210> 15
   <211> 5
   <212> **PRT**
   <213> artificial
<220>
   <223> synthetic sequence
<400> 15
   Lys Arg Ser Lys Arg
   1 5
<210> 16
   <211> 5
   <212> **PRT**
   <213> artificial
<220>
   <223> synthetic sequence
<400> 16
   Lys Arg Gly Ser Gly
   1 5
<210> 17
   <211> 9
   <212> **PRT**
   <213> artificial
<220>
   <223> synthetic sequence
<400> 17
   Lys Arg Lys Lys Arg Lys Lys Arg Lys
   1 5
<210> 18
   <211> 9
   <212> **PRT**
   <213> artificial
<220>
   <223> synthetic sequence
<400> 18
   Arg Arg Arg Arg Arg Arg Arg Arg Arg
   1 5
<210> 19
   <211> 8
   <212> **PRT**
   <213> artificial
<220>
   <223> synthetic sequence
<400> 19
   Lys Lys Lys Lys Lys Lys Lys Lys
   1               5
<210> 20
   <211> 10
   <212> PRT
   <213> artificial
<220>
   <223> synthetic sequence
<400> 20
   Lys Arg Lys Lys Arg Lys Lys Arg Lys Lys
   1               5                   10
<210> 21
   <211> 12
   <212> **PRT**
   <213> artificial
<220>
   <223> synthetic sequence
<400> 21
   Lys Arg Lys Lys Arg Lys Lys Arg Lys Lys Arg Lys
   1               5                   10
<210> 22
   <211> 14
   <212> PRT
   <213> artificial
<220>
   <223> synthetic sequence
<400> 22
   Lys Arg Lys Lys Arg Lys Lys Arg Lys Lys Arg Lys Lys Arg
   1               5                   10
<210> 23
   <211> 16
   <212> **PRT**
   <213> artificial
<220>
   <223> synthetic sequence
<400> 23
   Lys Lys Lys Lys Lys Lys Lys Lys Lys Lys Lys Lys Lys Lys Lys Lys
   1               5                   10                  15
<210> 24
   <211> 18
   <212> **PRT**
   <213> artificial
<220>
   <223> synthetic sequence
<400> 24
   Lys Arg Lys Lys Arg Lys Lys Arg Lys Lys Arg Lys Lys Arg Lys Lys
   1               5                   10                  15
   Arg Lys
<210> 25
   <211> 19
   <212> PRT
   <213> artificial
<220>
   <223> synthetic sequence
<400> 25
   Lys Arg Lys Lys Arg Lys Lys Arg Lys Lys Arg Lys Lys Arg Lys Lys
   1               5                   10                  15
   Arg Lys Lys
<210> 26
   <211> 19
   <212> PRT
   <213> artificial
<220>
   <223> synthetic sequence
<400> 26
   Arg Arg Arg Arg Arg Arg Arg Arg Arg Arg Arg Arg Arg Arg Arg Arg
   1               5                   10                  15
   Arg Arg Arg
<210> 27
   <211> 19
   <212> PRT
   <213> artificial
<220>
   <223> synthetic sequence
<400> 27
   Lys Lys Lys Lys Lys Lys Lys Lys Lys Lys Lys Lys Lys Lys Lys Lys
   1               5                   10                  15
   Lys Lys Lys
<210> 28
   <211> 20
   <212> PRT
   <213> artificial
<220>
   <223> synthetic sequence
<400> 28
   Lys Arg Lys Lys Arg Lys Lys Arg Lys Arg Ser Lys Arg Lys Lys Arg
   1               5                   10                  15
   Lys Lys Arg Lys
               20
<210> 29
   <211> 21
   <212> PRT
   <213> artificial
<220>
   <223> synthetic sequence
<400> 29
   Lys Arg Lys Lys Arg Lys Lys Arg Lys Arg Ser Lys Arg Lys Lys Arg
   1               5                   10                  15
   Lys Lys Arg Lys Lys
               20
<210> 30
   <211> 21
   <212> **PRT**
   <213> artificial
<220>
   <223> synthetic sequence
<400> 30
   Lys Arg Lys Lys Arg Lys Lys Arg Lys Lys Arg Lys Lys Arg Lys Lys
   1               5                   10                  15
   Arg Lys Lys Arg Lys
               20
<210> 31
   <211> 22
   <212> PRT
   <213> artificial
<220>
   <223> synthetic sequence
<400> 31
   Lys Arg Lys Lys Arg Lys Lys Arg Lys Arg Gly Ser Gly Lys Arg Lys
   1               5                   10                  15
   Lys Arg Lys Lys Arg Lys
               20
<210> 32
   <211> 24
   <212> **PRT**
   <213> artificial
<220>
   <223> synthetic sequence
<400> 32
   Lys Arg Lys Lys Arg Lys Lys Arg Lys Arg Gly Ser Gly Ser Gly Lys
   1               5                   10                  15
   Arg Lys Lys Arg Lys Lys Arg Lys
               20
<210> 33
   <211> 25
   <212> PRT
   <213> artificial
<220>
   <223> synthetic sequence
<400> 33
   Lys Arg Lys Lys Arg Lys Lys Arg Lys Lys Arg Lys Lys Arg Lys Lys
   1               5                   10                  15
   Arg Lys Lys Arg Lys Lys Arg Lys Lys
               20                  25
<211> 31
   <212> PRT
   <213> artificial
<220>
   <223> synthetic sequence
<400> 34
<210> 35
   <211> 38
   <212> PRT
   <213> artificial
<220>
   <223> synthetic sequence
<400> 35
   Lys Arg Lys Lys Arg Lys Lys Arg Lys Arg Gly Ser Gly Ser Gly Lys
   1               5                   10                  15
   Arg Lys Lys Arg Lys Lys Arg Lys Gly Ser Gly Ser Gly Lys Arg Lys
               20                  25                  30
   Lys Arg Lys Lys Arg Lys
           35
<210> 36
   <211> 39
   <212> PRT
   <213> artificial
<220>
   <223> synthetic sequence
<400> 36
   Lys Arg Lys Lys Arg Lys Lys Arg Lys Lys Arg Lys Lys Arg Lys Lys
   1               5                   10                  15
   Arg Lys Lys Arg Lys Lys Arg Lys Lys Arg Lys Lys Arg Lys Lys Arg
               20                  25                  30
   Lys Lys Arg Lys Lys Arg Lys
           35
<210> 37
   <211> 42
   <212> PRT
   <213> artificial
<220>
   <223> synthetic sequence
<400> 37
   Lys Arg Lys Lys Arg Lys Lys Arg Lys Arg Ser Lys Arg Lys Lys Arg
   1               5                   10                  15
   Lys Lys Arg Lys Arg Ser Lys Arg Lys Lys Arg Lys Lys Arg Lys Arg
               20                  25                  30
   Ser Lys Arg Lys Lys Arg Lys Lys Arg Lys
           35                  40
<210> 38
   <211> 37
   <212> PRT
   <213> Homo sapiens
<400> 38
   Leu Leu Gly Asp Phe Phe Arg Lys Ser Lys Glu Lys Ile Gly Lys Glu
   1               5                   10                  15
   Phe Lys Arg Ile Val Gln Arg Ile Lys Asp Phe Leu Arg Asn Leu Val
               20                  25                  30
   Pro Arg Thr Glu Ser
           35
<210> 39
   <211> 29
   <212> PRT
   <213> unknown
<220>
   <223> SMAP-29 sheep
<400> 39
   Arg Gly Leu Arg Arg Leu Gly Arg Lys Ile Ala His Gly Val Lys Lys
   1               5                   10                  15
   Tyr Gly Pro Thr Val Leu Arg Ile Ile Arg Ile Ala Gly
               20                  25
<210> 40
   <211> 13
   <212> **PRT**
   <213> unknown
<220>
   <223> Indolicidine bovine
<400> 40
   Ile Leu Pro Trp Lys Trp Pro Trp Trp Pro Trp Arg Arg
   1               5                   10
<210> 41
   <211> 18
   <212> **PRT**
   <213> unknown
<220>
   <223> Protegrin Porcine
<400> 41
   Arg Gly Gly Arg Leu Cys Tyr Cys Arg Arg Arg Phe Cys Val Cys Val
   1               5                   10                  15
   Gly Arg
<210> 42
   <211> 31
   <212> PRT
   <213> unknown
<220>
   <223> Cecropin P1 Mammal (pig)
<400> 42
   Ser Trp Leu Ser Lys Thr Ala Lys Lys Leu Glu Asn Ser Ala Lys Lys
   1               5                   10                  15
   Arg Ile Ser Glu Gly Ile Ala Ile Ala Ile Gln Gly Gly Pro Arg
               20                  25                  30
<210> 43
   <211> 23
   <212> PRT
   <213> unknown
<220>
   <223> Magainin frog
<400> 43
   Gly Ile Gly Lys Phe Leu His Ser Ala Lys Lys Phe Gly Lys Ala Phe
   1               5                   10                  15
   Val Gly Glu Ile Met Asn Ser
               20
<210> 44
   <211> 25
   <212> PRT
   <213> unknown
<220>
   <223> Pleurocidin fish
<400> 44
   Gly Trp Gly Ser Phe Phe Lys Lys Ala Ala His Val Gly Lys His Val
   1               5                   10                  15
   Gly Lys Ala Ala Leu Thr His Tyr Leu
               20                  25
<210> 45
   <211> 36
   <212> **PRT**
   <213> Aedes aegypti
<400> 45
   Gly Gly Leu Lys Lys Leu Gly Lys Lys Leu Glu Gly Ala Gly Lys Arg
   1               5                   10                  15
   Val Phe Asn Ala Ala Glu Lys Ala Leu Pro Val Val Ala Gly Ala Lys
               20                  25                  30
   Ala Leu Arg Lys
           35
<210> 46
   <211> 40
   <212> **PRT**
   <213> Drosophila melanogaster
<400> 46
   Gly Trp Leu Lys Lys Ile Gly Lys Lys Ile Glu Arg Val Gly Gln His
   1               5                   10                  15
   Thr Arg Asp Ala Thr Ile Gln Gly Leu Gly Ile Pro Gln Gln Ala Ala
               20                  25                  30
   Asn Val Ala Ala Thr Ala Arg Gly
           35                  40
<210> 47
   <211> 21
   <212> **PRT**
   <213> unknown
<220>
   <223> Buforin II vertebrate
<400> 47
   Thr Arg Ser Ser Arg Ala Gly Leu Gln Phe Pro Val Gly Arg Val His
   1               5                   10                  15
   Arg Leu Leu Arg Lys
               20
<210> 48
   <211> 39
   <212> PRT
   <213> unknown
<220>
   <223> Sarcotoxin IA Fly
<400> 48
   Gly Trp Leu Lys Lys Ile Gly Lys Lys Ile Glu Arg Val Gly Gln His
   1               5                   10                  15
   Thr Arg Asp Ala Thr Ile Gln Gly Leu Gly Ile Ala Gln Gln Ala Ala
               20                  25                  30
   Asn Val Ala Ala Thr Ala Arg
           35
<210> 49
   <211> 17
   <212> **PRT**
   <213> Apis mellifera
<400> 49
   Ala Asn Arg Pro Val Tyr Ile Pro Pro Pro Arg Pro Pro His Pro Arg
   1 5 10 15
   Leu
<210> 50
   <211> 24
   <212> PRT
   <213> unknown
<220>
   <223> Ascaphine 5 Frog
<400> 50
   Gly Ile Lys Asp Trp Ile Lys Gly Ala Ala Lys Lys Leu Ile Lys Thr
   1 5 10 15
   Val Ala Ser His Ile Ala Asn Gln
   20
<210> 51
   <211> 22
   <212> PRT
   <213> unknown
<220>
   <223> Nigrocine 2 Frog
<400> 51
   Gly Leu Leu Ser Lys Val Leu Gly Val Gly Lys Lys Val Leu Cys Gly
   1 5 10 15
   Val Ser Gly Leu Val Cys
   20
<210> 52
   <211> 24
   <212> PRT
   <213> unknown
<220>
   <223> Pseudin 1 Rana Frog
<400> 52
   Gly Leu Asn Thr Leu Lys Lys Val Phe Gln Gly Leu His Glu Ala Ile
   1 5 10 15
   Lys Leu Ile Asn Asn His Val Gln
   20
<210> 53
   <211> 18
   <212> PRT
   <213> unknown
<220>
   <223> Ranalexin Frog
<400> 53
   Phe Leu Gly Gly Leu Ile Val Pro Ala Met Ile Cys Ala Val Thr Lys
   1               5                   10                  15
   Lys Cys
<210> 54
   <211> 26
   <212> **PRT**
   <213> unknown
<220>
   <223> Melittin bee
<400> 54
   Gly Ile Gly Ala Val Leu Lys Val Leu Thr Thr Gly Leu Pro Ala Leu
   1               5                   10                  15
   Ile Ser Trp Ile Lys Arg Lys Arg Gln Gln
               20                  25
<210> 55
   <211> 25
   <212> PRT
   <213> unknown
<220>
   <223> Lycotoxin 1 Spider
<400> 55
   Ile Trp Leu Thr Ala Leu Lys Phe Leu Gly Lys His Ala Ala Lys Lys
   1               5                   10                  15
   Leu Ala Lys Gln Gln Leu Ser Lys Leu
               20                  25
<210> 56
   <211> 19
   <212> **PRT**
   <213> unknown
<220>
   <223> Parasin 1 Fish
<400> 56
   Lys Gly Arg Gly Lys Gln Gly Gly Lys Val Arg Ala Lys Ala Lys Thr
   1               5                   10                  15
   Arg Ser Ser
<210> 57
   <211> 39
   <212> PRT
   <213> unknown
<220>
   <223> Buforin I Toad
<400> 57
   Ala Gly Arg Gly Lys Gln Gly Gly Lys Val Arg Ala Lys Ala Lys Thr
   1               5                   10                  15
   Arg Ser Ser Arg Ala Gly Leu Gln Phe Pro Val Gly Arg Val His Arg
               20                  25                  30
   Leu Leu Arg Lys Gly Asn Tyr
           35
<210> 58
   <211> 34
   <212> PRT
   <213> unknown
<220>
   <223> Dermaseptin 1 Frog
<400> 58
   Ala Leu Trp Lys Thr Met Leu Lys Lys Leu Gly Thr Met Ala Leu His
   1               5                   10                  15
   Ala Gly Lys Ala Ala Leu Gly Ala Ala Ala Asp Thr Ile Ser Gln Gly
               20                  25                  30
   Thr Gln
<210> 59
   <211> 12
   <212> **PRT**
   <213> unknown
<220>
   <223> Bactenecin 1 Cow
<400> 59
   Arg Leu Cys Arg Ile Val Val Ile Arg Val Cys Arg
   1               5                   10
<210> 60
   <211> 21
   <212> PRT
   <213> unknown
<220>
   <223> Thanatin Insect
<400> 60
   Gly Ser Lys Lys Pro Val Pro Ile Ile Tyr Cys Asn Arg Arg Thr Gly
   1               5                   10                  15
   Lys Cys Gln Arg Met
               20
<210> 61
   <211> 19
   <212> **PRT**
   <213> unknown
<220>
   <223> Brevinin 1T Rana frogs
<400> 61
   Val Asn Pro Ile Ile Leu Gly Val Leu Pro Lys Val Cys Leu Ile Thr
   1               5                   10                  15
   Lys Lys Cys
<210> 62
   <211> 26
   <212> PRT
   <213> unknown
<220>
   <223> Ranateurin 1 Rana frog
<400> 62
   Ser Met Leu Ser Val Leu Lys Asn Leu Gly Lys Val Gly Leu Gly Phe
   1               5                   10                  15
   Val Ala Cys Lys Ile Asn Ile Lys Gln Cys
               20                  25
<210> 63
   <211> 46
   <212> PRT
   <213> unknown
<220>
   <223> Esculentin 1 Rana frogs
<400> 63
   Gly Ile Phe Ser Lys Leu Gly Arg Lys Lys Ile Lys Asn Leu Leu Ile
   1               5                   10                  15
   Ser Gly Leu Lys Asn Val Gly Lys Glu Val Gly Met Asp Val Val Arg
               20                  25                  30
   Thr Gly Ile Lys Ile Ala Gly Cys Lys Ile Lys Gly Glu Cys
           35                  40                  45
<210> 64
   <211> 17
   <212> **PRT**
   <213> Limulus polyphemus
<400> 64
   Arg Trp Cys Phe Arg Val Cys Tyr Arg Gly Ile Cys Tyr Arg Lys Cys
     1               5                   10                  15
   Arg
<210> 65
   <211> 25
   <212> PRT
   <213> unknown
<220>
   <223> Androctonin Scorpion
<400> 65
   Arg Ser Val Cys Arg Gln Ile Lys Ile Cys Arg Arg Arg Gly Gly Cys
   1               5                   10                  15
   Tyr Tyr Lys Cys Thr Asn Arg Pro Tyr
               20                  25
<210> 66
   <211> 30
   <212> PRT
   <213> Homo sapiens
<400> 66
   Asp Cys Tyr Cys Arg Ile Pro Ala Cys Ile Ala Gly Glu Arg Arg Tyr
   1               5                   10                  15
   Gly Thr Cys Ile Tyr Gln Gly Arg Leu Trp Ala Phe Cys Cys
               20                  25                  30
<210> 67
   <211> 38
   <212> **PRT**
   <213> unknown
<220>
   <223> beta-defensin cow
<400> 67
   Asn Pro Val Ser Cys Val Arg Asn Lys Gly Ile Cys Val Pro Ile Arg
   1               5                   10                  15
   Cys Pro Gly Ser Met Lys Gln Ile Gly Thr Cys Val Gly Arg Ala Val
               20                  25                  30
   Lys Cys Cys Arg Lys Lys
           35
<210> 68
   <211> 18
   <212> PRT
   <213> unknown
<220>
   <223> theta-defensin monkey
<400> 68
   Gly Phe Cys Arg Cys Leu Cys Arg Arg Gly Val Cys Arg Cys Ile Cys
   1               5                   10                  15
   Thr Arg
<210> 69
   <211> 40
   <212> PRT
   <213> unknown
<220>
   <223> defensin (sapecin A) insect
<400> 69
   Ala Thr Cys Asp Leu Leu Ser Gly Thr Gly Ile Asn His Ser Ala Cys
   1               5                   10                  15
   Ala Ala His Cys Leu Leu Arg Gly Asn Arg Gly Gly Tyr Cys Asn Gly
               20                  25                  30
   Lys Ala Val Cys Val Cys Arg Asn
           35                  40
<210> 70
   <211> 46
   <212> PRT
   <213> unknown
<220>
   <223> Thionin (crambin) plant
<400> 70
   Thr Thr Cys Cys Pro Ser Ile Val Ala Arg Ser Asn Phe Asn Val Cys
   1               5                   10                  15
   Arg Ile Pro Gly Thr Pro Glu Ala Ile Cys Ala Thr Tyr Thr Gly Cys
               20                  25                  30
   Ile Ile Ile Pro Gly Ala Thr Cys Pro Gly Asp Tyr Ala Asn
           35                  40                  45
<210> 71
   <211> 50
   <212> **PRT**
   <213> unknown
<220>
   <223> defensin from radish
<400> 71
   Gln Lys Leu Cys Gln Arg Pro Ser Gly Thr Trp Ser Gly Val Cys Gly
   1               5                   10                  15
   Asn Asn Asn Ala Cys Lys Asn Gln Cys Ile Arg Leu Glu Lys Ala Arg
               20                  25                  30
   His Gly Ser Cys Asn Tyr Val Phe Pro Ala His Cys Ile Cys Tyr Phe
           35                  40                  45
   Pro Cys
       50
<210> 72
   <211> 44
   <212> PRT
   <213> Drosophila melanogaster
<400> 72
   Asp Cys Leu Ser Gly Arg Tyr Lys Gly Pro Cys Ala Val Trp Asp Asn
   1 5 10 15
   Glu Thr Cys Arg Arg Val Cys Lys Glu Glu Gly Arg Ser Ser Gly His
   20 25 30
   Cys Ser Pro Ser Leu Lys Cys Trp Cys Glu Gly Cys
   35 40
<210> 73
   <211> 25
   <212> PRT
   <213> Homo sapiens
<400> 73
   Asp Thr His Phe Pro Ile Cys Ile Phe Cys Cys Gly Cys Cys His Arg
   1 5 10 15
   Ser Lys Cys Gly Met Cys Cys Lys Thr
   20 25
<210> 74
   <211> 44
   <212> PRT
   <213> unknown
<220>
   <223> Bac 5 Cow
<400> 74
   Arg Phe Arg Pro Pro Ile Arg Arg Pro Pro Ile Arg Pro Pro Phe Tyr
   1 5 10 15
   Pro Pro Phe Arg Pro Pro Ile Arg Pro Pro Ile Phe Pro Pro Ile Arg
   20 25 30
   Pro Pro Phe Arg Pro Pro Leu Gly Arg Pro Phe Pro
   35 40
<210> 75
   <211> 39
   <212> PRT
   <213> unknown
<220>
   <223> PR-39 Pig
<400> 75
   Arg Arg Arg Pro Arg Pro Pro Tyr Leu Pro Arg Pro Arg Pro Pro Pro
   1               5                   10                  15
   Phe Phe Pro Pro Arg Leu Pro Pro Arg Ile Pro Pro Gly Phe Pro Pro
               20                  25                  30
   Arg Phe Pro Pro Arg Phe Pro
           35
<210> 76
   <211> 20
   <212> PRT
   <213> unknown
<220>
   <223> Pyrrhocoricin Insect
<400> 76
   Val Asp Lys Gly Ser Tyr Leu Pro Arg Pro Thr Pro Pro Arg Pro Ile
   1               5                   10                  15
   Tyr Asn Arg Asn
               20
<210> 77
   <211> 24
   <212> **PRT**
   <213> Homo sapiens
<400> 77
   Asp Ser His Ala Lys Arg His His Gly Tyr Lys Arg Lys Phe His Glu
   1               5                   10                  15
   Lys His His Ser His Arg Gly Tyr
               20
<210> 78
   <211> 26
   <212> PRT
   <213> unknown
<220>
   <223> Macedocin
<400> 78
   Gly Lys Asn Gly Val Phe Lys Thr Ile Ser His Glu Cys His Leu Asn
   1               5                   10                  15
   Thr Trp Ala Phe Leu Ala Thr Cys Cys Ser
               20                  25
<210> 79
   <211> 22
   <212> PRT
   <213> unknown
<220>
   <223> Macedocin (Trunc)
<400> 79
   Gly Lys Asn Gly Val Phe Lys Thr Ile Ser His Glu Cys His Leu Asn
   1               5                   10                  15
   Thr Trp Ala Phe Leu Ala
               20
<210> 80
   <211> 28
   <212> **PRT**
   <213> unknown
<220>
   <223> D16
<400> 80
   Ala Cys Lys Leu Lys Ser Leu Leu Lys Thr Leu Ser Lys Ala Lys Lys
   1               5                   10                  15
   Lys Lys Leu Lys Thr Leu Leu Lys Ala Leu Ser Lys
               20                  25
<210> 81
   <211> 17
   <212> **PRT**
   <213> unknown
<220>
   <223> CPF-C1
<400> 81
   Gly Phe Gly Ser Leu Leu Gly Lys Ala Leu Arg Leu Gly Ala Asn Val
   1               5                   10                  15
   Leu
<210> 82
   <211> 34
   <212> PRT
   <213> unknown
<220>
   <223> TL-ColM
<400> 82
   Glu Thr Leu Thr Val His Ala Pro Ser Pro Ser Thr Asn Leu Pro Ser
   1               5                   10                  15
   Tyr Gly Asn Gly Ala Phe Ser Leu Ser Ala Pro His Val Pro Gly Ala
               20                  25                  30
   Gly Pro
<210> 83
   <211> 26
   <212> **PRT**
   <213> unknown
<220>
   <223> TM-174E
<400> 83
   Leu Ile Ser Lys Gly Trp Pro Tyr Leu Leu Val Val Val Leu Gly Ala
   1               5                   10                  15
   Thr Ile Tyr Phe Trp Gly Asn Ser Asn Gly
               20                  25
<210> 84
   <211> 23
   <212> **PRT**
   <213> unknown
<220>
   <223> MSI-594
<400> 84
   Gly Ile Gly Lys Phe Leu Lys Lys Ala Lys Lys Gly Ile Gly Ala Val
   1               5                   10                  15
   Leu Lys Val Leu Thr Thr Gly
               20
<210> 85
   <211> 19
   <212> PRT
   <213> unknown
<220>
   <223> ECP19
<400> 85
   Arg Pro Pro Gln Phe Thr Arg Ala Gln Trp Phe Ala Ile Gln His Ile
   1 5 10 15
   Ser Leu Asn
<210> 86
   <211> 45
   <212> **PRT**
   <213> unknown
<220>
   <223> ECP45
<400> 86
   Arg Pro Pro Gln Phe Thr Arg Ala Gln Trp Phe Ala Ile Gln His Ile
   1 5 10 15
   Ser Leu Asn Pro Pro Arg Cys Thr Ile Ala Met Arg Ala Ile Asn Asn
   20 25 30
   Tyr Arg Trp Arg Cys Lys Asn Gln Asn Thr Phe Leu Arg
   35 40 45
<210> 87
   <211> 50
   <212> PRT
   <213> unknown
<220>
   <223> ColicinE3_1-51 (S37F)
<400> 87
   Ser Gly Gly Asp Gly Arg Gly His Asn Thr Gly Ala His Ser Thr Ser
   1 5 10 15
   Gly Asn Ile Asn Gly Gly Pro Thr Gly Leu Gly Val Gly Gly Gly Ala
   20 25 30
   Ser Asp Gly Phe Gly Trp Ser Ser Glu Asn Asn Pro Trp Gly Gly Gly
   35 40 45
   Ser Gly
   50
<210> 88
   <211> 68
   <212> PRT
   <213> unknown
<220>
   <223> ColicinE3_1-69 (S37F)
<400> 88
   Ser Gly Gly Asp Gly Arg Gly His Asn Thr Gly Ala His Ser Thr Ser
   1               5                   10                  15
   Gly Asn Ile Asn Gly Gly Pro Thr Gly Leu Gly Val Gly Gly Gly Ala
               20                  25                  30
   Ser Asp Gly Phe Gly Trp Ser Ser Glu Asn Asn Pro Trp Gly Gly Gly
           35                  40                  45
   Ser Gly Ser Gly Ile His Trp Gly Gly Gly Ser Gly His Gly Asn Gly
       50                  55                  60
   Gly Gly Asn Gly
   65
<210> 89
   <211> 52
   <212> PRT
   <213> unknown
<220>
   <223> ColicinD_1-53
<400> 89
   Ser Asp Tyr Glu Gly Ser Gly Pro Thr Glu Gly Ile Asp Tyr Gly His
   1               5                   10                  15
   Ser Met Val Val Trp Pro Ser Thr Gly Leu Ile Ser Gly Gly Asp Val
               20                  25                  30
   Lys Pro Gly Gly Ser Ser Gly Ile Ala Pro Ser Met Pro Pro Gly Trp
           35                  40                  45
   Gly Asp Tyr Ser
       50
<210> 90
   <211> 34
   <212> PRT
   <213> Limulus polyphemus
<400> 90
   Gly Phe Lys Leu Lys Gly Met Ala Arg Ile Ser Cys Leu Pro Asn Gly
   1               5                   10                  15
   Gln Trp Ser Asn Phe Pro Pro Lys Cys Ile Arg Glu Cys Ala Met Val
               20                  25                  30
   Ser Ser
<210> 91
   <211> 18
   <212> **PRT**
   <213> artificial
<220>
   <223> synthetic sequence
<400> 91
   Gly Phe Phe Ile Pro Ala Val Ile Leu Pro Ser Ile Ala Phe Leu Ile
   1               5                   10                  15
   Val Pro
<210> 92
   <211> 5
   <212> **PRT**
   <213> artificial
<220>
   <223> synthetic sequence
<400> 92
   Phe Phe Val Ala Pro
   1               5
<210> 93
   <211> 13
   <212> **PRT**
   <213> unknown
<220>
   <223> alpha4-helix of T4 lysozyme
<400> 93
   Pro Asn Arg Ala Lys Arg Val Ile Thr Thr Phe Arg Thr
   1               5                   10
<210> 94
   <211> 27
   <212> PRT
   <213> artificial
<220>
   <223> synthetic sequence
<400> 94
   Lys Arg Trp Val Lys Arg Val Lys Arg Val Lys Arg Trp Val Lys Arg
   1               5                   10                  15
   Val Val Arg Val Val Lys Arg Trp Val Lys Arg
               20                  25
<210> 95
   <211> 25
   <212> PRT
   <213> artificial
<220>
   <223> synthetic sequence
<400> 95
   Gly Lys Pro Gly Trp Leu Ile Lys Lys Ala Leu Val Phe Lys Lys Leu
   1               5                   10                  15
   Ile Arg Arg Pro Leu Lys Arg Leu Ala
               20                  25
<210> 96
   <211> 253
   <212> PRT
   <213> artificial
<220>
   <223> Lysostaphin-PK
<400> 96
   Ala His Glu His Ser Ala Gln Trp Leu Asn Asn Tyr Lys Lys Gly Tyr
   1               5                   10                  15
   Gly Tyr Gly Pro Tyr Pro Leu Gly Ile Asn Gly Gly Met His Tyr Gly
               20                  25                  30
   Val Asp Phe Phe Met Asn Ile Gly Thr Pro Val Lys Ala Ile Ser Ser
           35                  40                  45
   Gly Lys Ile Val Glu Ala Gly Trp Ser Asn Tyr Gly Gly Gly Asn Gln
       50                  55                  60
   Ile Gly Leu Ile Glu Asn Asp Gly Val His Arg Gln Trp Tyr Met His
   65                  70                  75                  80
   Leu Ser Lys Tyr Asn Val Lys Val Gly Asp Tyr Val Lys Ala Gly Gln
                   85                  90                  95
   Ile Ile Gly Trp Ser Gly Ser Thr Gly Tyr Ser Thr Ala Pro His Leu
               100                 105                 110
   His Phe Gln Arg Met Val Asn Ser Phe Ser Asn Ser Thr Ala Gln Asp
           115                 120                 125
   Pro Met Pro Phe Leu Lys Ser Ala Gly Tyr Gly Lys Ala Gly Gly Thr
       130                 135                 140
   Val Thr Pro Thr Pro Asn Thr Gly Trp Lys Thr Asn Lys Tyr Gly Thr
   145                 150                 155                 160
   Leu Tyr Lys Ser Glu Ser Ala Ser Phe Thr Pro Asn Thr Asp Ile Ile
                   165                 170                 175
   Thr Arg Thr Thr Gly Pro Phe Arg Ser Met Pro Gln Ser Gly Val Leu
               180                 185                 190
   Lys Ala Gly Gln Thr Ile His Tyr Asp Glu Val Met Lys Gln Asp Gly
           195                 200                 205
   His Val Trp Val Gly Tyr Thr Gly Asn Ser Gly Gln Arg Ile Tyr Leu
       210                 215                 220
   Pro Val Arg Thr Trp Asn Lys Ser Thr Asn Thr Leu Gly Val Leu Trp
   225                 230                 235                 240
   Gly Thr Ile Lys Lys Arg Lys Lys Arg Lys Lys Arg Lys
                   245                 250
<210> 97
   <211> 283
   <212> PRT
   <213> artificial
<220>
   <223> SarcotoxinIA-Lysostaphin
<400> 97
   Gly Trp Leu Lys Lys Ile Gly Lys Lys Ile Glu Arg Val Gly Gln His
   1               5                   10                  15
   Thr Arg Asp Ala Thr Ile Gln Gly Leu Gly Ile Ala Gln Gln Ala Ala
               20                  25                  30
   Asn Val Ala Ala Thr Ala Arg Ala His Glu His Ser Ala Gln Trp Leu
           35                  40                  45
   Asn Asn Tyr Lys Lys Gly Tyr Gly Tyr Gly Pro Tyr Pro Leu Gly Ile
       50                  55                  60
   Asn Gly Gly Met His Tyr Gly Val Asp Phe Phe Met Asn Ile Gly Thr
   65                  70                  75                  80
   Pro Val Lys Ala Ile Ser Ser Gly Lys Ile Val Glu Ala Gly Trp Ser
                   85                  90                  95
   Asn Tyr Gly Gly Gly Asn Gln Ile Gly Leu Ile Glu Asn Asp Gly Val
               100                 105                 110
   His Arg Gln Trp Tyr Met His Leu Ser Lys Tyr Asn Val Lys Val Gly
           115                 120                 125
   Asp Tyr Val Lys Ala Gly Gln Ile Ile Gly Trp Ser Gly Ser Thr Gly
       130                 135                 140
   Tyr Ser Thr Ala Pro His Leu His Phe Gln Arg Met Val Asn Ser Phe
   145                 150                 155                 160
   Ser Asn Ser Thr Ala Gln Asp Pro Met Pro Phe Leu Lys Ser Ala Gly
                   165                 170                 175
   Tyr Gly Lys Ala Gly Gly Thr Val Thr Pro Thr Pro Asn Thr Gly Trp
               180                 185                 190
   Lys Thr Asn Lys Tyr Gly Thr Leu Tyr Lys Ser Glu Ser Ala Ser Phe
           195                 200                 205
   Thr Pro Asn Thr Asp Ile Ile Thr Arg Thr Thr Gly Pro Phe Arg Ser
       210                 215                 220
   Met Pro Gln Ser Gly Val Leu Lys Ala Gly Gln Thr Ile His Tyr Asp
   225                 230                 235                 240
   Glu Val Met Lys Gln Asp Gly His Val Trp Val Gly Tyr Thr Gly Asn
                   245                 250                 255
   Ser Gly Gln Arg Ile Tyr Leu Pro Val Arg Thr Trp Asn Lys Ser Thr
               260                 265                 270
   Asn Thr Leu Gly Val Leu Trp Gly Thr Ile Lys
           275                 280
<210> 98
   <211> 517
   <212> PRT
   <213> artificial
<220>
   <223> LysK-Magainin
<400> 98
   Ala Lys Thr Gln Ala Glu Ile Asn Lys Arg Leu Asp Ala Tyr Ala Lys
   1               5                   10                  15
   Gly Thr Val Asp Ser Pro Tyr Arg Val Lys Lys Ala Thr Ser Tyr Asp
               20                  25                  30
   Pro Ser Phe Gly Val Met Glu Ala Gly Ala Ile Asp Ala Asp Gly Tyr
           35                  40                  45
   Tyr His Ala Gln Cys Gln Asp Leu Ile Thr Asp Tyr Val Leu Trp Leu
       50                  55                  60
   Thr Asp Asn Lys Val Arg Thr Trp Gly Asn Ala Lys Asp Gln Ile Lys
   65                  70                  75                  80
   Gln Ser Tyr Gly Thr Gly Phe Lys Ile His Glu Asn Lys Pro Ser Thr
                   85                  90                  95
   Val Pro Lys Lys Gly Trp Ile Ala Val Phe Thr Ser Gly Ser Tyr Glu
               100                 105                 110
   Gln Trp Gly His Ile Gly Ile Val Tyr Asp Gly Gly Asn Thr Ser Thr
           115                 120                 125
   Phe Thr Ile Leu Glu Gln Asn Trp Asn Gly Tyr Ala Asn Lys Lys Pro
       130                 135                 140
   Thr Lys Arg Val Asp Asn Tyr Tyr Gly Leu Thr His Phe Ile Glu Ile
   145                 150                 155                 160
   Pro Val Lys Ala Gly Thr Thr Val Lys Lys Glu Thr Ala Lys Lys Ser
   165 170 175
   Ala Ser Lys Thr Pro Ala Pro Lys Lys Lys Ala Thr Leu Lys Val Ser
   180 185 190
   Lys Asn His Ile Asn Tyr Thr Met Asp Lys Arg Gly Lys Lys Pro Glu
   195 200 205
   Gly Met Val Ile His Asn Asp Ala Gly Arg Ser Ser Gly Gln Gln Tyr
   210 215 220
   Glu Asn Ser Leu Ala Asn Ala Gly Tyr Ala Arg Tyr Ala Asn Gly Ile
   225 230 235 240
   Ala His Tyr Tyr Gly Ser Glu Gly Tyr Val Trp Glu Ala Ile Asp Ala
   245 250 255
   Lys Asn Gln Ile Ala Trp His Thr Gly Asp Gly Thr Gly Ala Asn Ser
   260 265 270
   Gly Asn Phe Arg Phe Ala Gly Ile Glu Val Cys Gln Ser Met Ser Ala
   275 280 285
   Ser Asp Ala Gln Phe Leu Lys Asn Glu Gln Ala Val Phe Gln Phe Thr
   290 295 300
   Ala Glu Lys Phe Lys Glu Trp Gly Leu Thr Pro Asn Arg Lys Thr Val
   305 310 315 320
   Arg Leu His Met Glu Phe Val Pro Thr Ala Cys Pro His Arg Ser Met
   325 330 335
   Val Leu His Thr Gly Phe Asn Pro Val Thr Gln Gly Arg Pro Ser Gln
   340 345 350
   Ala Ile Met Asn Lys Leu Lys Asp Tyr Phe Ile Lys Gln Ile Lys Asn
   355 360 365
   Tyr Met Asp Lys Gly Thr Ser Ser Ser Thr Val Val Lys Asp Gly Lys
   370 375 380
   Thr Ser Ser Ala Ser Thr Pro Ala Thr Arg Pro Val Thr Gly Ser Trp
   385 390 395 400
   Lys Lys Asn Gln Tyr Gly Thr Trp Tyr Lys Pro Glu Asn Ala Thr Phe
   405 410 415
   Val Asn Gly Asn Gln Pro Ile Val Thr Arg Ile Gly Ser Pro Phe Leu
   420 425 430
   Asn Ala Pro Val Gly Gly Asn Leu Pro Ala Gly Ala Thr Ile Val Tyr
   435 440 445
   Asp Glu Val Cys Ile Gln Ala Gly His Ile Trp Ile Gly Tyr Asn Ala
   450 455 460
   Tyr Asn Gly Asn Arg Val Tyr Cys Pro Val Arg Thr Cys Gln Gly Val
   465 470 475 480
   Pro Pro Asn Gln Ile Pro Gly Val Ala Trp Gly Val Phe Lys Gly Ile
   485 490 495
   Gly Lys Phe Leu His Ser Ala Lys Lys Phe Gly Lys Ala Phe Val Gly
   500 505 510
   Glu Ile Met Asn Ser
   515
<210> 99
   <211> 514
   <212> PRT
   <213> artificial
<220>
   <223> LysK-Ranalexin
<400> 99
   Ala Lys Thr Gln Ala Glu Ile Asn Lys Arg Leu Asp Ala Tyr Ala Lys
   1 5 10 15
   Gly Thr Val Asp Ser Pro Tyr Arg Val Lys Lys Ala Thr Ser Tyr Asp
   20 25 30
   Pro Ser Phe Gly Val Met Glu Ala Gly Ala Ile Asp Ala Asp Gly Tyr
   35 40 45
   Tyr His Ala Gln Cys Gln Asp Leu Ile Thr Asp Tyr Val Leu Trp Leu
   50 55 60
   Thr Asp Asn Lys Val Arg Thr Trp Gly Asn Ala Lys Asp Gln Ile Lys
   65 70 75 80
   Gln Ser Tyr Gly Thr Gly Phe Lys Ile His Glu Asn Lys Pro Ser Thr
   85 90 95
   Val Pro Lys Lys Gly Trp Ile Ala Val Phe Thr Ser Gly Ser Tyr Glu
               100                 105                 110
   Gln Trp Gly His Ile Gly Ile Val Tyr Asp Gly Gly Asn Thr Ser Thr
           115                 120                 125
   Phe Thr Ile Leu Glu Gln Asn Trp Asn Gly Tyr Ala Asn Lys Lys Pro
       130                 135                 140
   Thr Lys Arg Val Asp Asn Tyr Tyr Gly Leu Thr His Phe Ile Glu Ile
   145                 150                 155                 160
   Pro Val Lys Ala Gly Thr Thr Val Lys Lys Glu Thr Ala Lys Lys Ser
                   165                 170                 175
   Ala Ser Lys Thr Pro Ala Pro Lys Lys Lys Ala Thr Leu Lys Val Ser
               180                 185                 190
   Lys Asn His Ile Asn Tyr Thr Met Asp Lys Arg Gly Lys Lys Pro Glu
           195                 200                 205
   Gly Met Val Ile His Asn Asp Ala Gly Arg Ser Ser Gly Gln Gln Tyr
       210                 215                 220
   Glu Asn Ser Leu Ala Asn Ala Gly Tyr Ala Arg Tyr Ala Asn Gly Ile
   225                 230                 235                 240
   Ala His Tyr Tyr Gly Ser Glu Gly Tyr Val Trp Glu Ala Ile Asp Ala
                   245                 250                 255
   Lys Asn Gln Ile Ala Trp His Thr Gly Asp Gly Thr Gly Ala Asn Ser
               260                 265                 270
   Gly Asn Phe Arg Phe Ala Gly Ile Glu Val Cys Gln Ser Met Ser Ala
           275                 280                 285
   Ser Asp Ala Gln Phe Leu Lys Asn Glu Gln Ala Val Phe Gln Phe Thr
       290                 295                 300
   Ala Glu Lys Phe Lys Glu Trp Gly Leu Thr Pro Asn Arg Lys Thr Val
   305                 310                 315                 320
   Arg Leu His Met Glu Phe Val Pro Thr Ala Cys Pro His Arg Ser Met
                   325                 330                 335
   Val Leu His Thr Gly Phe Asn Pro Val Thr Gln Gly Arg Pro Ser Gln
               340                 345                 350
   Ala Ile Met Asn Lys Leu Lys Asp Tyr Phe Ile Lys Gln Ile Lys Asn
           355                 360                 365
   Tyr Met Asp Lys Gly Thr Ser Ser Ser Thr Val Val Lys Asp Gly Lys
       370                 375                 380
   Thr Ser Ser Ala Ser Thr Pro Ala Thr Arg Pro Val Thr Gly Ser Trp
   385                 390                 395                 400
   Lys Lys Asn Gln Tyr Gly Thr Trp Tyr Lys Pro Glu Asn Ala Thr Phe
                   405                 410                 415
   Val Asn Gly Asn Gln Pro Ile Val Thr Arg Ile Gly Ser Pro Phe Leu
               420                 425                 430
   Asn Ala Pro Val Gly Gly Asn Leu Pro Ala Gly Ala Thr Ile Val Tyr
           435                 440                 445
   Asp Glu Val Cys Ile Gln Ala Gly His Ile Trp Ile Gly Tyr Asn Ala
       450                 455                 460
   Tyr Asn Gly Asn Arg Val Tyr Cys Pro Val Arg Thr Cys Gln Gly Val
   465                 470                 475                 480
   Pro Pro Asn Gln Ile Pro Gly Val Ala Trp Gly Val Phe Lys Phe Leu
                   485                 490                 495
   Gly Gly Leu Ile Lys Ile Val Pro Ala Met Ile Cys Ala Val Thr Lys
               500                 505                 510
   Lys Cys
<210> 100
   <211> 497
   <212> PRT
   <213> artificial
<220>
   <223> PlySK1249-PK
<400> 100
   Gly Lys His Leu Val Ile Cys Gly His Gly Gln Gly Arg Thr Thr Tyr
   1               5                   10                  15
   Asp Pro Gly Ala Val Asn Ala Lys Leu Gly Ile Thr Glu Ala Gly Lys
               20                  25                  30
   Val Arg Glu Leu Ala Lys Leu Met Ser Lys Tyr Ser Gly Gln Gln Ile
   35 40 45
   Asp Phe Ile Thr Glu Gln Asn Val Tyr Asp Tyr Arg Ser Ile Thr Ser
   50 55 60
   Ile Gly Lys Gly Tyr Asp Ser Ile Thr Glu Leu His Phe Asn Ala Phe
   65 70 75 80
   Asn Gly Ser Ala Lys Gly Thr Glu Val Leu Ile Gln Ser Ser Leu Glu
   85 90 95
   Ala Asp Lys Glu Asp Met Ala Ile Leu Ser Leu Leu Ser Arg Tyr Phe
   100 105 110
   Gln Asn Arg Gly Ile Lys Lys Val Asp Trp Leu Tyr Asn Ala Asn Gln
   115 120 125
   Ala Ala Ser Arg Gly Tyr Thr Tyr Arg Leu Val Glu Ile Ala Phe Ile
   130 135 140
   Asp Asn Glu Gln Asp Met Ala Ile Phe Glu Thr Lys Lys Glu Asp Ile
   145 150 155 160
   Ala Lys Gly Leu Val Ser Ala Ile Thr Gly Val Glu Val Lys Thr Ile
   165 170 175
   Val Pro Ser Thr Pro Ser Ser Thr Val Gly Ser Ser Gly Thr Pro Ser
   180 185 190
   Lys Pro Ile Tyr Leu Val Gly Asp Ser Leu Arg Val Leu Pro His Ala
   195 200 205
   Thr His Tyr Gln Thr Gly Gln Lys Ile Ala Asn Trp Val Lys Gly Arg
   210 215 220
   Thr Tyr Lys Ile Leu Gln Glu Lys Asn Val His Gln Ser Asn Ser Leu
   225 230 235 240
   Arg Ala Tyr Leu Leu Asp Gly Ile Lys Ser Trp Val Leu Glu Gln Asp
   245 250 255
   Val Glu Gly Thr Thr Lys Gly His Ser Glu Gln Thr Tyr Gln Ala Gln
   260 265 270
   Lys Gly Asp Thr Tyr Tyr Gly Ile Ala Arg Lys Phe Gly Leu Thr Val
   275 280 285
   Asp Ala Leu Leu Ala Val Asn Gly Leu Lys Lys Thr Asp Ile Leu Arg
       290                 295                 300
   Val Gly Gln Thr Leu Lys Val Asn Ala Ala Ser Arg Ile Thr Thr Ala
   305                 310                 315                 320
   Ile Pro Thr Ser Val Ala Ser Arg Val Val Ala Ser Ala Leu Ser Lys
                   325                 330                 335
   Val Gly Gln Lys Val Thr Val Pro Ser Asn Pro Tyr Gly Gly Gln Cys
               340                 345                 350
   Val Ala Leu Val Asp Lys Ile Val Gln Glu Leu Thr Asp Lys Asn Met
           355                 360                 365
   Ser Tyr Thr Asn Ala Ile Asp Cys Leu Lys Lys Ala Lys Ser Asn Gly
       370                 375                 380
   Phe Gln Val Ile Tyr Asp Ala Trp Gly Val Asn Pro Lys Ala Gly Asp
   385                 390                 395                 400
   Phe Tyr Val Ile Glu Thr Asp Gly Leu Val Tyr Gly His Ile Gly Val
                   405                 410                 415
   Cys Val Thr Asp Ser Asp Gly Lys Ser Ile Asp Gly Val Glu Gln Asn
               420                 425                 430
   Ile Asp Gly Tyr Ser Asp His Asn Lys Asn Gly Ile Asn Asp Gln Leu
           435                 440                 445
   Glu Ile Gly Gly Gly Gly Ile Thr Arg Arg Val Lys Arg Gln Trp Met
       450                 455                 460
   Ala Asp Gly Ser Leu Tyr Asp Ser Thr Gly Thr Val Lys Leu Gly Lys
   465                 470                 475                 480
   Val Val Gly Trp Phe Arg Ile Ser Lys Arg Lys Lys Arg Lys Lys Arg
                   485                 490                 495
   Lys
<210> 101
   <211> 509
   <212> **PRT**
   <213> artificial
<220>
   <223> BuforinII-PlySK1249
<400> 101
   Thr Arg Ser Ser Arg Ala Gly Leu Gln Phe Pro Val Gly Arg Val His
   1               5                   10                  15
   Arg Leu Leu Arg Lys Gly Lys His Leu Val Ile Cys Gly His Gly Gln
               20                  25                  30
   Gly Arg Thr Thr Tyr Asp Pro Gly Ala Val Asn Ala Lys Leu Gly Ile
           35                  40                  45
   Thr Glu Ala Gly Lys Val Arg Glu Leu Ala Lys Leu Met Ser Lys Tyr
       50                  55                  60
   Ser Gly Gln Gln Ile Asp Phe Ile Thr Glu Gln Asn Val Tyr Asp Tyr
   65                  70                  75                  80
   Arg Ser Ile Thr Ser Ile Gly Lys Gly Tyr Asp Ser Ile Thr Glu Leu
                   85                  90                  95
   His Phe Asn Ala Phe Asn Gly Ser Ala Lys Gly Thr Glu Val Leu Ile
               100                 105                 110
   Gln Ser Ser Leu Glu Ala Asp Lys Glu Asp Met Ala Ile Leu Ser Leu
           115                 120                 125
   Leu Ser Arg Tyr Phe Gln Asn Arg Gly Ile Lys Lys Val Asp Trp Leu
       130                 135                 140
   Tyr Asn Ala Asn Gln Ala Ala Ser Arg Gly Tyr Thr Tyr Arg Leu Val
   145                 150                 155                 160
   Glu Ile Ala Phe Ile Asp Asn Glu Gln Asp Met Ala Ile Phe Glu Thr
                   165                 170                 175
   Lys Lys Glu Asp Ile Ala Lys Gly Leu Val Ser Ala Ile Thr Gly Val
               180                 185                 190
   Glu Val Lys Thr Ile Val Pro Ser Thr Pro Ser Ser Thr Val Gly Ser
           195                 200                 205
   Ser Gly Thr Pro Ser Lys Pro Ile Tyr Leu Val Gly Asp Ser Leu Arg
       210                 215                 220
   Val Leu Pro His Ala Thr His Tyr Gln Thr Gly Gln Lys Ile Ala Asn
   225                 230                 235                 240
   Trp Val Lys Gly Arg Thr Tyr Lys Ile Leu Gln Glu Lys Asn Val His
                   245                 250                 255
   Gln Ser Asn Ser Leu Arg Ala Tyr Leu Leu Asp Gly Ile Lys Ser Trp
               260                 265                 270
   Val Leu Glu Gln Asp Val Glu Gly Thr Thr Lys Gly His Ser Glu Gln
           275                 280                 285
   Thr Tyr Gln Ala Gln Lys Gly Asp Thr Tyr Tyr Gly Ile Ala Arg Lys
       290                 295                 300
   Phe Gly Leu Thr Val Asp Ala Leu Leu Ala Val Asn Gly Leu Lys Lys
   305                 310                 315                 320
   Thr Asp Ile Leu Arg Val Gly Gln Thr Leu Lys Val Asn Ala Ala Ser
                   325                 330                 335
   Arg Ile Thr Thr Ala Ile Pro Thr Ser Val Ala Ser Arg Val Val Ala
               340                 345                 350
   Ser Ala Leu Ser Lys Val Gly Gln Lys Val Thr Val Pro Ser Asn Pro
           355                 360                 365
   Tyr Gly Gly Gln Cys Val Ala Leu Val Asp Lys Ile Val Gln Glu Leu
       370                 375                 380
   Thr Asp Lys Asn Met Ser Tyr Thr Asn Ala Ile Asp Cys Leu Lys Lys
   385                 390                 395                 400
   Ala Lys Ser Asn Gly Phe Gln Val Ile Tyr Asp Ala Trp Gly Val Asn
                   405                 410                 415
   Pro Lys Ala Gly Asp Phe Tyr Val Ile Glu Thr Asp Gly Leu Val Tyr
               420                 425                 430
   Gly His Ile Gly Val Cys Val Thr Asp Ser Asp Gly Lys Ser Ile Asp
           435                 440                 445
   Gly Val Glu Gln Asn Ile Asp Gly Tyr Ser Asp His Asn Lys Asn Gly
       450                 455                 460
   Ile Asn Asp Gln Leu Glu Ile Gly Gly Gly Gly Ile Thr Arg Arg Val
   465                 470                 475                 480
   Lys Arg Gln Trp Met Ala Asp Gly Ser Leu Tyr Asp Ser Thr Gly Thr
   485 490 495
   Val Lys Leu Gly Lys Val Val Gly Trp Phe Arg Ile Ser
   500 505
<210> 102
   <211> 507
   <212> PRT
   <213> artificial
<220>
   <223> lambdaSa2lys-Cecropin A (D.me)
<400> 102
   Glu Ile Asn Thr Glu Ile Ala Ile Ala Trp Met Ser Ala Arg Gln Gly
   1               5                   10                  15
   Lys Val Ser Tyr Ser Met Asp Tyr Arg Asp Gly Pro Asn Ser Tyr Asp
               20                  25                  30
   Cys Ser Ser Ser Val Tyr Tyr Ala Leu Arg Ser Ala Gly Ala Ser Ser
           35                  40                  45
   Ala Gly Trp Ala Val Asn Thr Glu Tyr Met His Asp Trp Leu Ile Lys
       50                  55                  60
   Asn Gly Tyr Glu Leu Ile Ala Glu Asn Val Asp Trp Asn Ala Val Arg
   65                  70                  75                  80
   Gly Asp Ile Ala Ile Trp Gly Met Arg Gly His Ser Ser Gly Ala Gly
                   85                  90                  95
   Gly His Val Val Met Phe Ile Asp Pro Glu Asn Ile Ile His Cys Asn
               100                 105                 110
   Trp Ala Asn Asn Gly Ile Thr Val Asn Asn Tyr Asn Gln Thr Ala Ala
           115                 120                 125
   Ala Ser Gly Trp Met Tyr Cys Tyr Val Tyr Arg Leu Lys Ser Gly Ala
       130                 135                 140
   Ser Thr Gln Gly Lys Ser Leu Asp Thr Leu Val Lys Glu Thr Leu Ala
   145                 150                 155                 160
   Gly Asn Tyr Gly Asn Gly Glu Ala Arg Lys Ala Val Leu Gly Asn Gln
                   165                 170                 175
   Tyr Glu Ala Val Met Ser Val Ile Asn Gly Lys Thr Thr Thr Asn Gln
               180                 185                 190
   Lys Thr Val Asp Gln Leu Val Gln Glu Val Ile Ala Gly Lys His Gly
           195                 200                 205
   Asn Gly Glu Ala Arg Lys Lys Ser Leu Gly Ser Gln Tyr Asp Ala Val
       210                 215                 220
   Gln Lys Arg Val Thr Glu Leu Leu Lys Lys Gln Pro Ser Glu Pro Phe
   225                 230                 235                 240
   Lys Ala Gln Glu Val Asn Lys Pro Thr Glu Thr Lys Thr Ser Gln Thr
                   245                 250                 255
   Glu Leu Thr Gly Gln Ala Thr Ala Thr Lys Glu Glu Gly Asp Leu Ser
               260                 265                 270
   Phe Asn Gly Thr Ile Leu Lys Lys Ala Val Leu Asp Lys Ile Leu Gly
           275                 280                 285
   Asn Cys Lys Lys His Asp Ile Leu Pro Ser Tyr Ala Leu Thr Ile Leu
       290                 295                 300
   His Tyr Glu Gly Leu Trp Gly Thr Ser Ala Val Gly Lys Ala Asp Asn
   305                 310                 315                 320
   Asn Trp Gly Gly Met Thr Trp Thr Gly Gln Gly Asn Arg Pro Ser Gly
                   325                 330                 335
   Val Thr Val Thr Gln Gly Ser Ala Arg Pro Ser Asn Glu Gly Gly His
               340                 345                 350
   Tyr Met His Tyr Ala Ser Val Asp Asp Phe Leu Thr Asp Trp Phe Tyr
           355                 360                 365
   Leu Leu Arg Ala Gly Gly Ser Tyr Lys Val Ser Gly Ala Lys Thr Phe
       370                 375                 380
   Ser Glu Ala Ile Lys Gly Met Phe Lys Val Gly Gly Ala Val Tyr Asp
   385                 390                 395                 400
   Tyr Ala Ala Ser Gly Phe Asp Ser Tyr Ile Val Gly Ala Ser Ser Arg
                   405                 410                 415
   Leu Lys Ala Ile Glu Ala Glu Asn Gly Ser Leu Asp Lys Phe Asp Lys
               420                 425                 430
   Ala Thr Asp Ile Gly Asp Gly Ser Lys Asp Lys Ile Asp Ile Thr Ile
   435 440 445
   Glu Gly Ile Glu Val Thr Ile Asn Gly Ile Thr Tyr Glu Leu Thr Lys
   450 455 460
   Lys Pro Val Gly Trp Leu Lys Lys Ile Gly Lys Lys Ile Glu Arg Val
   465 470 475 480
   Gly Gln His Thr Arg Asp Ala Thr Ile Gln Gly Leu Gly Ile Pro Gln
   485 490 495
   Gln Ala Ala Asn Val Ala Ala Thr Ala Arg Gly
   500 505
<210> 103
   <211> 490
   <212> PRT
   <213> artificial
<220>
   <223> Magainin-lambdaSa2lys
<400> 103
   Gly Ile Gly Lys Phe Leu His Ser Ala Lys Lys Phe Gly Lys Ala Phe
   1               5                   10                  15
   Val Gly Glu Ile Met Asn Ser Glu Ile Asn Thr Glu Ile Ala Ile Ala
               20                  25                  30
   Trp Met Ser Ala Arg Gln Gly Lys Val Ser Tyr Ser Met Asp Tyr Arg
           35                  40                  45
   Asp Gly Pro Asn Ser Tyr Asp Cys Ser Ser Ser Val Tyr Tyr Ala Leu
       50                  55                  60
   Arg Ser Ala Gly Ala Ser Ser Ala Gly Trp Ala Val Asn Thr Glu Tyr
   65                  70                  75                  80
   Met His Asp Trp Leu Ile Lys Asn Gly Tyr Glu Leu Ile Ala Glu Asn
                   85                  90                  95
   Val Asp Trp Asn Ala Val Arg Gly Asp Ile Ala Ile Trp Gly Met Arg
               100                 105                 110
   Gly His Ser Ser Gly Ala Gly Gly His Val Val Met Phe Ile Asp Pro
           115                 120                 125
   Glu Asn Ile Ile His Cys Asn Trp Ala Asn Asn Gly Ile Thr Val Asn
       130                 135                 140
   Asn Tyr Asn Gln Thr Ala Ala Ala Ser Gly Trp Met Tyr Cys Tyr Val
   145                 150                 155                 160
   Tyr Arg Leu Lys Ser Gly Ala Ser Thr Gln Gly Lys Ser Leu Asp Thr
                   165                 170                 175
   Leu Val Lys Glu Thr Leu Ala Gly Asn Tyr Gly Asn Gly Glu Ala Arg
               180                 185                 190
   Lys Ala Val Leu Gly Asn Gln Tyr Glu Ala Val Met Ser Val Ile Asn
           195                 200                 205
   Gly Lys Thr Thr Thr Asn Gln Lys Thr Val Asp Gln Leu Val Gln Glu
       210                 215                 220
   Val Ile Ala Gly Lys His Gly Asn Gly Glu Ala Arg Lys Lys Ser Leu
   225                 230                 235                 240
   Gly Ser Gln Tyr Asp Ala Val Gln Lys Arg Val Thr Glu Leu Leu Lys
                   245                 250                 255
   Lys Gln Pro Ser Glu Pro Phe Lys Ala Gln Glu Val Asn Lys Pro Thr
               260                 265                 270
   Glu Thr Lys Thr Ser Gln Thr Glu Leu Thr Gly Gln Ala Thr Ala Thr
           275                 280                 285
   Lys Glu Glu Gly Asp Leu Ser Phe Asn Gly Thr Ile Leu Lys Lys Ala
       290                 295                 300
   Val Leu Asp Lys Ile Leu Gly Asn Cys Lys Lys His Asp Ile Leu Pro
   305                 310                 315                 320
   Ser Tyr Ala Leu Thr Ile Leu His Tyr Glu Gly Leu Trp Gly Thr Ser
                   325                 330                 335
   Ala Val Gly Lys Ala Asp Asn Asn Trp Gly Gly Met Thr Trp Thr Gly
               340                 345                 350
   Gln Gly Asn Arg Pro Ser Gly Val Thr Val Thr Gln Gly Ser Ala Arg
           355                 360                 365
   Pro Ser Asn Glu Gly Gly His Tyr Met His Tyr Ala Ser Val Asp Asp
       370                 375                 380
   Phe Leu Thr Asp Trp Phe Tyr Leu Leu Arg Ala Gly Gly Ser Tyr Lys
   385 390 395 400
   Val Ser Gly Ala Lys Thr Phe Ser Glu Ala Ile Lys Gly Met Phe Lys
   405 410 415
   Val Gly Gly Ala Val Tyr Asp Tyr Ala Ala Ser Gly Phe Asp Ser Tyr
   420 425 430
   Ile Val Gly Ala Ser Ser Arg Leu Lys Ala Ile Glu Ala Glu Asn Gly
   435 440 445
   Ser Leu Asp Lys Phe Asp Lys Ala Thr Asp Ile Gly Asp Gly Ser Lys
   450 455 460
   Asp Lys Ile Asp Ile Thr Ile Glu Gly Ile Glu Val Thr Ile Asn Gly
   465 470 475 480
   Ile Thr Tyr Glu Leu Thr Lys Lys Pro Val
   485 490
<210> 104
   <211> 349
   <212> **PRT**
   <213> artificial
<220>
   <223> Ply511-PK
<400> 104
   Val Lys Tyr Thr Val Glu Asn Lys Ile Ile Ala Gly Leu Pro Lys Gly
   1               5                   10                  15
   Lys Leu Lys Gly Ala Asn Phe Val Ile Ala His Glu Thr Ala Asn Ser
               20                  25                  30
   Lys Ser Thr Ile Asp Asn Glu Val Ser Tyr Met Thr Arg Asn Trp Lys
           35                  40                  45
   Asn Ala Phe Val Thr His Phe Val Gly Gly Gly Gly Arg Val Val Gln
       50                  55                  60
   Val Ala Asn Val Asn Tyr Val Ser Trp Gly Ala Gly Gln Tyr Ala Asn
   65                  70                  75                  80
   Ser Tyr Ser Tyr Ala Gln Val Glu Leu Cys Arg Thr Ser Asn Ala Thr
                   85                  90                  95
   Thr Phe Lys Lys Asp Tyr Glu Val Tyr Cys Gln Leu Leu Val Asp Leu
               100                 105                 110
   Ala Lys Lys Ala Gly Ile Pro Ile Thr Leu Asp Ser Gly Ser Lys Thr
           115                 120                 125
   Ser Asp Lys Gly Ile Lys Ser His Lys Trp Val Ala Asp Lys Leu Gly
       130                 135                 140
   Gly Thr Thr His Gln Asp Pro Tyr Ala Tyr Leu Ser Ser Trp Gly Ile
   145                 150                 155                 160
   Ser Lys Ala Gln Phe Ala Ser Asp Leu Ala Lys Val Ser Gly Gly Gly
                   165                 170                 175
   Asn Thr Gly Thr Ala Pro Ala Lys Pro Ser Thr Pro Ala Pro Lys Pro
               180                 185                 190
   Ser Thr Pro Ser Thr Asn Leu Asp Lys Leu Gly Leu Val Asp Tyr Met
           195                 200                 205
   Asn Ala Lys Lys Met Asp Ser Ser Tyr Ser Asn Arg Asp Lys Leu Ala
       210                 215                 220
   Lys Gln Tyr Gly Ile Ala Asn Tyr Ser Gly Thr Ala Ser Gln Asn Thr
   225                 230                 235                 240
   Thr Leu Leu Ser Lys Ile Lys Gly Gly Ala Pro Lys Pro Ser Thr Pro
                   245                 250                 255
   Ala Pro Lys Pro Ser Thr Ser Thr Ala Lys Lys Ile Tyr Phe Pro Pro
               260                 265                 270
   Asn Lys Gly Asn Trp Ser Val Tyr Pro Thr Asn Lys Ala Pro Val Lys
           275                 280                 285
   Ala Asn Ala Ile Gly Ala Ile Asn Pro Thr Lys Phe Gly Gly Leu Thr
       290                 295                 300
   Tyr Thr Ile Gln Lys Asp Arg Gly Asn Gly Val Tyr Glu Ile Gln Thr
   305                 310                 315                 320
   Asp Gln Phe Gly Arg Val Gln Val Tyr Gly Ala Pro Ser Thr Gly Ala
                   325                 330                 335
   Val Ile Lys Lys Lys Arg Lys Lys Arg Lys Lys Arg Lys
               340                 345
<210> 105
   <211> 351
   <212> PRT
   <213> artificial
<220>
   <223> Cpl-1-Indolicidin
<400> 105
   Val Lys Lys Asn Asp Leu Phe Val Asp Val Ser Ser His Asn Gly Tyr
   1               5                   10                  15
   Asp Ile Thr Gly Ile Leu Glu Gln Met Gly Thr Thr Asn Thr Ile Ile
               20                  25                  30
   Lys Ile Ser Glu Ser Thr Thr Tyr Leu Asn Pro Cys Leu Ser Ala Gln
           35                  40                  45
   Val Glu Gln Ser Asn Pro Ile Gly Phe Tyr His Phe Ala Arg Phe Gly
       50                  55                  60
   Gly Asp Val Ala Glu Ala Glu Arg Glu Ala Gln Phe Phe Leu Asp Asn
   65                  70                  75                  80
   Val Pro Met Gln Val Lys Tyr Leu Val Leu Asp Tyr Glu Asp Asp Pro
                   85                  90                  95
   Ser Gly Asp Ala Gln Ala Asn Thr Asn Ala Cys Leu Arg Phe Met Gln
               100                 105                 110
   Met Ile Ala Asp Ala Gly Tyr Lys Pro Ile Tyr Tyr Ser Tyr Lys Pro
           115                 120                 125
   Phe Thr His Asp Asn Val Asp Tyr Gln Gln Ile Leu Ala Gln Phe Pro
       130                 135                 140
   Asn Ser Leu Trp Ile Ala Gly Tyr Gly Leu Asn Asp Gly Thr Ala Asn
   145                 150                 155                 160
   Phe Glu Tyr Phe Pro Ser Met Asp Gly Ile Arg Trp Trp Gln Tyr Ser
                   165                 170                 175
   Ser Asn Pro Phe Asp Lys Asn Ile Val Leu Leu Asp Asp Glu Glu Asp
               180                 185                 190
   Asp Lys Pro Lys Thr Ala Gly Thr Trp Lys Gln Asp Ser Lys Gly Trp
           195                 200                 205
   Trp Phe Arg Arg Asn Asn Gly Ser Phe Pro Tyr Asn Lys Trp Glu Lys
       210                 215                 220
   Ile Gly Gly Val Trp Tyr Tyr Phe Asp Ser Lys Gly Tyr Cys Leu Thr
   225                 230                 235                 240
   Ser Glu Trp Leu Lys Asp Asn Glu Lys Trp Tyr Tyr Leu Lys Asp Asn
                   245                 250                 255
   Gly Ala Met Ala Thr Gly Trp Val Leu Val Gly Ser Glu Trp Tyr Tyr
               260                 265                 270
   Met Asp Asp Ser Gly Ala Met Val Thr Gly Trp Val Lys Tyr Lys Asn
           275                 280                 285
   Asn Trp Tyr Tyr Met Thr Asn Glu Arg Gly Asn Met Val Ser Asn Glu
       290                 295                 300
   Phe Ile Lys Ser Gly Lys Gly Trp Tyr Phe Met Asn Thr Asn Gly Glu
   305                 310                 315                 320
   Leu Ala Asp Asn Pro Ser Phe Thr Lys Glu Pro Asp Gly Leu Ile Thr
                   325                 330                 335
   Val Ala Ile Leu Pro Trp Lys Trp Pro Trp Trp Pro Trp Arg Arg
               340                 345                 350
<210> 106
   <211> 361
   <212> PRT
   <213> artificial
<220>
   <223> Magainin-Cpl-1
<400> 106
   Gly Ile Gly Lys Phe Leu His Ser Ala Lys Lys Phe Gly Lys Ala Phe
   1               5                   10                  15
   Val Gly Glu Ile Met Asn Ser Val Lys Lys Asn Asp Leu Phe Val Asp
               20                  25                  30
   Val Ser Ser His Asn Gly Tyr Asp Ile Thr Gly Ile Leu Glu Gln Met
           35                  40                  45
   Gly Thr Thr Asn Thr Ile Ile Lys Ile Ser Glu Ser Thr Thr Tyr Leu
       50                  55                  60
   Asn Pro Cys Leu Ser Ala Gln Val Glu Gln Ser Asn Pro Ile Gly Phe
   65                  70                  75                  80
   Tyr His Phe Ala Arg Phe Gly Gly Asp Val Ala Glu Ala Glu Arg Glu
                   85                  90                  95
   Ala Gln Phe Phe Leu Asp Asn Val Pro Met Gln Val Lys Tyr Leu Val
               100                 105                 110
   Leu Asp Tyr Glu Asp Asp Pro Ser Gly Asp Ala Gln Ala Asn Thr Asn
           115                 120                 125
   Ala Cys Leu Arg Phe Met Gln Met Ile Ala Asp Ala Gly Tyr Lys Pro
       130                 135                 140
   Ile Tyr Tyr Ser Tyr Lys Pro Phe Thr His Asp Asn Val Asp Tyr Gln
   145                 150                 155                 160
   Gln Ile Leu Ala Gln Phe Pro Asn Ser Leu Trp Ile Ala Gly Tyr Gly
                   165                 170                 175
   Leu Asn Asp Gly Thr Ala Asn Phe Glu Tyr Phe Pro Ser Met Asp Gly
               180                 185                 190
   Ile Arg Trp Trp Gln Tyr Ser Ser Asn Pro Phe Asp Lys Asn Ile Val
           195                 200                 205
   Leu Leu Asp Asp Glu Glu Asp Asp Lys Pro Lys Thr Ala Gly Thr Trp
       210                 215                 220
   Lys Gln Asp Ser Lys Gly Trp Trp Phe Arg Arg Asn Asn Gly Ser Phe
   225                 230                 235                 240
   Pro Tyr Asn Lys Trp Glu Lys Ile Gly Gly Val Trp Tyr Tyr Phe Asp
                   245                 250                 255
   Ser Lys Gly Tyr Cys Leu Thr Ser Glu Trp Leu Lys Asp Asn Glu Lys
               260                 265                 270
   Trp Tyr Tyr Leu Lys Asp Asn Gly Ala Met Ala Thr Gly Trp Val Leu
           275                 280                 285
   Val Gly Ser Glu Trp Tyr Tyr Met Asp Asp Ser Gly Ala Met Val Thr
       290                 295                 300
   Gly Trp Val Lys Tyr Lys Asn Asn Trp Tyr Tyr Met Thr Asn Glu Arg
   305                 310                 315                 320
   Gly Asn Met Val Ser Asn Glu Phe Ile Lys Ser Gly Lys Gly Trp Tyr
                   325                 330                 335
   Phe Met Asn Thr Asn Gly Glu Leu Ala Asp Asn Pro Ser Phe Thr Lys
               340                 345                 350
   Glu Pro Asp Gly Leu Ile Thr Val Ala
           355                 360
<210> 107
   <211> 336
   <212> **PRT**
   <213> artificial
<220>
   <223> PK-OBPgp279
<400> 107
   Lys Arg Lys Lys Arg Lys Lys Arg Lys Lys Asn Ser Glu Lys Asn Ala
   1               5                   10                  15
   Ser Ile Ile Met Ser Ile Gln Arg Thr Leu Ala Ser Leu Ser Leu Tyr
               20                  25                  30
   Gly Gly Arg Ile Asp Gly Leu Phe Gly Glu Lys Cys Arg Gly Ala Ile
           35                  40                  45
   Ile Leu Met Leu Asn Lys Val Tyr Pro Asn Phe Ser Thr Asn Lys Leu
       50                  55                  60
   Pro Ser Asn Thr Tyr Glu Ala Glu Ser Val Phe Thr Phe Leu Gln Thr
   65                  70                  75                  80
   Ala Leu Ala Gly Val Gly Leu Tyr Thr Ile Thr Ile Asp Gly Lys Trp
                   85                  90                  95
   Gly Gly Thr Ser Gln Gly Ala Ile Asp Ala Leu Val Lys Ser Tyr Arg
               100                 105                 110
   Gln Ile Thr Glu Ala Glu Arg Ala Gly Ser Thr Leu Pro Leu Gly Leu
           115                 120                 125
   Ala Thr Val Met Ser Lys His Met Ser Ile Glu Gln Leu Arg Ala Met
       130                 135                 140
   Leu Pro Thr Asp Arg Gln Gly Tyr Ala Glu Val Tyr Ile Asp Pro Leu
   145                 150                 155                 160
   Asn Glu Thr Met Asp Ile Phe Glu Ile Asn Thr Pro Leu Arg Ile Ala
                   165                 170                 175
   His Phe Met Ala Gln Ile Leu His Glu Thr Ala Cys Phe Lys Tyr Thr
               180                 185                 190
   Glu Glu Leu Ala Ser Gly Lys Ala Tyr Glu Gly Arg Ala Asp Leu Gly
           195                 200                 205
   Asn Thr Arg Pro Gly Asp Gly Pro Leu Phe Lys Gly Arg Gly Leu Leu
       210                 215                 220
   Gln Ile Thr Gly Arg Leu Asn Tyr Val Lys Cys Gln Val Tyr Leu Arg
   225                 230                 235                 240
   Glu Lys Leu Lys Asp Pro Thr Phe Asp Ile Thr Ser Ser Val Thr Cys
                   245                 250                 255
   Ala Gln Gln Leu Ser Glu Ser Pro Leu Leu Ala Ala Leu Ala Ser Gly
               260                 265                 270
   Tyr Phe Trp Arg Phe Ile Lys Pro Lys Leu Asn Glu Thr Ala Asp Lys
           275                 280                 285
   Asp Asp Ile Tyr Trp Val Ser Val Tyr Val Asn Gly Tyr Ala Lys Gln
       290                 295                 300
   Ala Asn Pro Tyr Tyr Pro Asn Arg Asp Lys Glu Pro Asn His Met Lys
   305                 310                 315                 320
   Glu Arg Val Gln Met Leu Ala Val Thr Lys Lys Ala Leu Gly Ile Val
                   325                 330                 335
<210> 108
   <211> 356
   <212> PRT
   <213> artificial
<220>
   <223> SMAP-29-OBPgp279
<400> 108
   Arg Gly Leu Arg Arg Leu Gly Arg Lys Ile Ala His Gly Val Lys Lys
   1               5                   10                  15
   Tyr Gly Pro Thr Val Leu Arg Ile Ile Arg Ile Ala Gly Lys Asn Ser
               20                  25                  30
   Glu Lys Asn Ala Ser Ile Ile Met Ser Ile Gln Arg Thr Leu Ala Ser
           35                  40                  45
   Leu Ser Leu Tyr Gly Gly Arg Ile Asp Gly Leu Phe Gly Glu Lys Cys
       50                  55                  60
   Arg Gly Ala Ile Ile Leu Met Leu Asn Lys Val Tyr Pro Asn Phe Ser
   65                  70                  75                  80
   Thr Asn Lys Leu Pro Ser Asn Thr Tyr Glu Ala Glu Ser Val Phe Thr
                   85                  90                  95
   Phe Leu Gln Thr Ala Leu Ala Gly Val Gly Leu Tyr Thr Ile Thr Ile
               100                 105                 110
   Asp Gly Lys Trp Gly Gly Thr Ser Gln Gly Ala Ile Asp Ala Leu Val
           115                 120                 125
   Lys Ser Tyr Arg Gln Ile Thr Glu Ala Glu Arg Ala Gly Ser Thr Leu
       130                 135                 140
   Pro Leu Gly Leu Ala Thr Val Met Ser Lys His Met Ser Ile Glu Gln
   145                 150                 155                 160
   Leu Arg Ala Met Leu Pro Thr Asp Arg Gln Gly Tyr Ala Glu Val Tyr
                   165                 170                 175
   Ile Asp Pro Leu Asn Glu Thr Met Asp Ile Phe Glu Ile Asn Thr Pro
               180                 185                 190
   Leu Arg Ile Ala His Phe Met Ala Gln Ile Leu His Glu Thr Ala Cys
           195                 200                 205
   Phe Lys Tyr Thr Glu Glu Leu Ala Ser Gly Lys Ala Tyr Glu Gly Arg
       210                 215                 220
   Ala Asp Leu Gly Asn Thr Arg Pro Gly Asp Gly Pro Leu Phe Lys Gly
   225                 230                 235                 240
   Arg Gly Leu Leu Gln Ile Thr Gly Arg Leu Asn Tyr Val Lys Cys Gln
                   245                 250                 255
   Val Tyr Leu Arg Glu Lys Leu Lys Asp Pro Thr Phe Asp Ile Thr Ser
               260                 265                 270
   Ser Val Thr Cys Ala Gln Gln Leu Ser Glu Ser Pro Leu Leu Ala Ala
           275                 280                 285
   Leu Ala Ser Gly Tyr Phe Trp Arg Phe Ile Lys Pro Lys Leu Asn Glu
       290                 295                 300
   Thr Ala Asp Lys Asp Asp Ile Tyr Trp Val Ser Val Tyr Val Asn Gly
   305                 310                 315                 320
   Tyr Ala Lys Gln Ala Asn Pro Tyr Tyr Pro Asn Arg Asp Lys Glu Pro
                   325                 330                 335
   Asn His Met Lys Glu Arg Val Gln Met Leu Ala Val Thr Lys Lys Ala
               340                 345                 350
   Leu Gly Ile Val
           355
<210> 109
   <211> 320
   <212> PRT
   <213> artificial
<220>
   <223> SMAP-29-EL188
<400> 109
   Arg Gly Leu Arg Arg Leu Gly Arg Lys Ile Ala His Gly Val Lys Lys
   1               5                   10                  15
   Tyr Gly Pro Thr Val Leu Arg Ile Ile Arg Ile Ala Gly Asn Phe Arg
               20                  25                  30
   Thr Lys Asn Gly Tyr Arg Asp Leu Gln Ala Leu Val Lys Glu Leu Gly
           35                  40                  45
   Leu Tyr Thr Gly Gln Ile Asp Gly Val Trp Gly Lys Gly Thr Ser Ser
       50                  55                  60
   Ser Thr Glu Thr Leu Leu Arg Gly Tyr Ala Glu Val Val Gly Lys Asn
   65                  70                  75                  80
   Thr Gly Gly Ile Gly Leu Pro Thr Thr Ser Asp Ala Ser Gly Tyr Asn
                   85                  90                  95
   Val Ile Thr Ala Leu Gln Arg Asn Leu Ala Phe Leu Gly Leu Tyr Ser
               100                 105                 110
   Leu Thr Val Asp Gly Ile Trp Gly Asn Gly Thr Leu Ser Gly Leu Asp
           115                 120                 125
   Lys Ala Phe Glu Val Tyr Lys Glu Arg Tyr Arg Thr Pro Thr Tyr Asp
       130                 135                 140
   Ile Ala Trp Ser Gly Lys Val Ser Pro Ala Phe Thr Ala Lys Val Lys
   145                 150                 155                 160
   Asp Trp Cys Gly Val His Val Pro Asn His Arg Ala Pro His Trp Leu
                   165                 170                 175
   Met Ala Cys Met Ala Phe Glu Thr Gly Gln Thr Phe Ser Pro Ser Ile
               180                 185                 190
   Lys Asn Ala Ala Gly Ser Glu Ala Tyr Gly Leu Ile Gln Phe Met Ser
           195                 200                 205
   Pro Ala Ala Asn Asp Leu Asn Val Pro Leu Ser Val Ile Arg Ser Met
       210                 215                 220
   Asp Gln Leu Thr Gln Leu Asp Leu Val Phe Lys Tyr Phe Glu Met Trp
   225                 230                 235                 240
   Met Lys Arg Gly Lys Arg Tyr Thr Gln Leu Glu Asp Phe Tyr Leu Thr
                   245                 250                 255
   Ile Phe His Pro Ala Ser Val Gly Lys Lys Ala Asp Glu Val Leu Phe
               260                 265                 270
   Leu Gln Gly Ser Lys Ala Tyr Leu Gln Asn Lys Gly Phe Asp Val Asp
           275                 280                 285
   Lys Asp Gly Lys Ile Thr Leu Gly Glu Ile Ser Ser Thr Leu Tyr Thr
       290                 295                 300
   Thr Tyr Tyr Lys Gly Leu Leu Pro Glu Asn Arg His Val Ile Ser Tyr
   305                 310                 315                 320
<210> 110
   <211> 300
   <212> **PRT**
   <213> artificial
<220>
   <223> PK-EL188
<400> 110
   Lys Arg Lys Lys Arg Lys Lys Arg Lys Asn Phe Arg Thr Lys Asn Gly
   1               5                   10                  15
   Tyr Arg Asp Leu Gln Ala Leu Val Lys Glu Leu Gly Leu Tyr Thr Gly
               20                  25                  30
   Gln Ile Asp Gly Val Trp Gly Lys Gly Thr Ser Ser Ser Thr Glu Thr
           35                  40                  45
   Leu Leu Arg Gly Tyr Ala Glu Val Val Gly Lys Asn Thr Gly Gly Ile
       50                  55                  60
   Gly Leu Pro Thr Thr Ser Asp Ala Ser Gly Tyr Asn Val Ile Thr Ala
   65                  70                  75                  80
   Leu Gln Arg Asn Leu Ala Phe Leu Gly Leu Tyr Ser Leu Thr Val Asp
                   85                  90                  95
   Gly Ile Trp Gly Asn Gly Thr Leu Ser Gly Leu Asp Lys Ala Phe Glu
               100                 105                 110
   Val Tyr Lys Glu Arg Tyr Arg Thr Pro Thr Tyr Asp Ile Ala Trp Ser
           115                 120                 125
   Gly Lys Val Ser Pro Ala Phe Thr Ala Lys Val Lys Asp Trp Cys Gly
       130                 135                 140
   Val His Val Pro Asn His Arg Ala Pro His Trp Leu Met Ala Cys Met
   145                 150                 155                 160
   Ala Phe Glu Thr Gly Gln Thr Phe Ser Pro Ser Ile Lys Asn Ala Ala
                   165                 170                 175
   Gly Ser Glu Ala Tyr Gly Leu Ile Gln Phe Met Ser Pro Ala Ala Asn
               180                 185                 190
   Asp Leu Asn Val Pro Leu Ser Val Ile Arg Ser Met Asp Gln Leu Thr
           195                 200                 205
   Gln Leu Asp Leu Val Phe Lys Tyr Phe Glu Met Trp Met Lys Arg Gly
       210                 215                 220
   Lys Arg Tyr Thr Gln Leu Glu Asp Phe Tyr Leu Thr Ile Phe His Pro
   225                 230                 235                 240
   Ala Ser Val Gly Lys Lys Ala Asp Glu Val Leu Phe Leu Gln Gly Ser
                   245                 250                 255
   Lys Ala Tyr Leu Gln Asn Lys Gly Phe Asp Val Asp Lys Asp Gly Lys
               260                 265                 270
   Ile Thr Leu Gly Glu Ile Ser Ser Thr Leu Tyr Thr Thr Tyr Tyr Lys
           275                 280                 285
   Gly Leu Leu Pro Glu Asn Arg His Val Ile Ser Tyr
       290                 295                 300
<210> 111
   <211> 256
   <212> PRT
   <213> artificial
<220>
   <223> PvP-SE1gp146-Buforin II
<400> 111
   Asn Ala Ala Ile Ala Glu Ile Gln Arg Met Leu Ile Glu Gly Gly Phe
   1               5                   10                  15
   Ser Val Gly Lys Ser Gly Ala Asp Gly Leu Tyr Gly Pro Ala Thr Lys
               20                  25                  30
   Ala Ala Leu Gln Lys Cys Ile Ala Gln Ala Thr Ser Gly Asn Asn Lys
           35                  40                  45
   Gly Gly Thr Leu Lys Leu Thr Gln Ala Gln Leu Asp Lys Ile Phe Pro
       50                  55                  60
   Val Gly Ala Ser Ser Gly Arg Asn Ala Lys Phe Leu Lys Pro Leu Asn
   65                  70                  75                  80
   Asp Leu Phe Glu Lys Thr Glu Ile Asn Thr Val Asn Arg Val Ala Gly
                   85                  90                  95
   Phe Leu Ser Gln Ile Gly Val Glu Ser Ala Glu Phe Arg Tyr Val Arg
               100                 105                 110
   Glu Leu Gly Asn Asp Ala Tyr Phe Asp Lys Tyr Asp Thr Gly Pro Ile
           115                 120                 125
   Ala Glu Arg Leu Gly Asn Thr Pro Gln Lys Asp Gly Asp Gly Ala Lys
       130                 135                 140
   Tyr Lys Gly Arg Gly Leu Ile Gln Val Thr Gly Leu Ala Asn Tyr Lys
   145                 150                 155                 160
   Ala Cys Gly Lys Ala Leu Gly Leu Asp Leu Val Asn His Pro Glu Leu
                   165                 170                 175
   Leu Glu Gln Pro Glu Tyr Ala Val Ala Ser Ala Gly Trp Tyr Trp Asp
               180                 185                 190
   Thr Arg Asn Ile Asn Ala Ala Cys Asp Ala Asp Asp Ile Val Lys Ile
                   195                 200                 205
   Thr Lys Leu Val Asn Gly Gly Thr Asn His Leu Ala Glu Arg Thr Ala
       210                 215                 220
   Tyr Tyr Lys Lys Ala Lys Ser Val Leu Thr Ser Thr Arg Ser Ser Arg
   225                 230                 235                 240
   Ala Gly Leu Gln Phe Pro Val Gly Arg Val His Arg Leu Leu Arg Lys
                   245                 250                 255
<210> 112
   <211> 248
   <212> PRT
   <213> artificial
<220>
   <223> PvP-SE1gp146-Indolicidin
<400> 112
   Asn Ala Ala Ile Ala Glu Ile Gln Arg Met Leu Ile Glu Gly Gly Phe
   1               5                   10                  15
   Ser Val Gly Lys Ser Gly Ala Asp Gly Leu Tyr Gly Pro Ala Thr Lys
               20                  25                  30
   Ala Ala Leu Gln Lys Cys Ile Ala Gln Ala Thr Ser Gly Asn Asn Lys
           35                  40                  45
   Gly Gly Thr Leu Lys Leu Thr Gln Ala Gln Leu Asp Lys Ile Phe Pro
       50                  55                  60
   Val Gly Ala Ser Ser Gly Arg Asn Ala Lys Phe Leu Lys Pro Leu Asn
   65                  70                  75                  80
   Asp Leu Phe Glu Lys Thr Glu Ile Asn Thr Val Asn Arg Val Ala Gly
                   85                  90                  95
   Phe Leu Ser Gln Ile Gly Val Glu Ser Ala Glu Phe Arg Tyr Val Arg
               100                 105                 110
   Glu Leu Gly Asn Asp Ala Tyr Phe Asp Lys Tyr Asp Thr Gly Pro Ile
           115                 120                 125
   Ala Glu Arg Leu Gly Asn Thr Pro Gln Lys Asp Gly Asp Gly Ala Lys
       130                 135                 140
   Tyr Lys Gly Arg Gly Leu Ile Gln Val Thr Gly Leu Ala Asn Tyr Lys
   145                 150                 155                 160
   Ala Cys Gly Lys Ala Leu Gly Leu Asp Leu Val Asn His Pro Glu Leu
                   165                 170                 175
   Leu Glu Gln Pro Glu Tyr Ala Val Ala Ser Ala Gly Trp Tyr Trp Asp
               180                 185                 190
   Thr Arg Asn Ile Asn Ala Ala Cys Asp Ala Asp Asp Ile Val Lys Ile
           195                 200                 205
   Thr Lys Leu Val Asn Gly Gly Thr Asn His Leu Ala Glu Arg Thr Ala
       210                 215                 220
   Tyr Tyr Lys Lys Ala Lys Ser Val Leu Thr Ser Ile Leu Pro Trp Lys
   225                 230                 235                 240
   Trp Pro Trp Trp Pro Trp Arg Arg
                   245
<210> 113
   <211> 284
   <212> PRT
   <213> artificial
<220>
   <223> Phage003/001_gp110 - Nigrocin 2
<400> 113
   Ala Ile Leu Lys Leu Gly Asn Arg Gly Ser Glu Val Lys Ala Leu Gln
   1               5                   10                  15
   Gln Ser Leu Asn Lys Ile Gly Phe Ser Leu Thr Ala Asp Gly Ile Phe
               20                  25                  30
   Gly Lys Ala Thr Glu Asn Ala Val Lys Ser Val Gln Ala Gly Ala Gly
           35                  40                  45
   Leu Val Ile Asp Gly Ile Ala Gly Pro Lys Thr Phe Tyr Ala Ile Arg
       50                  55                  60
   Asn Ala Gly Asp Ala His Gln Glu His Leu Thr Glu Ala Asp Leu Val
   65                  70                  75                  80
   Asp Ala Ala Arg Glu Leu Gly Val Glu Leu Ala Ser Met Lys Ala Val
                   85                  90                  95
   Asn Gln Val Glu Ser Arg Gly Thr Gly Phe Thr Lys Thr Gly Lys Ile
               100                 105                 110
   Lys Thr Leu Phe Glu Arg His Ile Met Tyr Lys Lys Val Thr Ala Lys
   115 120 125
   Phe Gly Gln Ala Arg Ala Asn Ala Leu Tyr Gln Leu Tyr Pro Thr Leu
   130 135 140
   Val Asn Pro Asn Ser Gly Gly Tyr Ile Gly Gly Asp Ala Glu Leu Glu
   145 150 155 160
   Arg Leu Gln Gly Ala Ile Ala Leu Asp Glu Asp Cys Ala Tyr Glu Ser
   165 170 175
   Ala Ser Tyr Gly Leu Phe Gln Ile Met Gly Phe Asn Cys Gln Ile Cys
   180 185 190
   Gly Tyr Ser Asn Ala Lys Glu Met Phe Thr Asp Phe Leu Thr Gly Glu
   195 200 205
   Arg Ala His Leu Leu Ala Phe Val Lys Phe Ile Lys Ala Asp Ala Asn
   210 215 220
   Met Trp Lys Ala Leu Lys Asn Lys Asn Trp Ala Glu Phe Ala Arg Arg
   225 230 235 240
   Tyr Asn Gly Pro Ala Tyr Ala Lys Asn Gln Tyr Asp Thr Lys Leu Ala
   245 250 255
   Ala Ala Tyr Lys Ser Phe Cys Gly Leu Leu Ser Lys Val Leu Gly Val
   260 265 270
   Gly Lys Lys Val Leu Cys Gly Val Ser Gly Leu Cys
   275 280
<210> 114
   <211> 288
   <212> **PRT**
   <213> artificial
<220>
   <223> Pleuricidin - Phage003/001_gp110
<400> 114
   Gly Trp Gly Ser Phe Phe Lys Lys Ala Ala His Val Gly Lys His Val
   1 5 10 15
   Gly Lys Ala Ala Leu Thr His Tyr Leu Ala Ile Leu Lys Leu Gly Asn
   20 25 30
   Arg Gly Ser Glu Val Lys Ala Leu Gln Gln Ser Leu Asn Lys Ile Gly
   35 40 45
   Phe Ser Leu Thr Ala Asp Gly Ile Phe Gly Lys Ala Thr Glu Asn Ala
   50 55 60
   Val Lys Ser Val Gln Ala Gly Ala Gly Leu Val Ile Asp Gly Ile Ala
   65 70 75 80
   Gly Pro Lys Thr Phe Tyr Ala Ile Arg Asn Ala Gly Asp Ala His Gln
   85 90 95
   Glu His Leu Thr Glu Ala Asp Leu Val Asp Ala Ala Arg Glu Leu Gly
   100 105 110
   Val Glu Leu Ala Ser Met Lys Ala Val Asn Gln Val Glu Ser Arg Gly
   115 120 125
   Thr Gly Phe Thr Lys Thr Gly Lys Ile Lys Thr Leu Phe Glu Arg His
   130 135 140
   Ile Met Tyr Lys Lys Val Thr Ala Lys Phe Gly Gln Ala Arg Ala Asn
   145 150 155 160
   Ala Leu Tyr Gln Leu Tyr Pro Thr Leu Val Asn Pro Asn Ser Gly Gly
   165 170 175
   Tyr Ile Gly Gly Asp Ala Glu Leu Glu Arg Leu Gln Gly Ala Ile Ala
   180 185 190
   Leu Asp Glu Asp Cys Ala Tyr Glu Ser Ala Ser Tyr Gly Leu Phe Gln
   195 200 205
   Ile Met Gly Phe Asn Cys Gln Ile Cys Gly Tyr Ser Asn Ala Lys Glu
   210 215 220
   Met Phe Thr Asp Phe Leu Thr Gly Glu Arg Ala His Leu Leu Ala Phe
   225 230 235 240
   Val Lys Phe Ile Lys Ala Asp Ala Asn Met Trp Lys Ala Leu Lys Asn
   245 250 255
   Lys Asn Trp Ala Glu Phe Ala Arg Arg Tyr Asn Gly Pro Ala Tyr Ala
   260 265 270
   Lys Asn Gln Tyr Asp Thr Lys Leu Ala Ala Ala Tyr Lys Ser Phe Cys
   275 280 285
<210> 115
   <211> 315
   <212> PRT
   <213> artificial
<220>
   <223> Cecropin A (A.aegypti)-Smi01
<400> 115
   Gly Gly Leu Lys Lys Leu Gly Lys Lys Leu Glu Gly Ala Gly Lys Arg
   1               5                   10                  15
   Val Phe Asn Ala Ala Glu Lys Ala Leu Pro Val Val Ala Gly Ala Lys
               20                  25                  30
   Ala Leu Arg Lys Glu Tyr Asp Met Ile Leu Lys Phe Gly Ser Lys Gly
           35                  40                  45
   Asp Ala Val Ala Thr Leu Gln Lys Gln Leu Ala Lys Met Gly Tyr Lys
       50                  55                  60
   Gly Val Lys Asp Lys Pro Leu Ser Val Asp Gly His Phe Gly Glu Ser
   65                  70                  75                  80
   Thr Glu Phe Ala Val Ile Gln Leu Gln Arg Lys Phe Gly Leu Val Ala
                   85                  90                  95
   Asp Gly Lys Val Gly Asp Lys Thr Arg Gln Ala Leu Ala Gly Asp Ser
               100                 105                 110
   Val Ser Lys Phe Leu Lys Asp Glu Asp Tyr Lys Lys Ala Ala Ile Arg
           115                 120                 125
   Leu Lys Val Pro Glu Leu Val Ile Arg Val Phe Gly Ala Val Glu Gly
       130                 135                 140
   Leu Gly Val Gly Phe Leu Pro Asn Gly Lys Ala Lys Ile Leu Phe Glu
   145                 150                 155                 160
   Arg His Arg Met Tyr Phe Tyr Leu Cys Gln Ala Leu Gly Lys Thr Phe
                   165                 170                 175
   Ala Asn Ser Gln Val Lys Ile Thr Pro Asn Ile Val Asn Thr Leu Thr
               180                 185                 190
   Gly Gly Tyr Lys Gly Asp Ala Ala Glu Tyr Thr Arg Leu Ser Met Ala
           195                 200                 205
   Ile Asn Ile His Lys Glu Ser Ala Leu Met Ser Thr Ser Trp Gly Gln
       210                 215                 220
   Phe Gln Ile Met Gly Glu Asn Trp Lys Asp Leu Gly Tyr Ser Ser Val
   225                 230                 235                 240
   Gln Glu Phe Val Asp Gln Gln Gln Leu Asn Glu Gly Asn Gln Leu Glu
                   245                 250                 255
   Ala Phe Ile Arg Phe Ile Glu Trp Lys Pro Gly Leu Leu Glu Ala Leu
               260                 265                 270
   Arg Lys Gln Asp Trp Asp Thr Val Phe Thr Leu Tyr Asn Gly Lys Asn
           275                 280                 285
   Tyr Lys Lys Leu Gly Tyr Gln Ala Lys Phe Gln Lys Glu Trp Asp His
       290                 295                 300
   Leu Glu Pro Ile Tyr Arg Glu Lys Thr Ala Ala
   305                 310                 315
<210> 116
   <211> 318
   <212> PRT
   <213> artificial
<220>
   <223> SarcotoxinIA-Smi01
<400> 116
   Gly Trp Leu Lys Lys Ile Gly Lys Lys Ile Glu Arg Val Gly Gln His
   1               5                   10                  15
   Thr Arg Asp Ala Thr Ile Gln Gly Leu Gly Ile Ala Gln Gln Ala Ala
               20                  25                  30
   Asn Val Ala Ala Thr Ala Arg Glu Tyr Asp Met Ile Leu Lys Phe Gly
           35                  40                  45
   Ser Lys Gly Asp Ala Val Ala Thr Leu Gln Lys Gln Leu Ala Lys Met
       50                  55                  60
   Gly Tyr Lys Gly Val Lys Asp Lys Pro Leu Ser Val Asp Gly His Phe
   65                  70                  75                  80
   Gly Glu Ser Thr Glu Phe Ala Val Ile Gln Leu Gln Arg Lys Phe Gly
                   85                  90                  95
   Leu Val Ala Asp Gly Lys Val Gly Asp Lys Thr Arg Gln Ala Leu Ala
               100                 105                 110
   Gly Asp Ser Val Ser Lys Phe Leu Lys Asp Glu Asp Tyr Lys Lys Ala
           115                 120                 125
   Ala Ile Arg Leu Lys Val Pro Glu Leu Val Ile Arg Val Phe Gly Ala
       130                 135                 140
   Val Glu Gly Leu Gly Val Gly Phe Leu Pro Asn Gly Lys Ala Lys Ile
   145                 150                 155                 160
   Leu Phe Glu Arg His Arg Met Tyr Phe Tyr Leu Cys Gln Ala Leu Gly
                   165                 170                 175
   Lys Thr Phe Ala Asn Ser Gln Val Lys Ile Thr Pro Asn Ile Val Asn
               180                 185                 190
   Thr Leu Thr Gly Gly Tyr Lys Gly Asp Ala Ala Glu Tyr Thr Arg Leu
           195                 200                 205
   Ser Met Ala Ile Asn Ile His Lys Glu Ser Ala Leu Met Ser Thr Ser
       210                 215                 220
   Trp Gly Gln Phe Gln Ile Met Gly Glu Asn Trp Lys Asp Leu Gly Tyr
   225                 230                 235                 240
   Ser Ser Val Gln Glu Phe Val Asp Gln Gln Gln Leu Asn Glu Gly Asn
                   245                 250                 255
   Gln Leu Glu Ala Phe Ile Arg Phe Ile Glu Trp Lys Pro Gly Leu Leu
               260                 265                 270
   Glu Ala Leu Arg Lys Gln Asp Trp Asp Thr Val Phe Thr Leu Tyr Asn
           275                 280                 285
   Gly Lys Asn Tyr Lys Lys Leu Gly Tyr Gln Ala Lys Phe Gln Lys Glu
       290                 295                 300
   Trp Asp His Leu Glu Pro Ile Tyr Arg Glu Lys Thr Ala Ala
   305                 310                 315
<210> 117
   <211> 295
   <212> PRT
   <213> artificial
<220>
   <223> Cecropin A (A.aegypti)-KZ144
<400> 117
   Gly Gly Leu Lys Lys Leu Gly Lys Lys Leu Glu Gly Ala Gly Lys Arg
   1               5                   10                  15
   Val Phe Asn Ala Ala Glu Lys Ala Leu Pro Val Val Ala Gly Ala Lys
               20                  25                  30
   Ala Leu Arg Lys Lys Val Leu Arg Lys Gly Asp Arg Gly Asp Glu Val
           35                  40                  45
   Ser Gln Leu Gln Thr Leu Leu Asn Leu Ser Gly Tyr Asp Val Gly Lys
       50                  55                  60
   Pro Asp Gly Ile Phe Gly Asn Asn Thr Phe Asn Gln Val Val Lys Phe
   65                  70                  75                  80
   Gln Lys Asp Asn Ser Leu Asp Ser Asp Gly Ile Val Gly Lys Asn Thr
                   85                  90                  95
   Trp Ala Glu Leu Phe Ser Lys Tyr Ser Pro Pro Ile Pro Tyr Lys Thr
               100                 105                 110
   Ile Pro Met Pro Thr Ala Asn Lys Ser Arg Ala Ala Ala Thr Pro Val
           115                 120                 125
   Met Asn Ala Val Glu Asn Ala Thr Gly Val Arg Ser Gln Leu Leu Leu
       130                 135                 140
   Thr Phe Ala Ser Ile Glu Ser Ala Phe Asp Tyr Glu Ile Lys Ala Lys
   145                 150                 155                 160
   Thr Ser Ser Ala Thr Gly Trp Phe Gln Phe Leu Thr Gly Thr Trp Lys
                   165                 170                 175
   Thr Met Ile Glu Asn Tyr Gly Met Lys Tyr Gly Val Leu Thr Asp Pro
               180                 185                 190
   Thr Gly Ala Leu Arg Lys Asp Pro Arg Ile Ser Ala Leu Met Gly Ala
           195                 200                 205
   Glu Leu Ile Lys Glu Asn Met Asn Ile Leu Arg Pro Val Leu Lys Arg
       210                 215                 220
   Glu Pro Thr Asp Thr Asp Leu Tyr Leu Ala His Phe Phe Gly Pro Gly
   225                 230                 235                 240
   Ala Ala Arg Arg Phe Leu Thr Thr Gly Gln Asn Glu Leu Ala Ala Thr
   245 250 255
   His Phe Pro Lys Glu Ala Gln Ala Asn Pro Ser Ile Phe Tyr Asn Lys
   260 265 270
   Asp Gly Ser Pro Lys Thr Ile Gln Glu Val Tyr Asn Leu Met Asp Gly
   275 280 285
   Lys Val Ala Ala His Arg Lys
   290 295
<210> 118
   <211> 298
   <212> PRT
   <213> artificial
<220>
   <223> SarcotoxinIA-KZ144
<400> 118
   Gly Trp Leu Lys Lys Ile Gly Lys Lys Ile Glu Arg Val Gly Gln His
   1               5                   10                  15
   Thr Arg Asp Ala Thr Ile Gln Gly Leu Gly Ile Ala Gln Gln Ala Ala
               20                  25                  30
   Asn Val Ala Ala Thr Ala Arg Lys Val Leu Arg Lys Gly Asp Arg Gly
           35                  40                  45
   Asp Glu Val Ser Gln Leu Gln Thr Leu Leu Asn Leu Ser Gly Tyr Asp
       50                  55                  60
   Val Gly Lys Pro Asp Gly Ile Phe Gly Asn Asn Thr Phe Asn Gln Val
   65                  70                  75                  80
   Val Lys Phe Gln Lys Asp Asn Ser Leu Asp Ser Asp Gly Ile Val Gly
                   85                  90                  95
   Lys Asn Thr Trp Ala Glu Leu Phe Ser Lys Tyr Ser Pro Pro Ile Pro
               100                 105                 110
   Tyr Lys Thr Ile Pro Met Pro Thr Ala Asn Lys Ser Arg Ala Ala Ala
           115                 120                 125
   Thr Pro Val Met Asn Ala Val Glu Asn Ala Thr Gly Val Arg Ser Gln
       130                 135                 140
   Leu Leu Leu Thr Phe Ala Ser Ile Glu Ser Ala Phe Asp Tyr Glu Ile
   145                 150                 155                 160
   Lys Ala Lys Thr Ser Ser Ala Thr Gly Trp Phe Gln Phe Leu Thr Gly
   165 170 175
   Thr Trp Lys Thr Met Ile Glu Asn Tyr Gly Met Lys Tyr Gly Val Leu
   180 185 190
   Thr Asp Pro Thr Gly Ala Leu Arg Lys Asp Pro Arg Ile Ser Ala Leu
   195 200 205
   Met Gly Ala Glu Leu Ile Lys Glu Asn Met Asn Ile Leu Arg Pro Val
   210 215 220
   Leu Lys Arg Glu Pro Thr Asp Thr Asp Leu Tyr Leu Ala His Phe Phe
   225 230 235 240
   Gly Pro Gly Ala Ala Arg Arg Phe Leu Thr Thr Gly Gln Asn Glu Leu
   245 250 255
   Ala Ala Thr His Phe Pro Lys Glu Ala Gln Ala Asn Pro Ser Ile Phe
   260 265 270
   Tyr Asn Lys Asp Gly Ser Pro Lys Thr Ile Gln Glu Val Tyr Asn Leu
   275 280 285
   Met Asp Gly Lys Val Ala Ala His Arg Lys
   290 295
<210> 119
   <211> 288
   <212> PRT
   <213> artificial
<220>
   <223> SMAP29-KZ144
<400> 119
   Arg Gly Leu Arg Arg Leu Gly Arg Lys Ile Ala His Gly Val Lys Lys
   1 5 10 15
   Tyr Gly Pro Thr Val Leu Arg Ile Ile Arg Ile Ala Gly Lys Val Leu
   20 25 30
   Arg Lys Gly Asp Arg Gly Asp Glu Val Ser Gln Leu Gln Thr Leu Leu
   35 40 45
   Asn Leu Ser Gly Tyr Asp Val Gly Lys Pro Asp Gly Ile Phe Gly Asn
   50 55 60
   Asn Thr Phe Asn Gln Val Val Lys Phe Gln Lys Asp Asn Ser Leu Asp
   65                  70                  75                  80
   Ser Asp Gly Ile Val Gly Lys Asn Thr Trp Ala Glu Leu Phe Ser Lys
                   85                  90                  95
   Tyr Ser Pro Pro Ile Pro Tyr Lys Thr Ile Pro Met Pro Thr Ala Asn
               100                 105                 110
   Lys Ser Arg Ala Ala Ala Thr Pro Val Met Asn Ala Val Glu Asn Ala
           115                 120                 125
   Thr Gly Val Arg Ser Gln Leu Leu Leu Thr Phe Ala Ser Ile Glu Ser
       130                 135                 140
   Ala Phe Asp Tyr Glu Ile Lys Ala Lys Thr Ser Ser Ala Thr Gly Trp
   145                 150                 155                 160
   Phe Gln Phe Leu Thr Gly Thr Trp Lys Thr Met Ile Glu Asn Tyr Gly
                   165                 170                 175
   Met Lys Tyr Gly Val Leu Thr Asp Pro Thr Gly Ala Leu Arg Lys Asp
               180                 185                 190
   Pro Arg Ile Ser Ala Leu Met Gly Ala Glu Leu Ile Lys Glu Asn Met
           195                 200                 205
   Asn Ile Leu Arg Pro Val Leu Lys Arg Glu Pro Thr Asp Thr Asp Leu
       210                 215                 220
   Tyr Leu Ala His Phe Phe Gly Pro Gly Ala Ala Arg Arg Phe Leu Thr
   225                 230                 235                 240
   Thr Gly Gln Asn Glu Leu Ala Ala Thr His Phe Pro Lys Glu Ala Gln
                   245                 250                 255
   Ala Asn Pro Ser Ile Phe Tyr Asn Lys Asp Gly Ser Pro Lys Thr Ile
               260                 265                 270
   Gln Glu Val Tyr Asn Leu Met Asp Gly Lys Val Ala Ala His Arg Lys
           275                 280                 285
<210> 120
   <211> 4
   <212> PRT
   <213> artificial
<220>
   <223> linker
<400> 120
   Gly Ala Gly Ala
   1
<210> 121
   <211> 8
   <212> PRT
   <213> artificial
<220>
   <223> linker
<400> 121
   Gly Ala Gly Ala Gly Ala Gly Ala
   1               5
<210> 122
   <211> 12
   <212> PRT
   <213> artificial
<220>
   <223> linker
<400> 122
   Gly Ala Gly Ala Gly Ala Gly Ala Gly Ala Gly Ala
   1               5                   10
<210> 123
   <211> 299
   <212> PRT
   <213> Artificial Sequence
<220>
   <223> SMAP29-KZ144 his
<400> 123
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Lys Val Leu Arg Lys Gly Asp Arg Gly Asp Glu Val Ser Gln Leu Gln
           35                  40                  45
   Thr Leu Leu Asn Leu Ser Gly Tyr Asp Val Gly Lys Pro Asp Gly Ile
       50                  55                  60
   Phe Gly Asn Asn Thr Phe Asn Gln Val Val Lys Phe Gln Lys Asp Asn
   65 70 75 80
   Ser Leu Asp Ser Asp Gly Ile Val Gly Lys Asn Thr Trp Ala Glu Leu
   85 90 95
   Phe Ser Lys Tyr Ser Pro Pro Ile Pro Tyr Lys Thr Ile Pro Met Pro
   100 105 110
   Thr Ala Asn Lys Ser Arg Ala Ala Ala Thr Pro Val Met Asn Ala Val
   115 120 125
   Glu Asn Ala Thr Gly Val Arg Ser Gln Leu Leu Leu Thr Phe Ala Ser
   130 135 140
   Ile Glu Ser Ala Phe Asp Tyr Glu Ile Lys Ala Lys Thr Ser Ser Ala
   145 150 155 160
   Thr Gly Trp Phe Gln Phe Leu Thr Gly Thr Trp Lys Thr Met Ile Glu
   165 170 175
   Asn Tyr Gly Met Lys Tyr Gly Val Leu Thr Asp Pro Thr Gly Ala Leu
   180 185 190
   Arg Lys Asp Pro Arg Ile Ser Ala Leu Met Gly Ala Glu Leu Ile Lys
   195 200 205
   Glu Asn Met Asn Ile Leu Arg Pro Val Leu Lys Arg Glu Pro Thr Asp
   210 215 220
   Thr Asp Leu Tyr Leu Ala His Phe Phe Gly Pro Gly Ala Ala Arg Arg
   225 230 235 240
   Phe Leu Thr Thr Gly Gln Asn Glu Leu Ala Ala Thr His Phe Pro Lys
   245 250 255
   Glu Ala Gln Ala Asn Pro Ser Ile Phe Tyr Asn Lys Asp Gly Ser Pro
   260 265 270
   Lys Thr Ile Gln Glu Val Tyr Asn Leu Met Asp Gly Lys Val Ala Ala
   275 280 285
   His Arg Lys Leu Glu His His His His His His
   290 295
<210> 124
   <211> 300
   <212> PRT
   <213> artificial
<220>
   <223> SarcotoxinIA-KZ144his
<400> 124
   Met Lys Phe Phe Arg Lys Leu Lys Lys Ser Val Lys Lys Arg Ala Lys
   1               5                   10                  15
   Glu Phe Phe Lys Lys Pro Arg Val Ile Gly Val Ser Ile Pro Phe Gly
               20                  25                  30
   Ser Lys Val Leu Arg Lys Gly Asp Arg Gly Asp Glu Val Cys Gln Leu
           35                  40                  45
   Gln Thr Leu Leu Asn Leu Cys Gly Tyr Asp Val Gly Lys Pro Asp Gly
       50                  55                  60
   Ile Phe Gly Asn Asn Thr Phe Asn Gln Val Val Lys Phe Gln Lys Asp
   65                  70                  75                  80
   Asn Cys Leu Asp Ser Asp Gly Ile Val Gly Lys Asn Thr Trp Ala Glu
                   85                  90                  95
   Leu Phe Ser Lys Tyr Ser Pro Pro Ile Pro Tyr Lys Thr Ile Pro Met
               100                 105                 110
   Pro Thr Ala Asn Lys Ser Arg Ala Ala Ala Thr Pro Val Met Asn Ala
           115                 120                 125
   Val Glu Asn Ala Thr Gly Val Arg Ser Gln Leu Leu Leu Thr Phe Ala
       130                 135                 140
   Ser Ile Glu Ser Ala Phe Asp Tyr Glu Ile Lys Ala Lys Thr Ser Ser
   145                 150                 155                 160
   Ala Thr Gly Trp Phe Gln Phe Leu Thr Gly Thr Trp Lys Thr Met Ile
                   165                 170                 175
   Glu Asn Tyr Gly Met Lys Tyr Gly Val Leu Thr Asp Pro Thr Gly Ala
               180                 185                 190
   Leu Arg Lys Asp Pro Arg Ile Ser Ala Leu Met Gly Ala Glu Leu Ile
           195                 200                 205
   Lys Glu Asn Met Asn Ile Leu Arg Pro Val Leu Lys Arg Glu Pro Thr
       210                 215                 220
   Asp Thr Asp Leu Tyr Leu Ala His Phe Phe Gly Pro Gly Ala Ala Arg
   225 230 235 240
   Arg Phe Leu Thr Thr Gly Gln Asn Glu Leu Ala Ala Thr His Phe Pro
   245 250 255
   Lys Glu Ala Gln Ala Asn Pro Ser Ile Phe Tyr Asn Lys Asp Gly Ser
   260 265 270
   Pro Lys Thr Ile Gln Glu Val Tyr Asn Leu Met Asp Gly Lys Val Ala
   275 280 285
   Ala His Arg Lys Leu Glu His His His His His His
   290 295 300
<210> 125
   <211> 374
   <212> PRT
   <213> Artificial sequence
<220>
   <223> CecropinA(A. aegyptii)-OBPlyshis
<400> 125
   Met Gly Gly Leu Lys Lys Leu Gly Lys Lys Leu Glu Gly Ala Gly Lys
   1               5                   10                  15
   Arg Val Phe Asn Ala Ala Glu Lys Ala Leu Pro Val Val Ala Gly Ala
               20                  25                  30
   Lys Ala Leu Arg Lys Gly Ser Lys Asn Ser Glu Lys Asn Ala Ser Ile
           35                  40                  45
   Ile Met Ser Ile Gln Arg Thr Leu Ala Ser Leu Ser Leu Tyr Gly Gly
       50                  55                  60
   Arg Ile Asp Gly Leu Phe Gly Glu Lys Cys Arg Gly Ala Ile Ile Leu
   65                  70                  75                  80
   Met Leu Asn Lys Val Tyr Pro Asn Phe Ser Thr Asn Lys Leu Pro Ser
                   85                  90                  95
   Asn Thr Tyr Glu Ala Glu Ser Val Phe Thr Phe Leu Gln Thr Ala Leu
               100                 105                 110
   Ala Gly Val Gly Leu Tyr Thr Ile Thr Ile Asp Gly Lys Trp Gly Gly
           115                 120                 125
   Thr Ser Gln Gly Ala Ile Asp Ala Leu Val Lys Ser Tyr Arg Gln Ile
       130                 135                 140
   Thr Glu Ala Glu Arg Ala Gly Ser Thr Leu Pro Leu Gly Leu Ala Thr
   145                 150                 155                 160
   Val Met Ser Lys His Met Ser Ile Glu Gln Leu Arg Ala Met Leu Pro
                   165                 170                 175
   Thr Asp Arg Gln Gly Tyr Ala Glu Val Tyr Ile Asp Pro Leu Asn Glu
               180                 185                 190
   Thr Met Asp Ile Phe Glu Ile Asn Thr Pro Leu Arg Ile Ala His Phe
           195                 200                 205
   Met Ala Gln Ile Leu His Glu Thr Ala Cys Phe Lys Tyr Thr Glu Glu
       210                 215                 220
   Leu Ala Ser Gly Lys Ala Tyr Glu Gly Arg Ala Asp Leu Gly Asn Thr
   225                 230                 235                 240
   Arg Pro Gly Asp Gly Pro Leu Phe Lys Gly Arg Gly Leu Leu Gln Ile
                   245                 250                 255
   Thr Gly Arg Leu Asn Tyr Val Lys Cys Gln Val Tyr Leu Arg Glu Lys
               260                 265                 270
   Leu Lys Asp Pro Thr Phe Asp Ile Thr Ser Ser Val Thr Cys Ala Gln
           275                 280                 285
   Gln Leu Ser Glu Ser Pro Leu Leu Ala Ala Leu Ala Ser Gly Tyr Phe
       290                 295                 300
   Trp Arg Phe Ile Lys Pro Lys Leu Asn Glu Thr Ala Asp Lys Asp Asp
   305                 310                 315                 320
   Ile Tyr Trp Val Ser Val Tyr Val Asn Gly Tyr Ala Lys Gln Ala Asn
                   325                 330                 335
   Pro Tyr Tyr Pro Asn Arg Asp Lys Glu Pro Asn His Met Lys Glu Arg
               340                 345                 350
   Val Gln Met Leu Ala Val Thr Lys Lys Ala Leu Gly Ile Val Leu Glu
           355                 360                 365
   His His His His His His
       370
<210> 126
   <211> 299
   <212> PRT
   <213> artificial sequence
<220>
   <223> mutated KZ144 with C14S, C23S and C50Splus SMAP-29 and HisTag
<400> 126
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Lys Val Leu Arg Lys Gly Asp Arg Gly Asp Glu Val Ser Gln Leu Gln
           35                  40                  45
   Thr Leu Leu Asn Leu Ser Gly Tyr Asp Val Gly Lys Pro Asp Gly Ile
       50                  55                  60
   Phe Gly Asn Asn Thr Phe Asn Gln Val Val Lys Phe Gln Lys Asp Asn
   65                  70                  75                  80
   Ser Leu Asp Ser Asp Gly Ile Val Gly Lys Asn Thr Trp Ala Glu Leu
                   85                  90                  95
   Phe Ser Lys Tyr Ser Pro Pro Ile Pro Tyr Lys Thr Ile Pro Met Pro
               100                 105                 110
   Thr Ala Asn Lys Ser Arg Ala Ala Ala Thr Pro Val Met Asn Ala Val
           115                 120                 125
   Glu Asn Ala Thr Gly Val Arg Ser Gln Leu Leu Leu Thr Phe Ala Ser
       130                 135                 140
   Ile Glu Ser Ala Phe Asp Tyr Glu Ile Lys Ala Lys Thr Ser Ser Ala
   145                 150                 155                 160
   Thr Gly Trp Phe Gln Phe Leu Thr Gly Thr Trp Lys Thr Met Ile Glu
                   165                 170                 175
   Asn Tyr Gly Met Lys Tyr Gly Val Leu Thr Asp Pro Thr Gly Ala Leu
               180                 185                 190
   Arg Lys Asp Pro Arg Ile Ser Ala Leu Met Gly Ala Glu Leu Ile Lys
           195                 200                 205
   Glu Asn Met Asn Ile Leu Arg Pro Val Leu Lys Arg Glu Pro Thr Asp
       210                 215                 220
   Thr Asp Leu Tyr Leu Ala His Phe Phe Gly Pro Gly Ala Ala Arg Arg
   225                 230                 235                 240
   Phe Leu Thr Thr Gly Gln Asn Glu Leu Ala Ala Thr His Phe Pro Lys
                   245                 250                 255
   Glu Ala Gln Ala Asn Pro Ser Ile Phe Tyr Asn Lys Asp Gly Ser Pro
               260                 265                 270
   Lys Thr Ile Gln Glu Val Tyr Asn Leu Met Asp Gly Lys Val Ala Ala
           275                 280                 285
   His Arg Lys Leu Glu His His His His His His
       290                 295